(19) **Europäisches Patentamt / European Patent Office / Office européen des brevets**

(11) **EP 4 523 694 A1**

(12) **EUROPEAN PATENT APPLICATION**
published in accordance with Art. 153(4) EPC

(43) Date of publication:
19.03.2025 Bulletin 2025/12

(21) Application number: 23803649.5

(22) Date of filing: 12.05.2023

(51) International Patent Classification (IPC):
*A61K 35/741* (2015.01)    *A61K 39/395* (2006.01)
*A61P 1/04* (2006.01)    *G01N 33/15* (2006.01)

(52) Cooperative Patent Classification (CPC):
A61K 35/741; A61K 39/395; A61P 1/04;
G01N 33/15

(86) International application number:
PCT/JP2023/018019

(87) International publication number:
WO 2023/219175 (16.11.2023 Gazette 2023/46)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 13.05.2022 JP 2022079852

(71) Applicant: The University of Tokyo
Bunkyo-ku, Tokyo 113-8654 (JP)

(72) Inventors:
• SHINKURA Reiko
Tokyo 113-8654 (JP)

• MORITA Naoki
Tokyo 113-8654 (JP)
• TAKAHASHI Keishyuu
Tokyo 113-8654 (JP)

(74) Representative: Grünecker Patent- und
Rechtsanwälte
PartG mbB
Leopoldstraße 4
80802 München (DE)

Remarks:
The complete document including Reference
Table(s) and the Sequence Listing(s) can be
downloaded from the EPO website

(54) **METHOD AND COMPOSITION BOTH FOR TREATING OR DIAGNOSING INFLAMMATORY BOWEL DISEASE (IBD)**

(57) The present disclosure relates to: a method, a composition, and a method for producing the same for treating IBD using gastrointestinal contents or excretions modified with an IgA antibody; a method for obtaining an IgA antibody that restores the bacterial flora in the gastrointestinal tract of an IBD patient; a method, a composition, and a method for producing the same for modifying gastrointestinal contents or excretions of an IBD patient using an IgA antibody; and a method, a composition, and a method for producing the same for testing with the diagnostic pharmaceutical drug containing an IgA antibody in a patient treated with the IBD therapeutic agent containing gastrointestinal contents or excretions.

EP 4 523 694 A1

**Description**

Technical Field

The present disclosure relates to: a method, a composition, and a method for producing the same for treating inflammatory bowel disease (IBD) using gastrointestinal contents or excretions modified with IgA antibodies; a method for obtaining an IgA antibody that restores the bacterial flora in the gastrointestinal tract of IBD patients; a method, a composition, and a method for producing the same for modifying gastrointestinal contents or excretions of IBD patients using an IgA antibody; and a method, a composition, and a method for producing the same for testing with diagnostic pharmaceutical drug containing IgA antibodies in patients treated with IBD therapeutic agents containing gastrointestinal contents or excretions.

Background Art

It has been reported that dysbiosis of the intestinal flora is associated with the development of many diseases including inflammatory bowel disease (IBD), and it is conceivable that improving the intestinal flora is important for maintaining health. In the development of inflammatory bowel disease, there is a report that enterocolitis does not develop in a case of sterility in a model mouse of spontaneous enteritis, and develops when there is enterobacteria (Sandra C. Kim, et al: Non-Patent Literature 1, Monika Schaubeck, et al: Non-Patent Literature 2). This suggests that the intestinal flora is one of environmental factors greatly involved in the development of inflammatory bowel disease. As bacteria that cause inflammatory bowel disease, Escherichia coli (E. coli) (Arlette Darfeuille-Michaud, et al: Non-Patent Literature 3, Adeline Sivignon, et al: Non-Patent Literature 4), Fusobacterium varium (F. varium) (Toshifumi Ohkusa, et al.: Non Patent Literature 5), and the like, which have adhesiveness and invasiveness to intestinal epithelial cells, have been mentioned as candidates, but clear causative bacteria and the like also remain unknown. From the above, it is expected that controlling the intestinal flora leads to prevention and treatment of diseases. Currently, sterilization of enterobacteria by administrating an antibiotic is used as one of treatment methods for controlling the intestinal flora. It is an object to remove pathogenic bacteria by administering an antibiotic. However, some enteritis-causing bacteria known as pathogenic bacteria are antibiotic-resistant bacteria, and as a result, not only fermentative bacteria are eliminated together with the enteritis-causing bacteria, but also antibiotic-resistant bacteria become dominant, which may lead to an increase in severity of the disease and an easily infected state. As an approach to a novel treatment in consideration of this problem, it is considered to be desirable to eliminate only the enteritis-causing bacteria while leaving the fermentative bacteria that improve the function of the intestine. More than 200,000 people per year in Japan only develop inflammatory bowel disease (IBD), which is a disease that is steadily increasing. This disease is characterized by repeating two phases, an active phase and a remission phase. At present, the deterioration is stopped by suppressing the inflammation in the active phase by a steroid drug or an anti-inflammatory drug such as Asacol. However, these are merely symptomatic treatments, and it is a radical cure to improve the intestinal environment causing inflammation, but when samples of patients are actually examined, their intestinal florae vary and it is considered that suppression thereof with a single drug is difficult. Therefore, in the treatment of IBD, it is important to appropriately diagnose the affection state and to provide optimal treatment to each patient.

For the purpose of improving the intestinal flora for treating diseases such as inflammatory bowel disease (IBD), administration of bacterial preparation, fecal transplantation, and the like are performed, and it has been reported that there is a certain effect, but a novel therapeutic method is still expected.

Citation List

Non Patent Literature

Non Patent Literature 1: Sandra C. Kim, et al., Gastroenterology 128, 891-906, 2005
Non Patent Literature 2: Monika Schaubeck, et al., Gut 65, 225-237, 2016
Non Patent Literature 3: Arlette Darfeuille-Michaud, et al., Gastroenterology 127, 412-421, 2004
Non Patent Literature 4: Adeline Sivignon, et al., Inflamm Bowel Dis 21, 276-286, 2015
Non Patent Literature 5: Toshifumi Ohkusa, et al., Journal of Medical Microbiology 58, 535-545, 2009

Summary of Invention

Technical Problem

An object of the present disclosure is to provide, in a treatment method for administering gastrointestinal contents or excretions for treating IBD, a method for obtaining gastrointestinal contents or excretions that improve bacterial flora in a gastrointestinal tract by processing the gastrointestinal contents or excretions using an IgA antibody, a method for obtaining an IgA antibody related to the method, and a pharmaceutical composition used in these methods.

Solution to Problem

The present disclosure provides a method for treating IBD using gastrointestinal contents or excretions modified with an IgA antibody and a composition for use in the method, and a method for producing the same.
In yet another aspect, the present disclosure provides a method for obtaining an IgA antibody that restores the bacterial flora in the gastrointestinal tract of an IBD patient.
In yet another aspect, the present disclosure provides a method for modifying the gastrointestinal contents or excretions of an IBD patient using an IgA antibody, a composition for use in the method, and a method for producing the same.
The present disclosure, in yet another aspect, contains gastrointestinal contents or excretions.
Provided are a method for testing patients treated with an IBD therapeutic agent with a diagnostic pharmaceutical drug containing an IgA antibody, a pharmaceutical drug used in the method, and a method for producing the same.
More specifically, the present disclosure provides, in one aspect,
a pharmaceutical composition for treating IBD, which contains gastrointestinal contents or excretions processed with an IgA antibody that restores the bacterial flora in the gastrointestinal tract.
The present disclosure provides, in one aspect, a method for treating IBD, including:

(A1) a step of preparing gastrointestinal contents or excretions of an IBD patient;
(A2) a step of modifying the gastrointestinal contents or excretions with an IgA antibody that restores the bacterial flora in the gastrointestinal tract; and
(A3) a step of administering modified gastrointestinal contents or excretions to an IBD patient.

The present disclosure provides, in one aspect, a method for treating IBD, including:

(A1') a step of preparing gastrointestinal contents or excretions of an IBD patient;
(A2') a step of administering the gastrointestinal contents or excretions to an IBD patient; and
(A3') a step of administering to an IBD patient an IgA antibody that restores the bacterial flora in the gastrointestinal tract, thereby modifying the gastrointestinal contents or excretions.

The present disclosure provides, in one aspect, a method for screening for IgA antibodies that restore bacterial flora in the gastrointestinal tract, the method including:

(B1) a step of preparing gastrointestinal contents or excretions of an IBD patient;
(B2) a step of modifying the gastrointestinal contents or excretions with a candidate IgA antibody; and
(B3) a step of confirming that the modified gastrointestinal contents or excretions are capable of restoring the bacterial flora in the gastrointestinal tract.

The present disclosure provides, in one aspect,
a composition containing an IgA antibody for processing the gastrointestinal contents or excretions of an IBD patient.
The present disclosure provides, in one aspect, a method for processing the gastrointestinal contents or excretions of an IBD patient, the method including:

(C1) a step of preparing the gastrointestinal contents or excretions of an IBD patient; and
(C2) a step of bringing the gastrointestinal contents or excretions into contact with an IgA antibody that restores the bacterial flora in the gastrointestinal tract.

The present disclosure provides, in one aspect, a method for testing subjects for the presence or absence of IBD, or risk of developing IBD, the method including:

(D1) a step of preparing IgA antibody that restores the bacterial flora in the gastrointestinal tract of an IBD patient;
(D2) a step of contacting the gastrointestinal contents or excretions of a subject with the IgA antibody; and
(D3) a step of identifying bacteria that bind to the IgA antibody,
wherein if the IgA antibody and IBD-related bacteria are bonded, the subject is processed as suffering from IBD or having a risk of developing IBD.

The present disclosure provides, in one aspect,
a pharmaceutical drug for diagnosing the presence or absence of IBD morbidity or the risk of developing IBD, the drug including an IgA antibody that restores the bacterial flora in the gastrointestinal tract of an IBD patient.

The present disclosure provides, in one aspect, a method for testing the therapeutic effect of an IBD therapeutic agent, the method including:

(E1) a step of obtaining gastrointestinal contents or excretions of a patient who is, is to be, or has been administered with an IBD therapeutic agent containing gastrointestinal contents or excretions;
(E2) a step of contacting the gastrointestinal contents or excretions with an IgA antibody; and
(E3) a step of analyzing bacteria that bind to the IgA antibody,
wherein the treatment with the IBD therapeutic agent is continued if the analysis result indicates restored bacterial flora in the gastrointestinal tract.

The present disclosure provides, in one aspect,
an IgA antibody-containing pharmaceutical drug for testing the gastrointestinal contents or excretions of a patient who is, is to be, or has been administered with an IBD therapeutic agent containing the gastrointestinal contents or excretions.
Still more specifically, the present disclosure provides the following.

[Clause 1]

A pharmaceutical composition for treating inflammatory bowel disease (IBD), including gastrointestinal contents or excretions processed with an IgA antibody that restores bacterial flora in a gastrointestinal tract.

[Clause 2]

A pharmaceutical composition for treating IBD, including an IgA antibody that is used in combination with a composition containing gastrointestinal contents or excretions and restores bacterial flora in a gastrointestinal tract.

[Clause 3]

The pharmaceutical composition according to clause 1 or 2, wherein the IgA antibody that restores bacterial flora in a gastrointestinal tract has one or more of following properties:

(1) an IgA antibody that binds to more IBD-related bacteria when brought into contact with gastrointestinal contents or excretions derived from an IBD patient, when the IgA antibody is brought into contact with gastrointestinal contents or excretions derived from an IBD patient or is brought into contact with gastrointestinal contents or excretions derived from a healthy person;
(2) an IgA antibody wherein when a first binding profile created by bringing gastrointestinal contents or excretions derived from an IBD patient into contact with the IgA antibody and based on a type and amount of bacteria bound to the IgA antibody is compared with a second binding profile created by bringing gastrointestinal contents or excretions derived from a healthy person into contact with the IgA antibody and based on a type and amount of bacteria bound to the IgA antibody, binding to more types of IBD-related bacteria or a greater amount of binding to IBD-related bacteria is observed in the first binding profile;
(3) an IgA antibody that increases bacterial flora diversity of gastrointestinal contents or excretions after processing with the IgA antibody when bacterial flora diversity of gastrointestinal contents or excretions derived from an IBD patient before processing with the IgA antibody is compared with bacterial flora diversity of gastrointestinal contents or excretions after processing with IgA antibody;
(4) an IgA antibody that increases bacterial flora diversity of gastrointestinal contents or excretions after administration when gastrointestinal contents or excretions modified with the IgA antibody are administered to a patient and bacterial flora diversity in a gastrointestinal tract of the patient before administration is compared with bacterial flora diversity of gastrointestinal contents or excretions after administration;
(5) an IgA antibody that increases a content of a short-chain fatty acid after administration when gastrointestinal contents or excretions modified with the IgA antibody are administered to a patient and a content of a short-chain fatty acid in a gastrointestinal tract of the patient before administration is compared with a content of a short-chain fatty acid in gastrointestinal contents or excretions of the patient after administration; and

(6) an IgA antibody that decreases a content of Enterobacteriaceae in gastrointestinal contents or excretions after administration when gastrointestinal contents or excretions modified with the IgA antibody are administered to a patient, and a content of Enterobacteriaceae in gastrointestinal contents or excretions of a subject before administration is compared with a content of Enterobacteriaceae in gastrointestinal contents or excretions after administration.

[Clause 4]

The pharmaceutical composition according to clause 1 or 2, wherein the gastrointestinal contents or excretions are derived from a subject to be administered with the composition.

[Clause 5]

A method for screening for IgA antibodies that restore bacterial flora in a gastrointestinal tract, the method including:

(B1) a step of preparing gastrointestinal contents or excretions of an IBD patient;
(B2) a step of modifying the gastrointestinal contents or excretions with a candidate IgA antibody; and
(B3) a step of confirming that the modified gastrointestinal contents or excretions are capable of restoring the bacterial flora in the gastrointestinal tract.

[Clause 6]

The method according to clause 5, wherein (B3) the step of confirming that modified gastrointestinal contents or excretions are capable of restoring bacterial flora in a gastrointestinal tract is performed by any of the followings:

(1) confirming that the IgA antibody binds to more IBD-related bacteria when brought into contact with gastrointestinal contents or excretions derived from an IBD patient, when the IgA antibody is brought into contact with gastrointestinal contents or excretions derived from the IBD patient or is brought into contact with gastrointestinal contents or excretions derived from a healthy person;
(2) confirming that when a first binding profile created by bringing gastrointestinal contents or excretions derived from an IBD patients into contact with the IgA antibody and based on a type and amount of bacteria bound to the IgA antibody is compared with a second binding profile created by bringing gastrointestinal contents or excretions derived from a healthy person into contact with the IgA antibody and based on a type and amount of bacteria bound to the IgA antibody, binding to more types of IBD-related bacteria or a greater amount of binding to IBD-related bacteria is observed in the first binding profile;
(3) confirming that bacterial flora diversity of gastrointestinal contents or excretions after processing with the IgA antibody is increased when bacterial flora diversity of gastrointestinal contents or excretions derived from an IBD patient before processing with the IgA antibody is compared with bacterial flora diversity of gastrointestinal contents or excretions after processing with the IgA antibody;
(4) confirming that bacterial flora diversity of gastrointestinal contents or excretions after administration is increased when gastrointestinal contents or excretions modified with the IgA antibody are administered to patients and bacterial flora diversity in a gastrointestinal tract of patients before administration is compared with bacterial flora diversity of gastrointestinal contents or excretions after administration;
(5) increasing a content of a short-chain fatty acid after administration when gastrointestinal contents or excretions modified with the IgA antibody are administered to a patient and a content of a short-chain fatty acid in a gastrointestinal tract of the patient before administration is compared with a content of a short-chain fatty acid in gastrointestinal contents or excretions of the patient after administration; and
(6) decreasing a content of Enterobacteriaceae in gastrointestinal contents or excretions after administration when gastrointestinal contents or excretions modified with the IgA antibody are administered to a patient, and a content of Enterobacteriaceae in gastrointestinal contents or excretions of a subject before administration is compared with a content of Enterobacteriaceae in gastrointestinal contents or excretions after administration.

[Clause 7]

A method for processing gastrointestinal contents or excretions of an IBD patient, the method including:

(C1) a step of preparing gastrointestinal contents or excretions of an IBD patient; and
(C2) a step of bringing gastrointestinal contents or excretions into contact with, in vitro, IgA that restores bacterial flora in a gastrointestinal tract.

[Clause 8]

A composition for modifying bacterial flora of gastrointestinal contents or excretions in vitro, including IgA that restores bacterial flora of a gastrointestinal tract.

[Clause 9]

A method for testing subjects for presence or absence of IBD morbidity, or a risk of developing IBD for diagnosis, the method including:

(D1) a step of preparing IgA antibody that restores bacterial flora in a gastrointestinal tract of an IBD patient;
(D2) a step of contacting the gastrointestinal contents or excretions of a subject with the IgA antibody; and
(D3) a step of identifying bacteria that bind to the IgA antibody,
wherein if the IgA antibody and IBD-related bacteria are bonded, the subject is processed as suffering from IBD or having a risk of developing IBD.

[Clause 10]

A pharmaceutical drug for diagnosing presence or absence of IBD morbidity or a risk of developing IBD, the drug including an IgA antibody that restores bacterial flora in a gastrointestinal tract of an IBD patient.

[Clause 11]

A method for testing therapeutic effect of an IBD therapeutic agent, the method including:

(E1) a step of obtaining gastrointestinal contents or excretions of a patient who is, is to be, or has been administered with an IBD therapeutic agent containing gastrointestinal contents or excretions;
(E2) a step of contacting the gastrointestinal contents or excretions with an IgA antibody; and
(E3) a step of analyzing bacteria that bind to the IgA antibody,
wherein treatment with the IBD therapeutic agent is continued if the analysis result indicates restored bacterial flora in a gastrointestinal tract.

[Clause 12]

The method according to clause 11, wherein the gastrointestinal contents or excretions are modified with the IgA.

[Clause 13]

A diagnostic pharmaceutical drug containing an IgA antibody for testing therapeutic effect of an IBD therapeutic agent in a patient who is, is to be, or has been administered with an IBD therapeutic agent containing gastrointestinal contents or excretions.

[Clause 14]

The diagnostic pharmaceutical drug according to clause 13, wherein the gastrointestinal contents or excretions are modified with the IgA.

Advantageous Effects of Invention

The present disclosure has an effect of providing, in a treatment method for administering gastrointestinal contents or excretions for treating IBD, a method for obtaining gastrointestinal contents or excretions that improve bacterial flora in a gastrointestinal tract by processing the gastrointestinal contents or excretions using an IgA antibody, a method for obtaining an IgA antibody related to the method, and a pharmaceutical composition used in these methods.

Brief Description of Drawings

Fig. 1 is a diagram showing the results of testing binding characteristics to various enterobacteria contained in feces collected from healthy persons or IBD patients, respectively, by collecting endogenous polyclonal IgA antibody-binding bacteria from healthy persons or IBD patients. The vertical axis represents IgA Index.

Fig. 2 is a diagram showing the results of testing the binding between IgA antibodies obtained from the gastrointestinal tract of IBD patients and IBD-related bacteria. Binding capacity of IgA antibodies to IBD-related bacteria was evaluated by ELISA assay.

Fig. 3 is a diagram showing that endogenous polyclonal IgA antibodies have a high binding capacity to IBD-related bacteria.

Fig. 4 is a diagram showing that the RS_H000_L001 IgA antibody has a high binding capacity to IBD-related bacteria.

Fig. 5 is a diagram showing that RS_H007_L004 IgA antibody that is a variant of RS_H000_L001 IgA antibody has a high binding capacity to IBD-related bacteria.

Fig. 6 is a diagram showing that SNK0003A IgA antibody has a high binding capacity to IBD-related bacteria.

Fig. 7 is a diagram showing that SNK0001A IgA antibody has a high binding capacity to IBD-related bacteria.

Fig. 8 is a diagram showing that SNK0002A IgA antibody has a high binding capacity to IBD-related bacteria.

Fig. 9 is a diagram showing the results of confirming the binding characteristics of RS_H000_L001 IgA antibody to bacteria in feces acquired from healthy persons or IBD patients.

Fig. 10 is a diagram showing that the RS_H000_L001 antibody exhibits a potent proliferation inhibitory effect on Gemella morbillorum in an in vitro proliferation inhibitory test.

Fig. 11 is a schematic diagram showing a scheme of a mouse oral bacterial transplantation test using enterobacteria derived from an IBD patient (P10).

Fig. 12 is a diagram showing body weight changes of (1) whole bacterial flora/antibody non-administration group and (2) whole bacterial flora/RS_H000_L001 antibody oral administration group in a mouse oral bacterial transplantation test using enterobacteria derived from the IBD patient (P10).

Fig. 13 is a diagram showing body weight changes of (3) RS_H000_L001 antibody-non-binding bacteria/non-antibody administration group and (4) RS_H000_L001 antibody-non-binding bacteria/RS_H000_L001 antibody oral administration group in a mouse oral bacteria transplantation test using enterobacteria derived from the IBD patient (P10).

Fig. 14 is a diagram showing a result of diversity analysis of intestinal flora of a mouse after treatment in a mouse oral bacterial transplantation test using enterobacteria derived from the IBD patient (P10).

Fig. 15 is a schematic diagram showing a scheme of a mouse oral bacterial transplantation test using enterobacteria derived from an IBD patient (P13).

Fig. 16 is a diagram showing binding between bacterial flora derived from feces of the IBD patient (P13) and RS_H000_L001 antibody.

Fig. 17 is a diagram showing body weight changes of (1) whole bacterial flora/antibody non-administration group and (2) whole bacterial flora/RS_H000_L001 antibody oral administration group in a mouse oral bacterial transplantation test using enterobacteria derived from the IBD patient (P13).

Fig. 18 is a diagram showing, by Shannon index, a result of diversity analysis of intestinal flora of a mouse after treatment in a mouse oral bacterial transplantation test using enterobacteria derived from the IBD patient (P13).

Fig. 19 is a diagram showing the result of diversity analysis of intestinal flora in a mouse after treatment in a mouse oral bacterial transplantation test using enterobacteria derived from the IBD patient (P13), as a change in the relative abundance ratio with respect to Fusobacteriaceae.

Fig. 20 is a diagram showing the result of diversity analysis of intestinal flora in a mouse after treatment in a mouse oral bacterial transplantation test using enterobacteria derived from the IBD patient (P13), as a change in the relative abundance ratio with respect to Lachnospiraceae.

Fig. 21 is a diagram showing the result of diversity analysis of intestinal flora in a mouse after treatment in a mouse oral bacterial transplantation test using enterobacteria derived from the IBD patient (P13), as a change in the relative abundance ratio with respect to Bacteroidaceae.

Fig. 22 is a diagram showing a staining result of a large intestine tissue section after treatment in a mouse oral bacteria transplantation test using enterobacteria derived from the IBD patient (P13).

Fig. 23 is a diagram showing a result of confirming binding of SNK0004 IgA antibody to bacteria in feces acquired from healthy persons or IBD patients.

Fig. 24 is a diagram showing a result of confirming binding of SNK0005 IgA antibody to bacteria in feces acquired from healthy persons or IBD patients.

Fig. 25 is a diagram showing the results of confirming the binding characteristics of SNK0004 IgA antibody to bacteria in feces acquired from IBD patients.

Fig. 26 is a diagram showing the results of confirming the binding characteristics of SNK0005 IgA antibody to bacteria in feces acquired from IBD patients.

Fig. 27 is a diagram showing the results of confirming the binding characteristics of SNK0001 IgA antibody to bacteria in feces acquired from IBD patients.

Fig. 28 is a diagram showing the results of confirming the binding characteristics of SNK0002 IgA antibody to bacteria in feces acquired from IBD patients.

Fig. 29 is a diagram showing the results of confirming the binding characteristics of SNK0003 IgA antibody to bacteria in feces acquired from IBD patients.

Fig. 30 is a diagram showing a result of comparing ratios of bacteria derived from feces of IBD patients binding to RS_H000_L001, SNK0001, SNK0002, SNK0003, SNK0004, and SNK0005 antibodies.

Description of Embodiments

It is known that an immunoglobulin A (IgA) antibody, which is one of antibody molecule isotypes, is important for maintenance of normal intestinal flora and infection defense against pathogenic microorganisms that have entered an intestinal tract. The IgA antibody is an antibody that mainly works not only in serum but also on a mucosal surface such as an intestinal tract. The IgA antibody is produced from IgA antibody-producing cells present in the lamina propria. The IgA antibody is secreted into the lamina propria via J chains as a dimeric IgA antibody. The dimeric IgA antibody is transported to the intestinal lumen through mucosal epithelial cells. This transport is performed by a multimeric Ig receptor expressed in mucosal epithelial cells. The dimeric IgA antibody binds to this receptor, is endocytosed into a vesicle, and is transported to the intestinal lumen surface by transcytosis. The extracellular domain of the receptor is cleaved by a protease on the intestinal lumen surface, and the dimeric IgA antibody is secreted into the intestinal lumen while retaining the extracellular domain (secretion factor). The secretion factor attached to the dimeric IgA antibody protects the antibody from degradation by proteolytic enzymes in the intestinal lumen. Through the above process, the secreted dimeric IgA antibody binds to bacteria in the intestinal lumen to prevent adhesion and invasion of bacteria to the epithelium.

The inventors of the present disclosure have intensively studied the role of an intestinal IgA antibody in the control of the intestinal flora, and as a result, have clarified that a host recognizes and controls the enterobacteria by the IgA antibody secreted into its own intestinal tract. Then, the present inventors have clarified that some of the intestinal IgA antibodies act in a direction of excluding bacteria bound from the intestinal lumen by identifying the bacteria, and some act to maintain the diversity of the intestinal flora by identifying and binding to the bacteria and retaining the bound bacteria in the intestinal mucosal layer. As described above, it has been considered that the intestinal IgA antibody controls the entire bacterial flora configuration while complicatedly interacting with the enterobacteria in a direction in which bacteria that favor the host are retained and in a direction in which bacteria that harm the host are excluded. However, there are unclarified points of what of each enterobacteria each IgA antibody identifies and acts and how to act on the target molecule.

As a result of intensive studies by the inventors of the present disclosure, it has been found that the condition of the bacterial flora in the intestinal tract of inflammatory bowel disease (IBD) patients is different from that of the bacterial flora in the intestinal tract of healthy persons, and bacteria associated with exacerbation of IBD (IBD-related bacteria) are increased, and the binding capacity of the IgA antibody of IBD patients to these IBD-related bacteria is reduced. Then, the present inventors have conceived that IBD is improved by processing the gastrointestinal contents or excretions of IBD patients with the IgA antibody that restores the bacterial flora in the intestinal tract, and administering the processed matter to IBD patients by fecal transplantation or the like, and have succeeded in actually confirming the effect thereof.

(Definition)

Unless defined otherwise, all technical and scientific terms used herein have the same meaning as commonly understood by one of the ordinary skill in the art to which this disclosure belongs.

In the present description, when a plurality of ranges of numerical values is indicated, a range including a combination of any lower limit value and upper limit value of the plurality of ranges is also meant in the same manner.

The term "substantially" herein has the same meaning as commonly understood by one of the ordinary skill in the art to which this disclosure belongs, but is used with the intent to include, for example, conditions of interest and conditions that are not unavoidably achieved due to biological or chemical properties, taking into account that biological or chemical phenomena may not completely achieve the conditions of interest.

As used herein, the term "about" in connection with a numerical value means that the value may vary, for example, within 10%, 9%, 8%, 7%, 6%, 5%, 4%, 3%, 2%, 1%, 0.5%, 0.1%, 0.05%, or 0.01%.

As used herein, the term "comprising" has the same meaning as commonly understood by one of the ordinary skill in the art to which this disclosure belongs, but includes, for example, "comprising" and "consisting of", and specifically, a composition "comprising" A may include other components, B, in addition to including only A.

The terms "consisting of" or "composed of" as used herein with respect to a composition have the same meaning as commonly understood by one of the ordinary skill in the art to which this disclosure belongs, but are used to indicate components that solely comprise the composition. For example, a composition "consisting of" A comprises only A. In one aspect, however, a composition "consisting of " A encompasses aspects that include contaminants other than A that are unavoidable in producing based on biological and chemical properties.

As used herein, "antibody" is used in the broadest sense and includes, but is not limited to, monoclonal antibodies, polyclonal antibodies, and antibody fragments that exhibit the intended antigen binding activity. The full-length antibody includes a heavy chain and a light chain mainly composed of a polypeptide. The heavy chain and the light chain each contain a site called a variable region that recognizes an antigen, and the site is generally called a heavy chain variable region and a light chain variable region, respectively. The variable region more specifically has, in order from the amino terminal, sites referred to as CDR1 to 3, each of which is identified as a site that recognizes an antigen. These CDR1 to 3 are also referred to more specifically as a heavy chain CDR1, a heavy chain CDR2, a heavy chain CDR3, a light chain CDR1, a light chain CDR2, a light chain CDR3, and the like. In addition, regions other than the CDR1 to 3 of the heavy chain and the light chain are referred to as a heavy chain FR1 to 4 and a light chain FR1 to 4, respectively, in this order from the amino terminal. The antibody may be in the form of an antibody composed of two heavy chains and two light chains, or in the form of an antibody composed of one heavy chain and one light chain (also referred to as a single-chain antibody).

The antibody is classified into classes such as IgG, IgE, IgM, IgD, IgA, or IgY, which are further classified into subclasses such as IgG1, IgG2, IgG3, IgG4, IgA1, or IgA2.

As used herein, an "antigen-binding fragment" of the antibody refers to one or more fragments of the antibody that retains the ability to specifically bind to an antigen. It has been found that the ability of an antibody to specifically bind to an antigen can also be maintained by fragments consisting of a part thereof. As one aspect, an "antigen-binding fragment " of an antibody may be, but is not limited to, a Fab fragment consisting of the light chain variable region (VL), the heavy chain variable region (VH), the light chain constant region (CL), and a CH1 domain that is a part of the heavy chain constant region, a F(ab') 2 fragment including two Fab fragments linked by a disulfide bridge at the hinge region, an Fd fragment consisting of the VH and CH1 domains, an Fv fragment consisting of the VL and VH domains of a single arm of an antibody, a dAb fragment including a single variable domain, and an isolated complementarity determining region (CDR).

The antibody of the present disclosure may be a CDR-grafted antibody. In one example, in the CDR-grafted antibody, some or all sequences of the CDR regions of an antibody derived from one animal species are substituted with CDR sequences of another animal species. For example, CDRs of one or more murine antibodies have been substituted with CDR sequences of human antibodies.

Methods known to one of the ordinary skill in the art can be used in identifying the heavy chain CDR1 to 3 in the heavy chain variable region and the light chain CDR1 to 3 in the light chain variable region of an antibody. For example, "Kabat definition" (Kabat et al., Ann.NY Acad, Sci. 1971, Vol. 190, pp. 382-391 and Kabat, E.A. et al. Sequences of Proteins of Immunological Interest, 5th Ed. 1991, U.S. Department of Health and Human Services, NIH Publication, pp. 91-3242), "Chothia definition" (Chothia et al., Nature, 1989, Vol. 342, pp. 877-883), "Contact definition" (MacCallum et al., J.Mol.Biol., 1996, Vol. 262, pp. 732-745), "IGMT" (Lefranc, M.-P., Nucl.Acids Res., 33, D593-D597 (2005)), and the like, which are known to one of the ordinary skill in the art, can be used. To identify CDR sequences within an antibody, the CDR may be identified based on information from public databases (for example, https://www.ncbi.nlm.nih.gov/igblast).

In the present description, the "identity" refers to the degree of the same amino acid sequence or base sequence of two or more comparable amino acid sequences or base sequences with respect to each other. Therefore, as the identity between two amino acid sequences or base sequences is higher, the identity or similarity between the sequences is higher. The level of amino acid sequence or base sequence identity is typically determined using FASTA, a tool for sequence analysis, and default parameters. Alternatively, it can be determined using the algorithm BLAST (for example, Karlin S, Altschul SF.Proc.Natl Acad Sci USA. 87:2264-2268 (1990), Karlin S, Altschul SF.Natl Acad Sci USA. 90:5873-7 (1993), etc.) by Karlin and Altschul. Programs called BLASTN and BLASTX based on such a BLAST algorithm have been developed (for example, Altschul SF, GishW, Miller W, Myers EW, Lipman DJ. J Mol Biol. 215:403-10 (1990), and the like). Specific techniques for these analysis methods are known and can be referred to the NCBI website. For example, a certain amino acid sequence A being specific percentage identical to another amino acid sequence B means that the amino acid sequence A and the amino acid sequence B have the percentage identity.

As used herein, the "monoclonal" is a modifier indicating a characteristic of an antibody or the like obtained from a substantially homogeneous population of antibodies. The individual antibodies included in such a population of antibodies are identical except for possible naturally occurring mutations that may be present in minor amounts.

In the present description, the "conservative substitution technique" means a technique in which an amino acid residue is substituted with an amino acid residue having a similar side chain.

For example, substitution between amino acid residues having a basic side chain such as lysine, arginine, and histidine corresponds to a conservative substitution technique. In addition, the substitution between amino acid residues having an acidic side chain, such as aspartic acid and glutamic acid; amino acid residues having a non-charged polar side chain, such as glycine, asparagine, glutamine, serine, threonine, tyrosine, and cysteine; amino acid residues having a nonpolar side chain, such as alanine, valine, leucine, isoleucine, proline, phenylalanine, methionine, and tryptophan; amino acid residues having a β-branched side chain, such as threonine, valine, and isoleucine; and amino acid residues having an aromatic side chain such as tyrosine, phenylalanine, tryptophan, and histidine corresponds to a conservative substitution technique.

In the present description, the "gastrointestinal tract" has a usual meaning in the technical field of the present disclosure, and typically means a tubular tissue from the oral cavity to the rectum, through which fed food passes from entering the body until discharged as excretion to the outside of the body. For example, the "gastrointestinal tract" includes the oral cavity, the throat, the esophagus, the stomach, the duodenum, the small intestine, and the large intestine.

As used herein, the "gastrointestinal contents" includes any substance present in the lumen of the "gastrointestinal tract". Examples thereof include fed food and drink, substances derived from host tissues excreted in the lumen, metabolites thereof, and mixtures thereof.

In the present description, the "excretion" includes all substances discharged from the "gastrointestinal tract". Typically, the discharged substance includes feces.

1. Method for treating IBD with gastrointestinal contents or excretions modified with IgA antibody

The present disclosure provides, in one aspect, a method for treating IBD using gastrointestinal contents or excretions modified with an IgA antibody.

The method for treating IBD of the present disclosure includes, in one aspect, the following steps.

(A1) A step of preparing gastrointestinal contents or excretions of an IBD patient,

(A2) A step of modifying the gastrointestinal contents or excretions with an IgA antibody that restores the bacterial flora in the gastrointestinal tract; and

(A3) A step of administering the modified gastrointestinal contents or excretions to the IBD patient.

The method for treating IBD of the present disclosure includes, in one aspect, the following steps.

(A1') A step of preparing gastrointestinal contents or excretions of an IBD patient.

(A2') A step of administering the gastrointestinal contents or excretions to the IBD patient.

(A3') A step of modifying the gastrointestinal contents or excretions by administering an IgA antibody that restores the bacterial flora in the gastrointestinal tract to an IBD patient.

(IgA antibody)

The bacterial flora in the gastrointestinal tract of IBD patients is clearly different from that of healthy persons, and has a configuration that exacerbates IBD. On the other hand, each IBD patient has a different specific bacterial flora configuration. Therefore, as the IgA antibody used in the present method, an IgA antibody obtained in accordance with the IBD patient to be treated is used as an IgA antibody that restores the bacterial flora in the gastrointestinal tract of the patient.

The confirmation that the IgA antibody can restore the bacterial flora in the gastrointestinal tract of an IBD patient is performed by, for example, analyzing the amount of IBD-related bacteria that bind to the IgA; analyzing a binding profile of a plurality of bacteria that bind to the IgA antibody; testing a physicochemical or biological property of gastrointestinal contents or excretions modified with the IgA antibody; for example, confirming that the bacterial flora diversity in the gastrointestinal contents or excretions in the gastrointestinal tract is increased; confirming that the bacterial flora diversity in the intestinal tract is increased by administering the gastrointestinal contents or excretions in the gastrointestinal tract; confirming that administration of the gastrointestinal contents or excretions in the gastrointestinal tract increases short-chain fatty acids in the intestinal tract; confirming that the administration of the gastrointestinal contents or excretions reduces Enterobacteriaceae present in the intestine; and confirming that the administration of the gastrointestinal contents or excretions improves the symptoms of IBD.

In a case of confirming that the IgA antibody can restore the bacterial flora in the gastrointestinal tract the IBD patient by analyzing the IBD-related bacteria that bind to the IgA antibody, for example, this can be performed by comparing the amount of binding between the IgA antibody and an enterobacteria (IBD-related bacteria) known to be associated with the deterioration of IBD for the gastrointestinal contents or excretions derived from the IBD patient and the gastrointestinal contents or excretions derived from the healthy person, and confirming that the IgA antibody binds to a larger amount of the IBD-related bacteria for the gastrointestinal contents or excretions derived from the IBD patient. This confirmation test can be performed using a method known to one of the ordinary skill in the art, and for example, FACS analysis, ELISA analysis, or the like can be used. IBD-related bacteria for the present disclosure are bacteria known to one of the ordinary skill in the art as enterobacteria known to be associated with exacerbation of IBD. Nonlimiting examples of such IBD-related bacteria include Enterobacteriaceae.

In a case of confirming that the IgA antibody can restore the bacterial flora in the gastrointestinal tract of IBD patients by analyzing the binding profile of a plurality of bacteria that bind to the IgA antibody, for example, this can be performed by contacting the IgA antibody with the gastrointestinal contents or excretions derived from the IBD patient, comparing a binding profile created based on the type and amount of bacteria bound to the IgA antibody with a binding profile created based on the type and amount of bacteria bound to the IgA antibody with the gastrointestinal contents or excretions derived from healthy persons, and confirming that in the binding profile created from the gastrointestinal contents or excretions derived from the IBD patient, binding to more types of IBD-related bacteria or a larger amount of binding to IBD-related bacteria is observed. In one example, the binding profile can be shown by quantitative data with the IgA Index, which is determined from the amounts of binding and no binding to IgA for each bacterium Andrew L. Kau, et al Sci Transl Med 7, 2015, Hirosuke Sugahara, et al., Frontiers in Microbiology 8, 1757, 2017). This confirmation test can be performed using a method known to one of the ordinary skill in the art, and for example, FACS analysis, ELISA analysis, analysis by a next-generation sequencer, and the like can be used.

In a case of confirming that the IgA antibody can restore the bacterial flora in the gastrointestinal tract of IBD patients by confirming that the bacterial flora aspect in the gastrointestinal contents or excretions processed with the IgA antibody is increased, for example, it is possible to confirm that the IgA antibody can restore the bacterial flora in the gastrointestinal tract of the IBD patient by comparing the bacterial flora diversity of the gastrointestinal contents or excretions before processing with the IgA antibody with the bacterial flora diversity of the gastrointestinal contents or excretions after processing with the IgA antibody and confirming that the bacterial flora diversity of the gastrointestinal contents or excretions after processing with the IgA antibody is increased. This confirmation test can be performed using a method known to one of the ordinary skill in the art, and for example, FACS analysis, ELISA analysis, analysis by a next-generation sequencer, and the like can be used.

In a case of confirming that the IgA antibody can restore the bacterial flora in the intestinal tract of IBD patients by administering the gastrointestinal contents or excretions processed with the IgA antibody to the subject, for example, it is possible to confirm that the IgA antibody can restore the bacterial flora in the gastrointestinal tract of IBD patients by comparing the bacterial flora diversity in the intestinal tract of the subject before administration with the bacterial flora diversity in the intestinal tract after administration and confirming the bacterial flora diversity of the gastrointestinal contents or excretions after administration is increased. This confirmation test can be performed using a method known to one of the ordinary skill in the art using the gastrointestinal contents or excretions of the subject after administration as a sample, and for example, FACS analysis, ELISA analysis, analysis by a next generation sequencer, and the like can be used. In the present test, IBD patients or IBD model animals may be used as subjects.

In a case of confirming that the IgA antibody can restore the bacterial flora in the gastrointestinal tract of IBD patients by confirming that the short-chain fatty acid in the intestinal tract is increased by administering the gastrointestinal contents or excretions, for example, it is possible to confirm that the IgA antibody can restore the bacterial flora in the gastrointestinal tract of IBD patients by comparing the content of the short-chain fatty acid in the gastrointestinal contents or excretions of the subject before the administration with the content of the short-chain fatty acid in the gastrointestinal contents or excretions after the administration, and confirming that the content of the short-chain fatty acid in the gastrointestinal contents or excretions after the administration is increased. This confirmation test can be performed using a method known to one of the ordinary skill in the art, and for example, chromatography analysis or the like can be used. In the present test, IBD patients or IBD model animals may be used as subjects.

In a case of confirming that the IgA antibody can restore the bacterial flora in the gastrointestinal tract of IBD patients by confirming that Enterobacteriaceae in an intestinal tract is reduced by administering the gastrointestinal contents or excretions, for example, it is possible to confirm that the IgA antibody can restore the bacterial flora in the gastrointestinal tract of the IBD patient by comparing the content of short-chain fatty acid in the gastrointestinal contents or excretions of the subject before the administration with the content of Enterobacteriaceae in the gastrointestinal contents or excretions after the administration, and confirming that the content of Enterobacteriaceae in the gastrointestinal contents or excretions after the administration is reduced. This confirmation test can be performed using a method known to one of the ordinary skill in the art, and for example, a bacterial colony culture test or the like can be used. In the present test, IBD patients or IBD model animals may be used as subjects.

In a case of confirming that the IgA antibody can restore the bacterial flora in the gastrointestinal tract of IBD patients by confirming that the symptoms of IBD are improved by administering the gastrointestinal contents or excretions, for example, it is possible to confirm that the IgA antibody can restore the bacterial flora in the gastrointestinal tract of the IBD patient by comparing the IBD symptoms of the subject before administration with the IBD symptoms of the subject after administration, and confirming that the symptoms after administration are improved. The confirmation test can be performed using an IBD diagnostic index known to one of the ordinary skill in the art. In the present test, IBD patients or IBD model animals may be used as subjects.

It can be confirmed previously that the IgA antibody can restore the bacterial flora in the gastrointestinal tract of IBD patients as a method different from the treatment method of the present disclosure, and as one of the steps constituting the present treatment method, the step (A1-2) may be performed using the gastrointestinal contents or excretions obtained in the step (A1).

The IgA antibody used in the method of the present disclosure may further bind other compounds or may fuse heterologous peptides as long as the binding characteristics are not lost.

The IgA antibody used in the method of the present disclosure may have an amino acid sequence derived from the same species as that of IBD patients, or may have an amino acid derived from a heterologous species such as a heterologous organism related thereto. Specific examples thereof include human-derived, mouse-derived, rat-derived, hamster-derived, rabbit-derived, goat-derived, donkey-derived, but-derived, bovine-derived, equine-derived, chicken derived, simian-derived, chimpanzee-derived, camel-derived, and llama-derived.

As the IgA antibody of the present disclosure, an IgA antibody produced from an antibody-producing cell derived from a B cell such as a hybridoma may be used, and an IgA antibody produced by introducing a nucleic acid encoding the antibody into a cell other than the immune system using a genetic recombination technique may be used as a recombinant antibody.

In one aspect, an IgA antibody of the disclosure includes:

a heavy chain variable region including the amino acid sequence of the heavy chain CDR1, the amino acid sequence of the heavy chain CDR2, and the amino acid sequence of the heavy chain CDR3 of the heavy chain variable region including the amino acid sequence represented by SEQ ID NO: 7; and

a light chain variable region including the amino acid sequence of the light chain CDR1, the amino acid sequence of the light chain CDR2, and the amino acid sequence of the light chain CDR3 of the light chain variable region including the amino acid sequence represented by SEQ ID NO: 8.

| Sequence name (SEQ ID NO) | Amino acid sequence |
|---|---|
| SNK0001_HV (SEQ ID NO: 7) | MEWIWIFLFILSGTAGVQSQVQLQQSGAELARPG ASVKLSCKASGYTFTSYGISWVKQRTGQGLEWIG EIYPRSGNTYYNEKFKGKATLTADKSSSTAYMEL RSLTSEDSAVYFCARLASSYYGSSYDWYFDVWGT GTTVTVSS |
| SNK0001_LV (SEQ ID NO: 8) | MRTPAQFLGILLLWFPGIKCDIKMTQSPSSMYAS LGERVTITCKASQDINSYLSWFQQKPGKSPKTLI YRANRLVDGVPSRFSGSGSGQDYSLTISSLEYED MGIYYCLQYDEFPLTFGAGTKLELK |

In one aspect, an IgA antibody of the disclosure includes:
a heavy chain variable region including
a heavy chain CDR1 including an amino acid sequence represented by SEQ ID NO: 1,
a heavy chain CDR2 including the amino acid sequence represented by SEQ ID NO: 2, and
a heavy chain CDR3 including the amino acid sequence represented by SEQ ID NO: 3; and
a light chain variable region including
a light chain CDR1 including an amino acid sequence represented by SEQ ID NO: 4,
a light chain CDR2 including an amino acid sequence represented by SEQ ID NO: 5, and
a light chain CDR3 including an amino acid sequence represented by SEQ ID NO: 6.

| Sequence name (SEQ ID NO) | Amino acid sequence |
|---|---|
| SNK0001_HCDR1 (SEQ ID NO: 1) | SYGIS |
| SNK0001_HCDR2 (SEQ ID NO: 2) | EIYPRSGNTYYNEKFK |
| SNK0001_HCDR3 (SEQ ID NO: 3) | FCARLASS |
| SNK0001_LCDR1 (SEQ ID NO: 4) | KASQDINSYLS |
| SNK0001_LCDR2 (SEQ ID NO: 5) | RANRLVD |
| SNK0001_LCDR3 (SEQ ID NO: 6) | LQYDEFPLT |

In one aspect, an IgA antibody of the disclosure includes:

a heavy chain variable region including the amino acid sequence represented by SEQ ID NO: 7 or a sequence having at least 90%, at least 95%, at least 98%, or at least 99% sequence identity thereof; and
a light chain variable region including the amino acid sequence represented by SEQ ID NO: 8 or a sequence having at least 90%, at least 95%, at least 98%, or at least 99% sequence identity thereof.

In one aspect, the IgA antibody of the present disclosure is a recombinant purified antibody SNK0001AR obtained by determining a nucleic acid sequence encoding an IgM antibody SNK0001M produced by a hybridoma obtained from a spleen-derived B cell, and applying a recombination technology to form an IgA antibody.

The antibody SNK0001AR has the following amino acid sequence configuration.

| SNK0001AR amino acid sequence | | |
|---|---|---|
| Heavy chain | Full-length sequence | MEWIWIFLFILSGTAGVQSQVQLQQSGAELARPGASVKLSCKASGYTFT SYGISWVKQRTGQGLEWIGEIYPRSGNTYYNEKFKGKATLTADKSSSTA YMELRSLTSEDSAVYFCARLASSYYGSSYDWYFDVWGTGTTVTVSSESA RNPTIYPLTLPRALSSDPVIIGCLIHDYFPSGTMNVTWGKSGKDITTVN FPPALASGGGYTMSSQLTLPAVECPEGESVKCSVQHDSNAVQELDVKCS GPPPPCPPCPPSCHPSLSLQRPALEDLLLGSDASLTCTLNGLRNPEGAV FTWEPSTGKDAVQKKAVQNSCGCYSVSSVLPGCAERWNSGASFKCTVTH PESDTLTGTIAKITVNTFPPQVHLLPPPSEELALNELVSLTCLVRAFNP KEVLVRWLHGNEELSPESYLVFEPLKEPGEGATTYLVTSVLRVSAELWK QGDQYSCMVGHEALPMNFTQKTIDRLSGKPTNVSVSVIMSEGDGICY |
| | Variable region | MEWIWIFLFILSGTAGVQSQVQLQQSGAELARPGASVKLSCKASGYTFT SYGISWVKQRTGQGLEWIGEIYPRSGNTYYNEKFKGKATLTADKSSSTA YMELRSLTSEDSAVYFCARLASSYYGSSYDWYFDVWGTGTTVTVSS |
| | CDR1 | SYGIS |
| | CDR2 | EIYPRSGNTYYNEKFK |
| | CDR3 | FCARLASS |
| Light chain | Full-length sequence | MRTPAQFLGILLLWFPGIKCDIKMTQSPSSMYASLGERVTITCKASQDI NSYLSWFQQKPGKSPKTLIYRANRLVDGVPSRFSGSGSGQDYSLTISSL EYEDMGIYYCLQYDEFPLTFGAGTKLELKRADAAPTVSIFPPSSEQLTS GGASVVCFLNNFYPKDINVKWKIDGSERQNGVLNSWTDQDSKDSTYSMS STLTLTKDEYERHNSYTCEATHKTSTSPIVKSFNRNEC |
| | Variable region | MRTPAQFLGILLLWFPGIKCDIKMTQSPSSMYASLGERVTITCKASQDI NSYLSWFQQKPGKSPKTLIYRANRLVDGVPSRFSGSGSGQDYSLTISSL EYEDMGIYYCLQYDEFPLTFGAGTKLELK |
| | CDR1 | KASQDINSYLS |
| | CDR2 | RANRLVD |
| | CDR3 | LQYDEFPLT |

In one aspect, an IgA antibody of the disclosure includes:
a heavy chain variable region including the amino acid sequence of the heavy chain CDR1, the amino acid sequence of the heavy chain CDR2, and the amino acid sequence of the heavy chain CDR3 of the heavy chain variable region including the amino acid sequence represented by SEQ ID NO: 17; and
a light chain variable region including the amino acid sequence of the light chain CDR1, the amino acid sequence of the light chain CDR2, and the amino acid sequence of the light chain CDR3 of the light chain variable region including the amino acid sequence represented by SEQ ID NO: 18.

| Sequence name (SEQ ID NO) | Amino acid sequence |
|---|---|
| SNK0002_HV (SEQ ID NO: 17) | MGRLTSSFLLLIVPAYVLSQVTLKESGPGILQPSQ TLSLTCSFSGFSLSTFGMGVGWIRQPSGKGLEWLA HIWWDDDKYYNPALKSRLTISKDTSKNQVFLKIAN VDTADTATYYCARIAGFDYWGQGTTLTVSS |
| SNK0002_LV (SEQ ID NO: 18) | MHFQVQIFSFLLISASVIMSRGQIVLTQSPAIMSA SPGEKVTITCSASSSVSYMHWFQQKPGTSPKLWIY STSNLASGVPARFSGSGSGTSYSLTISRMEAEDAA TYYCQQRSSYPYTFGGGTKLEIK |

In one aspect, an IgA antibody of the disclosure includes:
a heavy chain variable region including
a heavy chain CDR1 including an amino acid sequence represented by SEQ ID NO: 11,
a heavy chain CDR2 including the amino acid sequence represented by SEQ ID NO: 12, and
a heavy chain CDR3 including the amino acid sequence represented by SEQ ID NO: 13; and
a light chain variable region including
a light chain CDR1 including an amino acid sequence represented by SEQ ID NO: 14,
a light chain CDR2 including an amino acid sequence represented by SEQ ID NO: 15, and
a light chain CDR3 including an amino acid sequence represented by SEQ ID NO: 16.

| Sequence name (SEQ ID NO) | Amino acid sequence |
|---|---|
| SNK0002_HCDR1 (SEQ ID NO: 11) | TFGMG |
| SNK0002_HCDR2 (SEQ ID NO: 12) | LAHIWWDDDEYYNFAL |
| SNK0002_HCDR3 (SEQ ID NO: 13) | YYCARIAG |
| SNK0002_LCDR1 (SEQ ID NO: 14) | SASSSVSYMHW |
| SNK0002 LCDR2 (SEQ ID NO: 15) | STSNLASG |
| SNK0002_LCDR3 (SEQ ID NO: 16) | QRSSYPYTF |

In one aspect, an IgA antibody of the disclosure includes:

a heavy chain variable region including the amino acid sequence represented by SEQ ID NO: 17 or a sequence having at least 90%, at least 95%, at least 98%, or at least 99% sequence identity thereto; and
a light chain variable region including the amino acid sequence represented by SEQ ID NO: 18 or a sequence having at least 90%, at least 95%, at least 98%, or at least 99% sequence identity thereto.

In one aspect, the IgA antibody of the present disclosure is a recombinant purified antibody SNK0002AR obtained by determining the nucleic acid sequence of an IgM antibody SNK0002M produced by a hybridoma obtained from spleen-derived B cells and applying a recombination technique to form an IgA antibody.
The antibody SNK0002AR has the following amino acid sequence configuration.

| SNK0002AR amino acid sequence | | |
|---|---|---|
| Heavy chain | Full-length sequence | MGRLTSSFLLLIVPAYVLSQVTLKESGPGILQPSQTLSLTCSFSGFSLSTFGMGV GWIRQPSGKGLEWLAHIWWDDDKYYNPALKSRLTISKDTSKNQVFLKIANVDTAD TATYYCARIAGFDYWGQGTTLTVSSESARNPTIYPLTLPRALSSDPVIIGCLIHD YFPSGTMNVTWGKSGKDITTVNFPPALASGGGYTMSSQLTLPAVECPEGESVKCS VQHDSNAVQELDVKCSGPPPPCPPCPPSCHPSLSLQRPALEDLLLGSDASLTCTL NGLRNPEGAVFTWEPSTGKDAVQKKAVQNSCGCYSVSSVLPGCAERWNSGASFKC TVTHPESDTLTGTIAKITVNTFPPQVHLLPPPSEELALNELVSLTCLVRAFNPKE VLVRWLHGNEELSPESYLVFEPLKEPGEGATTYLVTSVLRVSAELWKQGDQYSCM VGHEALPMNFTQKTIDRLSGKPTNVSVSVIMSEGDGICY |
| | Variable region | MGRLTSSFLLLIVPAYVLSQVTLKESGPGILQPSQTLSLTCSFSGFSLSTFGMGV GWIRQPSGKGLEWLAHIWWDDDKYYNPALKSRLTISKDTSKNQVFLKIANVDTAD TATYYCARIAGFDYWGQGTTLTVSS |
| | CDR1 | TFGMG |
| | CDR2 | LAHIWWDDDKYYNPAL |
| | CDR3 | YYCARIAG |
| Light chain | Full-length sequence | MHFQVQIFSFLLISASVIMSRGQIVLTQSPAIMSASPGEKVTITCSASSSVSYMH WFQQKPGTSPKLWIYSTSNLASGVPARFSGSGSGTSYSLTISRMEAEDAATYYCQ QRSSYPYTFGGGTKLEIKRADAAPTVSIFPPSSEQLTSGGASVVCFLNNFYPKDI NVKWKIDGSERQNGVLNSWTDQDSKDSTYSMSSTLTLTKDEYERHNSYTCEATHK TSTSPIVKSFNRNEC |
| | Variable region | MHFQVQIFSFLLISASVIMSRGQIVLTQSPAIMSASPGEKVTITCSASSSVSYMH WFQQKPGTSPKLWIYSTSNLASGVPARFSGSGSGTSYSLTISRMEAEDAATYYCQ QRSSYPYTFGGGTKLEIK |
| | CDR1 | SARSSSVSYMHW |
| | CDR2 | STSNLASG |
| | CDR3 | QRSSYPYTF |

In one aspect, an IgA antibody of the disclosure includes:
a heavy chain variable region including the amino acid sequence of the heavy chain CDR1, the amino acid sequence of the heavy chain CDR2, and the amino acid sequence of the heavy chain CDR3 of the heavy chain variable region including the amino acid sequence represented by SEQ ID NO: 27; and
a light chain variable region including the amino acid sequence of the light chain CDR1, the amino acid sequence of the light chain CDR2, and the amino acid sequence of the light chain CDR3 of the light chain variable region including the amino acid sequence represented by SEQ ID NO: 28.

| Sequence name (SEQ ID NO) | Amino acid sequence |
|---|---|
| SNK0003_HV (SEQ ID NO: 27) | MGFSRIFLFLLSVTTGVHSQAYLQQSGAELVRPGAS VRMSCKASDYTFTSYNIHWVKQTPRQGLEWIGAIYS GNGATSHNQKFKGRATLTVDKSSSTAYMQLSSLTSE DSAVYFCTRVGLRSPFDFWGQGTTLTVSS |
| SNK0003_LV (SEQ ID NO: 28) | MESQTQVFVYMLLWLSGVDGDIVMTQSLKFMST SGGDRVSVTCKASQSVGTSVAWYQQKPGQSPKP LIYSASYRYSGVPDRFTGSGSGTDFSLTISNVQ SEDLAEYFCQQYNNYPYTFGGGTKLEIK |

In one aspect, an IgA antibody of the disclosure includes:
a heavy chain variable region including
a heavy chain CDR1 including an amino acid sequence represented by SEQ ID NO: 21,

a heavy chain CDR2 including the amino acid sequence represented by SEQ ID NO: 22, and
a heavy chain CDR3 including the amino acid sequence represented by SEQ ID NO: 23; and
a light chain variable region including
a light chain CDR1 including an amino acid sequence represented by SEQ ID NO: 24,
a light chain CDR2 including an amino acid sequence represented by SEQ ID NO: 25, and
a light chain CDR3 including an amino acid sequence represented by SEQ ID NO: 26.

| Sequence name (SEQ ID NO) | Amino acid sequence |
|---|---|
| SNK0003_HCDR1 (SEQ ID NO 21) | SYNIH |
| SNK0003_HCDR2 (SEQ ID NO: 22) | AIYSGNGATSENQKFK |
| SNKG003_HCDR3 (SEQ ID NO: 23) | FCTRVGLR |
| SNK0003_LCDR1 (SEQ ID NO: 24) | KASQSVGTSVA |
| SNK0003_LCDR2 (SEQ ID NO: 25) | SASYRYS |
| SNK0003_LCDR3 (SEQ ID NO: 26) | QQYNNYPYT |

In one aspect, an IgA antibody of the disclosure includes:

a heavy chain variable region including the amino acid sequence represented by SEQ ID NO: 27 or a sequence having at least 90%, at least 95%, at least 98%, or at least 99% sequence identity thereto; and
a light chain variable region including the amino acid sequence represented by SEQ ID NO: 28 or a sequence having at least 90%, at least 95%, at least 98%, or at least 99% sequence identity thereto.

In one aspect, the IgA antibody of the present disclosure is IgA antibody SNK0003A produced by a hybridoma obtained from intestinal lamina propria-derived B cells. The nucleic acid sequence encoding the antibody SNK0003A has been determined, and the recombinant purified antibody SNK0003AR produced by applying recombinant technology can also be used.

The antibody SNK0003A has the following amino acid sequence configuration.

| SNK0003A amino acid sequence | | |
|---|---|---|
| Heavy chain | Full-length sequence | MGFSRIFLFLLSVTTGVHSQAYLQQSGAELVRPGASVRMSCKASDYTFTSYNIHW VKQTPRQGLEWIGAIYSGNGATSHNQKFKGRATLTVDKSSSTAYMQLSSLTSEDS AVYFCTRVGLRSPFDFWGQGTTLTVSSESARNPTIYPLTLPRALSSDPVIIGCLI HDYFPSGTMNVTWGKSGKDITTVNFPPALASGGGYTMSSQLTLPAVECPEGESVK CSVQHDSNAVQELDVKCSGPPPPCPPCPPSCHPSLSLQRPALEDLLLGSDASLTC TLNGLRNPEGAVFTWEPSTGKDAVQKKAVQNSCGCYSVSSVLPGCAERWNSGASF KCTVTHPESDTLTGTIAKITVNTFPPQVHLLPPPSEELALNELVSLTCLVRAFNP KEVLVRWLHGNEELSPESYLVFEPLKEPGEGATTYLVTSVLRVSAELWKQGDQYS CMVGHEALPMNFTQKTIDRLSGKPTNVSVSVIMSEGDGICY |
| | Variable region | MGFSRIFLFLLSVTTGVHSQAYLQQSGAELVRPGASVRMSCKASDYTFTSYNIHW VKQTPRQGLEWIGAIYSGNGATSHNQKFKGRATLTVDKSSSTAYMQLSSLTSEDS AVYFCTRVGLRSPFDFWGQGTTLTVSS |
| | CDR1 | SYNIH |
| | COR2 | AIYSGNGATSHNQKFK |
| | CDR3 | FCTRVGLR |

(continued)

| SNK0003A amino acid sequence | | |
|---|---|---|
| Light chain | Full-length sequence | MESQTQVFVYMLLWLSGVDGDIVMTQSLKFMSTSGGDRVSVTCKASQSVGTSVAW YQQKPGQSPKPLIYSASYRYSGVPDRFTGSGSGTDFSLTISNVQSEDLAEYFCQQ YNNYPYTFGGGTKLEIKRADAAPTVSIFPPSSEQLTSGGASVVCFLNNFYPKDIN VKWKIDGSERQNGVLNSWTDQDSKDSTYSMSSTLTLTKDEYERHNSYTCEATHKT STSPIVKSFNRNEC |
| | Variable region | MESQTQVFVYMLLWLSGVDGDIVMTQSLKFMSTSGGDRVSVTCKASQSVGTSVAW YQQKPGQSPKPLIYSASYRYSGVPDRFTGSGSGTDFSLTISNVQSEDLAEYFCQQ YNNYPYTFGGGTKLEIK |
| | COR1 | KASQSVGTSVA |
| | CDR2 | SASYRYS |
| | CDR3 | QQYNNYPYT |

In one aspect, the IgA antibody of the present disclosure is a monoclonal antibody that binds to Clostridium difficile bacteria, including:

a heavy chain variable region including:

a heavy chain CDR1 including the amino acid sequence represented by SEQ ID NO: 31;
a heavy chain CDR2 including the amino acid sequence represented by SEQ ID NO: 32; and
a heavy chain CDR3 including the amino acid sequence represented by SEQ ID NO: 33, and
a light chain variable region including:

a light chain CDR1 including the amino acid sequence represented by SEQ ID NO: 41;
a light chain CDR2 including the amino acid sequence represented by SEQ ID NO: 42; and
a light chain CDR3 including the amino acid sequence represented by SEQ ID NO: 43.

In one aspect, the IgA antibody of the present disclosure includes a heavy chain variable region including the amino acid sequence represented by SEQ ID NO: 37, or a sequence having at least 90%, at least 95%, at least 98%, or at least 99% sequence identity thereto, and a light chain variable region including the amino acid sequence represented by SEQ ID NO: 38, or a sequence having at least 90%, at least 95%, at least 98%, or at least 99% sequence identity thereto.

In one aspect, the IgA antibody of the present disclosure is an IgA antibody that has at least one amino acid mutation in at least one region selected from heavy chains CDR1 to 3, light chains CDR1 to 3, and light chain FR1, with respect to a reference antibody including a heavy chain variable region including the amino acid sequence represented by SEQ ID NO: 37, and a light chain variable region including the amino acid sequence represented by SEQ ID NO: 38, and binds to the amino acid sequence RQEEHIELIAS (SEQ ID NO: 72) in the E. coli SHMT protein and the amino acid sequence VLDMMKLEKPE (SEQ ID NO: 73) in the iPGM protein of C. difficile.

| Sequence name (SEQ ID NO) | Amino acid sequence |
|---|---|
| W27G2_HV (SEQ ID NO: 37) | MKCSWIIFFLMAVVTGVNSEVQLQQSGSEL VKSGASVKLSCTVSGFNFTDYYIHWVRQRT EQGLEWIGRIDPENDETTYAPKFQGKATMT ADTSSNTAYLQLTSLTSEDTAVYYCARSTV LDYWGHGTTLTVSS |
| W27G2_LV (SEQ ID NO: 38) | MKLPVRLLVLMFWIPGFSSDVLMTQTPLSL PVSLGDQASISCRASQSIVHTNGNTYLEWY LQKPGQSPKLLIYKVSNRFSGVPDRFSGSG SGTDFILKISRVEAEDLGVYYCFQGSHVPP TFGGGTKLEVK |

At least one amino acid mutation in the above antibody can be identified by applying techniques available to one of the ordinary skill in the art using the results of three-dimensional structural analysis of complexes of an antibody having the amino acid sequence of the above reference antibody and E. coli SHMT protein, and an antibody having the amino acid sequence of the reference antibody and C. difficile iPGM protein. The number of amino acid mutations with respect to the reference antibody may be 1, 2, 3, 4, 5, 6, 7, 8, 9, 10, 11, 12, 13, 14, 15, 16, 17, 18, 19, 20 or more.

Such a reference antibody is not particularly limited as long as it has W27G2_HV (SEQ ID NO: 37) as a heavy chain variable region and W27G2_LV (SEQ ID NO: 38) as a light chain variable region. For example, an IgG antibody (W27RS_H000_L000GR) can be used. The antibody W27RS_H000_L000GR has the same heavy chain variable region and light chain variable region as the antibody W27G2 produced by a hybridoma obtained from B cells derived from the intestinal mucosa lamina propria.

| W27RS_H000_L000GR amino acid sequence | | |
|---|---|---|
| Heavy chain | Variable region | MKCSWIIFFLMAVVTGVNSEVQLQQSGSELVKSGASVKLSCTVSGFNFTDYYIHW VRQRTEQGLEWIGRIDPENDETTYAPKFQGKATMTADTSSNTAYLQLTSLTSEDT AVYYCARSTVLDYWGHGTTLTVSS |
| | CDR1 | DYYIH |
| | CDR2 | RIDENDETTYAPKFQ |
| | CDR3 | YCARSTVL |
| Light chain | Variable region | MKLPVRLLVLMFWIPGFSSDVLMTQTPLSLPVSLGDQASISCRASQSIVH TNGNTYLEWYLQKPGQSPKLLIYKVSNRFSGVPDRFSGSGSGTDFILKIS RVEAEDLGVYYCFQGSHVPPTFGGGTKLEVK |
| | CDR1 | RASQSIVHTNG |
| | CDR2 | KLLIYKV |
| | CDR3 | GVYYCFQGS |

Whether an antibody having the specified mutation actually binds to the amino acid sequence RQEEHIELIAS in the E. coli SHMT protein and the amino acid sequence VLDMMKLEKPE in the iPGM protein of C. difficile can be confirmed by methods known to one of the ordinary skill in the art, such as ELISA or Western blotting.

In one aspect, the antibody that binds to the C. difficile bacteria of the present disclosure is an antibody including:

a heavy chain variable region including a heavy chain CDR1 having the amino acid sequence of $X_1$YYIH,
a heavy chain CDR2 having the amino acid sequence of RIDPEN$X_2X_3$TTYAPKFQ,
a heavy chain CDR3 having the amino acid sequence of YCARSTVL; and
a light chain variable region including a light chain CDR1 having the amino acid sequence of R$X_4$SQSIVHTNG,
a light chain CDR2 having the amino acid sequence of KLLIYKV,
a light chain CDR3 having the amino acid sequence of GVYYFQGS, wherein
a light chain FR1 including the amino acid sequence of TPLSLPVSLGDQA or the amino acid sequence of SPAS$X_5$SVSLGDR$X_6$,
$X_1$, $X_2$, and $X_3$ are each independently a neutral polar amino acid or an acidic polar amino acid, $X_4$ is a nonpolar amino acid or a neutral polar amino acid, and $X_5$ and $X_6$ are each independently a nonpolar amino acid.

The above antibody was designed, based on three-dimensional structural analysis, to have a binding mode similar to that of the W27G2 antibody with respect to the amino acid sequence RQEEHIELIAS in the E. coli SHMT protein and the amino acid sequence VLDMMKLEKPE in the iPGM protein of C. difficile, and actually exhibits the same binding mode and physiological activity as the W27G2 antibody with respect to these bacteria.

In one aspect, the IgA antibody of the present disclosure is an antibody that binds to Clostridium difficile bacteria, and

includes a heavy chain variable region including a heavy chain CDR1 having the amino acid sequence of $X_1$YYIH, a heavy chain CDR2 having the amino acid sequence of RIDPEN$X_2X_3$TTYAPKFQ, a heavy chain CDR3 having the amino acid sequence of YCARSTVL, and a light chain variable region including a light chain CDR1 having the amino acid sequence of R$X_4$SQSIVHTNG, a light chain CDR2 having the amino acid sequence of KLLIYKV, and a light chain CDR3 having the amino acid sequence of GVYYFQGS, wherein
a light chain FR1 includes the amino acid sequence TPLSLPVSLGDQA or the amino acid sequence SPAS$X_5$SVSLGDR$X_6$,

$X_1$, $X_2$, and $X_3$ are each independently a neutral polar amino acid or an acidic polar amino acid, $X_4$ is a nonpolar amino acid or a neutral polar amino acid, and $X_5$ and $X_6$ are each independently a nonpolar amino acid, $X_1$ is aspartic acid, $X_2$ is aspartic acid, $X_3$ is glutamic acid, and $X_4$ is alanine.

In one aspect, the heavy chain of the IgA antibody of the present disclosure includes:
a heavy chain CDR1 including the amino acid sequence represented by SEQ ID NO: 31 or 41;
a heavy chain CDR2 including the amino acid sequence represented by any one of SEQ ID NOs: 32, 42 to 44; and
a heavy chain CDR3 including the amino acid sequence represented by SEQ ID NO: 33.

| Sequence name (SEQ ID NO) | Amino acid sequence |
|---|---|
| W27G2_HCDR1 (SEQ ID NO: 31) | RYYIH |
| W27G2_HCDR2 (SEQ ID NO: 32) | RIDPENDENTTYAPKFQ |
| W27G2_HCDR3 (SEQ ID NO: 33) | YCARSTVL |
| RS_HCDR1_D50N (SEQ ID NO: 41) | NYYIH |
| RS_HCDR2_D75N (SEQ ID NO: 42) | RIDPENNETTYAPKFQ |
| RS_HCDR2_E76Q (SEQ ID NO: 43) | RIDPENDQTTYAPKFQ |
| RS_HCDR2_D75N_E76Q (SEQ ID NO: 44) | RIDPENNQTTYAPKFQ |

In one aspect, the heavy chain of the IgA antibody of the present disclosure includes:

a heavy chain CDR1 including the amino acid sequence represented by SEQ ID NO: 31;
a heavy chain CDR2 including the amino acid sequence represented by SEQ ID NO: 32; and
a heavy chain CDR3 including the amino acid sequence represented by SEQ ID NO: 33.

In one aspect, the heavy chain of the IgA antibody of the present disclosure includes:

a heavy chain CDR1 including the amino acid sequence represented by SEQ ID NO: 31;
a heavy chain CDR2 including the amino acid sequence represented by SEQ ID NO: 42; and
a heavy chain CDR3 including the amino acid sequence represented by SEQ ID NO: 33.

In one aspect, the heavy chain of the IgA antibody of the present disclosure includes:

a heavy chain CDR1 including the amino acid sequence represented by SEQ ID NO: 31;
a heavy chain CDR2 including the amino acid sequence represented by SEQ ID NO: 43; and
a heavy chain CDR3 including the amino acid sequence represented by SEQ ID NO: 33.

In one aspect, the heavy chain of the IgA antibody of the present disclosure includes:

a heavy chain CDR1 including the amino acid sequence represented by SEQ ID NO: 31;
a heavy chain CDR2 including the amino acid sequence represented by SEQ ID NO: 44; and
a heavy chain CDR3 including the amino acid sequence represented by SEQ ID NO: 33.

In one aspect, the heavy chain of the IgA antibody of the present disclosure includes:

a heavy chain CDR1 including the amino acid sequence represented by SEQ ID NO: 41;
a heavy chain CDR2 including the amino acid sequence represented by SEQ ID NO: 32; and
a heavy chain CDR3 including the amino acid sequence represented by SEQ ID NO: 33.

In one aspect, the heavy chain of the IgA antibody of the present disclosure includes:

a heavy chain CDR1 including the amino acid sequence represented by SEQ ID NO: 41;
a heavy chain CDR2 including the amino acid sequence represented by SEQ ID NO: 42; and
a heavy chain CDR3 including the amino acid sequence represented by SEQ ID NO: 33.

In one aspect, the heavy chain of the IgA antibody of the present disclosure includes:

a heavy chain CDR1 including the amino acid sequence represented by SEQ ID NO: 41;
a heavy chain CDR2 including the amino acid sequence represented by SEQ ID NO: 43; and
a heavy chain CDR3 including the amino acid sequence represented by SEQ ID NO: 33.

In one aspect, the heavy chain of the IgA antibody of the present disclosure includes:

a heavy chain CDR1 including the amino acid sequence represented by SEQ ID NO: 41;
a heavy chain CDR2 including the amino acid sequence represented by SEQ ID NO: 44; and
a heavy chain CDR3 including the amino acid sequence represented by SEQ ID NO: 33.

In one aspect, the light chain of the IgA antibody of the present disclosure includes:
a light chain CDR1 including the amino acid sequence represented by SEQ ID NO: 34 or 52;
a light chain CDR2 including the amino acid sequence represented by SEQ ID NO: 35; and
a light chain CDR3 including the amino acid sequence represented by SEQ ID NO: 36.

| Sequence name (SEQ ID NO) | Amino acid sequence |
| --- | --- |
| W27G2_LCDR1 (SEQ ID NO: 34) | RASQSIVHTNG |
| W27G2_LCDR2 (SEQ ID NO: 35) | KLLIYKV |
| W27G2 LCDR3 (SEQ ID NO: 36) | GVYYCFQGS |
| RS_LCDR1_A44S (SEQ ID NO: 52) | RSSQSIVHTNG |

In one aspect, the light chain of the IgA antibody of the present disclosure includes:

a light chain CDR1 including the amino acid sequence represented by SEQ ID NO: 34;
a light chain CDR2 including the amino acid sequence represented by SEQ ID NO: 35; and
a light chain CDR3 including the amino acid sequence represented by SEQ ID NO: 36.

In one aspect, the light chain of the IgA antibody of the present disclosure includes:

a light chain CDR1 including the amino acid sequence represented by SEQ ID NO: 52;
a light chain CDR2 including the amino acid sequence represented by SEQ ID NO: 35; and
a light chain CDR3 including the amino acid sequence represented by SEQ ID NO: 36.

In one aspect, the IgA antibody of the present disclosure includes a sequence that binds to Protein L in the light chain variable region. An antibody molecule or an antigen-binding fragment thereof including a sequence binding to Protein L can be purified using a Protein L column.
In one aspect, an IgA antibody including a sequence that binds to Protein L of the present disclosure includes a light chain variable region including the amino acid sequence $SPASX_5SVSLGDRX_6$, wherein $X_5$ and $X_6$ are each independently a non-polar amino acid.
In one aspect, an IgA antibody including a sequence that binds to Protein L of the disclosure includes a light chain variable region including the amino acid sequence of $SPASX_5SVSLGDRX_6$, wherein $X_5$ is leucine or methionine and $X_6$ is alanine or valine.
In one aspect, the IgA antibody including a sequence binding Protein L of the disclosure includes a light chain variable region including an amino acid sequence represented by one selected from the group consisting of SEQ ID NO: 53 to 55.

| Sequence name (SEQ ID NO) | Amino acid sequence |
| --- | --- |
| RS_LFR1_L001 (SEQ ID NO: 53) | SPASLSVSLGDRA |
| RS_LFR1_L002 (SEQ ID NO: 54) | SPASLSVSLGDRV |
| RS_LFR1_L003 (SEQ ID NO: 55) | SPASMSVSLGDRA |
| RS_LFR1_L000 (SEQ ID NO: 56) | TPLSLPVSLGDQA |

In one aspect, the IgA antibody including a sequence that binds to Protein L of the disclosure includes a light chain variable region including an amino acid sequence represented by SEQ ID NO: 54.

This sequence is obtained by introducing a mutation into the sequence TPLSLPVSLGDQA (SEQ ID NO: 56) of the light chain FR1 region of the W27G2 antibody such that Protein L binds to the antibody molecule.

In one aspect, the IgA antibody including a sequence that binds Protein L of the disclosure or antigen-binding fragment thereof includes a light chain variable region including an amino acid sequence represented by SEQ ID NO: 55.

This sequence is obtained by further introducing a mutation into the light chain FR1 region of the light chain variable region including the amino acid sequence represented by SEQ ID NO: 54 such that Protein L and the antibody molecule bind more firmly.

In one aspect, the IgA antibody including a sequence that binds Protein L of the disclosure or antigen-binding fragment thereof includes a light chain variable region including an amino acid sequence represented by SEQ ID NO: 58.

This sequence is obtained by further introducing a mutation into the light chain FR1 region of the light chain variable region including the amino acid sequence represented by SEQ ID NO: 54 such that Protein L and the antibody molecule bind more firmly.

The IgA antibody of the present disclosure may have any combination of the heavy chain and the light chain described above, and may or may not include a sequence that binds to Protein L.

In one aspect, the IgA antibody or antigen-binding fragment thereof of the present disclosure includes:

a heavy chain variable region including

a heavy chain CDR1 including an amino acid sequence represented by SEQ ID NO: 31 or 41,
a heavy chain CDR2 including an amino acid sequence represented by any of SEQ ID NOs: 32 and 42 to 44, and
a heavy chain CDR3 including the amino acid sequence represented by SEQ ID NO: 33; and
a light chain variable region including:

a light chain CDR1 including an amino acid sequence represented by SEQ ID NO: 34 or 52,
a light chain CDR2 including the amino acid sequence represented by SEQ ID NO: 35, and
a light chain CDR3 including an amino acid sequence represented by SEQ ID NO: 36, and
a light chain FR1 including an amino acid sequence represented by any of SEQ ID NOs: 53 to 56.

For example, the IgA antibody of the disclosure includes a combination of any of the following heavy chain variable regions and any of the light chain variable regions.

| Heavy chain variable region | Amino acid sequence |
|---|---|
| W27G2_HV (SEQ ID NO: 37) | MKCSWIIFFLMAVVTGVNSEVQLQQSGSELVKSGASVKLS CTVSGFNFTDYYIHWVRQRTEQGLEWIGRIDPENDETTYA PKFQGKATMTADTSSNTAYLQLTSLTSEDTAVYYCARSTV LDYWGHGTTLTVSS |
| RS_HV001 (SEQ ID NO: 45) | MKCSWIIFFLMAVVTGVNSEVQLQQSGSELVKSGASVKLS CTVSGFNFTNYYIHWVRQRTEQGLEWIGRIDPENDETTYA PKFQGKATMTADTSSNTAYLQLTSLTSEDTAVYYCARSTV LDYWGHGTTLTVSS |
| RS_HV002 (SEQ ID NO: 46) | MKCSWIIFFLMAVVTGVNSEVQLQQSGSELVKSGASVKLS CTVSGFNFTDYYIHWVRQRTEQGLEWIGRIDPENNETTYA PKFQGKATMTADTSSNTAYLQLTSLTSEDTAVYYCARSTV LDYWGHGTTLTVSS |
| RS_HV003 (SEQ ID NO: 47) | MKCSWIIFFLMAVVTGVNSEVQLQQSGSELVKSGASVKLS CTVSGFNFTDYYIHWVRQRTEQGLEWIGRIDPENDQTTYA PKFQGKATMTADTSSNTAYLQLTSLTSEDTAVYYCARSTV LDYWGHGTTLTVSS |

(continued)

| Heavy chain variable region | Amino acid sequence |
|---|---|
| RS_HV004 (SEQ ID NO: 48) | MKCSWIIFFLMAVVTGVNSEVQLQQSGSELVKSGASVKLS CTVSGFNFTNYYIHWVRQRTEQGLEWIGRIDPENNETTYA PKFQGKATMTADTSSNTAYLQLTSLTSEDTAVYYCARSTV LDYWGHGTTLTVSS |
| RS_HV005 (SEQ ID NO: 49) | MKCSWIIFFLMAVVTGVNSEVQLQQSGSELVKSGASVKLS CTVSGFNFTNYYIHWVRQRTEQGLEWIGRIDPENDQTTYA PKFQGKATMTADTSSNTAYLQLTSLTSEDTAVYYCARSTV LDYWGHGTTLTVSS |
| RS_HV006 (SEQ ID NO: 50) | MKCSWIIFFLMAVVTGVNSEVQLQQSGSELVKSGASVKLS CTVSGFNFTDYYIHWVRQRTEQGLEWIGRIDPENNQTTYA PKFQGKATMTADTSSNTAYLQLTSLTSEDTAVYYCARSTV LDYWGHGTTLTVSS |
| RS_HV007 (SEQ ID NO: 51) | MKCSWIIFFLMAVVTGVNSEVQLQQSGSELVKSGASVKLS CTVSGFNFTNYYIHWVRQRTEQGLEWIGRIDPENNQTTYA PKFQGKATMTADTSSNTAYLQLTSLTSEDTAVYYCARSTV LDYWGHGTTLTVSS |

| Light chain variable region | Amino acid sequence |
|---|---|
| W27G2_LV (SEQ ID NO: 38) | MKLPVRLLVLMFWIPGFSSDVLMTQTPASLPVSLGDQASISC RASQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV001 (SEQ ID NO: 57) | MKLPVRLLVLMFWIPGFSSDVLMTQSPASLSVSLGDRATISC RASQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV002 (SEQ ID NO: 58) | MKLPVRLLVLMFWIPGFSSDVLMTQSPASLSVSLGDRVTISC RASQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV003 (SEQ ID NO: 59) | MKLPVRLLVLMFWIPGFSSDVLMTQSPASLSVSLGDRVTISC RSSQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV004 (SEQ ID NO: 60) | MKLPVRLLVLMFWIPGFSSDVLMTQSPASMSVSLGDRATISC RSSQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV005 (SEQ ID NO: 61) | MKLPVRLLVLMFWIPGFSSDVLMTQSPASLSVSLGDRATISC RSSQSIVHTNGNTYLEWYLQKPGQSPKLLIYKVSNRFSGVPD RFSGSGSGTDFILKISRVEAEDLGVYYCFQGSHVPPTFGGGT KLEVK |

Preferably, the IgA antibody of the present disclosure includes a combination of a heavy chain variable region and a light chain variable region as described below.

| Heavy chain variable region | Light chain variable region |
|---|---|
| ·W27G2_HV (SEQ ID NO: 37) | RS_LV001 (SEQ ID NO: 57) |
| W27G2_HV (SEQ ID NO: 37) | RS_LV004 (SEQ ID NO: 60) |
| W27G2_HV (SEQ ID NO: 37) | RS_LV005 (SEQ ID NO: 61) |
| RS_HV001 (SEQ ID NO: 45) | RS_LV005 (SEQ ID NO: 61) |
| RS_HV002 (SEQ ID NO: 46) | RS_LV005 (SEQ ID NO: 61) |
| RS_HV003 (SEQ ID NO: 47) | RS_LV005 (SEQ ID NO: 61) |
| RS_HV004 (SEQ ID NO: 48) | RS_LV005 (SEQ ID NO: 61) |
| RS_HV005 (SEQ ID NO: 49) | RS_LV005 (SEQ ID NO: 61) |
| RS_HV006 (SEQ ID NO: 50) | RS_LV005 (SEQ ID NO: 61) |
| RS_HV007 (SEQ ID NO: 51) | RS_LV001 (SEQ ID NO: 57) |
| RS_HV007 (SEQ ID NO: 51) | RS_LV002 (SEQ ID NO: 58) |
| RS_HV007 (SEQ ID NO: 51) | RS_LV003 (SEQ ID NO: 59) |
| RS_HV007 (SEQ ID NO: 51) | RS_LV004 (SEQ ID NO: 60) |
| RS_HV007 (SEQ ID NO: 51) | RS_LV005 (SEQ ID NO: 61) |

In one aspect, IgA antibodies and related antibodies thereof used as IgA of the present disclosure are shown below.

| Antibody name | Heavy chain variable region | Light chain variable region | Explanation |
|---|---|---|---|
| SNK0001M | SNK0001_HV | SNK0001_LV | Hybridoma-produced IgM |
| SNK0001MR | SNK0001_HV | SNK0001_LV | CHO cell-produced recombinant IgM |
| SNK0001MA | SNK0001_HV | SNK0001_LV | CHO cell-produced recombinant IgM |
| SNK0002M | SNK0002_HV | SNK0002_LV | Hybridoma-produced IgM |
| SNK0002MR | SNK0002_HV | SNK0002_LV | CHO cell-produced recombinant IgM |
| SNK0002MA | SNK0002_HV | SNK0002_LV | CHO cell-produced recombinant IgA |
| SNK0003A | SNK0003_HV | SNK0003_LV | Hybridoma-produced IgA |
| SNK0003AR | SNK0003_HV | SNK0003_LV | CHO cell-produced recombinant IgA |
| W27G2 | W27G2_HV | W27G2_LV | Hybridoma-produced IgA |
| RS_H000_L001 | W27G2_HV | RS_LV001 | CHO cell-produced recombinant IgA<br>Protein L binding type |
| RS_H000_L005 | W27G2_HV | RS_LV005 | CHO cell-produced recombinant IgA<br>Protein L binding type<br>Mutation in light chain CDR1 of W27G2 |
| RS_H001_L005 | RS_HV001 | RS_LV005 | CHO cell-produced recombinant IgA<br>Protein L binding type<br>Mutation in heavy chain CDR1 of W27G2<br>Mutation in light chain CDR1 of W27G2 |
| RS_H002_L005 | RS_HV002 | RS_LV005 | CHO cell-produced recombinant IgA<br>Protein L binding type<br>Mutation in heavy chain CDR2 of W27G2<br>Mutation in light chain CDR1 of W27G2 |
| RS_H003_L005 | RS_HV003 | RS_LV005 | CHO cell-produced recombinant IgA |

(continued)

| Antibody name | Heavy chain variable region | Light chain variable region | Explanation |
|---|---|---|---|
| | | | Protein L binding type<br>Mutation in heavy chain CDR2 of W27G2<br>Mutation in light chain CDR1 of W27G2 |
| RS_H004_L005 | RS_HV004 | RS_LV005 | CHO cell-produced recombinant IgA<br>Protein L binding type<br>Mutation in heavy chain CDR1 and CDR2 of W27G2<br>Mutation in light chain CDR1 of W27G2 |
| RS_H005_L005 | RS_HV005 | RS_LV005 | CHO cell-produced recombinant IgA Protein L binding type<br>Mutation in heavy chain CDR1 and CDR2 of W27G2<br>Mutation in light chain CDR1 of W27G2 |
| RS_H006_L005 | RS_HV006 | RS_LV005 | CHO cell-produced recombinant IgA Protein L binding type<br>Mutation in heavy chain CDR2 of W27G2<br>Mutation in light chain CDR1 of W27G2 |
| RS_H007_L001 | RS_HV007 | RS_LV001 | CHO cell-produced recombinant IgA Protein L binding type<br>Mutation in heavy chain CDR1 and CDR2 of W27G2 |
| RS_H007_L002 | RS_HV007 | RS_LV002 | CHO cell-produced recombinant IgA<br>Protein L binding type<br>Mutation in heavy chain CDR1 and CDR2 of W27G2 |
| RS_H007_L003 | RS_HV007 | RS_LV003 | CHO cell-produced recombinant IgA<br>Protein L binding type<br>Mutation in heavy chain CDR1 and CDR2 of W27G2<br>Mutation in light chain CDR1 and CDR2 of W27G2 |
| RS_H007_L004 | RS_HV007 | RS_LV004 | CHO cell-produced recombinant IgA<br>Protein L binding type<br>Mutation in heavy chain CDR1 and CDR2 of W27G2<br>Mutation in light chain CDR1 of W27G2 |
| RS_H007_L005 | RS_HV007 | RS_LV005 | CHO cell-produced recombinant IgA<br>Protein L binding type<br>Mutation in heavy chain CDR1 and CDR2 of W27G2<br>Mutation in light chain CDR1 of W27G2 |

An antibody produced in CHO cells by introducing a mutation into the heavy chain or the light chain so as not to substantially change the antigen specificity with respect to the W27G2 antibody is referred to as an RS variant recombinant purified antibody. These RS variant recombinant purified antibodies further have mutations for binding to protein L.

The IgA antibody of the present disclosure can have a mutation in a constituent amino acid sequence, for example, a heavy chain variable region, a light chain variable region, a heavy chain CDR1 to 3, or a light chain CDR1 to 3 thereof, as long as the antigen-binding characteristics thereof are not lost. The mutation may be a substitution, a deletion, an insertion, or the like, and is not limited thereto. For example, in the case of substitution, a conservative substitution technique can be adopted. Furthermore, analyzing the binding mode between the antibody and the antigen in detail using three-dimensional structure analysis or the like allows the IgA antibody of the present disclosure to introduce various mutations as long as the antigen-binding characteristics thereof are not lost.

(Nucleic acid)

A nucleic acid encoding an antibody or antigen-binding fragment thereof of the present disclosure may be a ribonucleotide or a deoxynucleotide. In addition, the form of the nucleic acid is not particularly limited, and may be a single-stranded form or a double-stranded form. The codons used in the nucleic acid sequence are not particularly limited, and various codons can be appropriately selected and used according to the purpose. For example, appropriate selection is possible in consideration of codon frequency and the like according to the type of host cell to be adopted at the time of production. In one aspect, a nucleic acid encoding an antibody of the present disclosure or antigen-binding fragment thereof expresses an antibody according to the present disclosure or antigen-binding fragment thereof and is utilized for production.

When the base nucleic acid encoding the antibody of the present disclosure or antigen-binding fragment thereof encodes an antigen-binding fragment, for example, the two domains of the Fv fragment, VL and VH, may be encoded by separate nucleic acid molecules, and utilizing recombinant techniques, a single protein chain (single-chain Fv (scFv)) in which the VL and VH region pairs form a monovalent molecule may be encoded by a single nucleic acid.

Sequence information related to a base nucleic acid encoding an antibody of the present disclosure or an antigen-binding fragment thereof is exemplified below.

>SEQ ID NO 1:SNK0001_HCDR1
SYGIS

>SEQ ID NO 2: SNK0001_HCDR2
EIYPRSGNTYYNEKFK

>SEQ ID NO 3: SNK0001_HCDR3
FCARLASS

>SEQ ID NO 4: SNK0001_LCDR1
KASQDINSYLS

>SEQ ID NO 5: SNK0001_LCDR2
RANRLVD

>SEQ ID NO 6: SNK0001_LCDR3
LQYDEFPLT

>SEQ ID NO 7: SNK0001_HV

MEWIWIFLFILSGTAGVQSQVQLQQSGAELARPGASVKLSCKASGYTFTSYGISWVKQR

TGQGLEWIGEIYPRSGNTYYNEKFKGKATLTADKSSSTAYMELRSLTSEDSAVYFCARL

ASSYYGSSYDWYFDVWGTGTTVTVSS

>SEQ ID NO 8: SNK0001_LV

MRTPAQFLGILLLWFPGIKCDIKMTQSPSSMYASLGERVTITCKASQDINSYLSWFQQK

PGKSPKTLIYRANRLVDGVPSRFSGSGSGQDYSLTISSLEYEDMGIYYCLQYDEFPLTF

GAGTKLELK

(SNK0001AR Antibody heavy chain amino acid sequence)

>SEQ ID NO 9: SNK0001AR_H

MEWIWIFLFILSGTAGVQSQVQLQQSGAELARPGASVKLSCKASGYTFTSYGISWVKQR

TGQGLEWIGEIYPRSGNTYYNEKFKGKATLTADKSSSTAYMELRSLTSEDSAVYFCARL

ASSYYGSSYDWYFDVWGTGTTVTVSSESARNPTIYPLTLPRALSSDPVIIGCLIHDYFP

SGTMNVTWGKSGKDITTVNFPPALASGGGYTMSSQLTLPAVECPEGESVKCSVQHDSNA

VQELDVKCSGPPPPCPPCPPSCHPSLSLQRPALEDLLLGSDASLTCTLNGLRNPEGAVF

TWEPSTGKDAVQKKAVQNSCGCYSVSSVLPGCAERWNSGASFKCTVTHPESDTLTGTIA

KITVNTFPPQVHLLPPPSEELALNELVSLTCLVRAFNPKEVLVRWLHGNEELSPESYLV

FEPLKEPGEGATTYLVTSVLRVSAELWKQGDQYSCMVGHEALPMNFTQKTIDRLSGKPT

NVSVSVIMSEGDGICY

(SNK0001AR Antibody light chain amino acid sequence)

>SEQ ID NO 10: SNK0001AR_L

MRTPAQFLGILLLWFPGIKCDIKMTQSPSSMYASLGERVTITCKASQDINSYLSWFQQK

PGKSPKTLIYRANRLVDGVPSRFSGSGSGQDYSLTISSLEYEDMGIYYCLQYDEFPLTF

GAGTKLELKRADAAPTVSIFPPSSEQLTSGGASVVCFLNNFYPKDINVKWKIDGSERQN

GVLNSWTDQDSKDSTYSMSSTLTLTKDEYERHNSYTCEATHKTSTSPIVKSFNRNEC

>SEQ ID NO 11: SNK0002_HCDR1
TFGMG

>SEQ ID NO 12: SNK0002_HCDR2
LAHIWWDDDKYYNPAL

>SEQ ID NO 13: SNK0002_HCDR3
YYCARIAG

>SEQ ID NO 14: SNK0002_LCDR1
SASSSVSYMHW

>SEQ ID NO 15: SNK0002_LCDR2
STSNLASG

>SEQ ID NO 16: SNK0002_LCDR3
QRSSYPYTF

>SEQ ID NO 17: SNK0002_HV

MGRLTSSFLLLIVPAYVLSQVTLKESGPGILQPSQTLSLTCSFSGFSLSTFGMGVGWIR

QPSGKGLEWLAHIWWDDDKYYNPALKSRLTISKDTSKNQVFLKIANVDTADTATYYCAR

IAGFDYWGQGTTLTVSS

>SEQ ID NO 18: SNK0002_LV

MHFQVQIFSFLLISASVIMSRGQIVLTQSPAIMSASPGEKVTITCSASSSVSYMHWFQQ

KPGTSPKLWIYSTSNLASGVPARFSGSGSGTSYSLTISRMEAEDAATYYCQQRSSYPYT

FGGGTKLEIK

(SNK0002AR Antibody heavy chain amino acid sequence)

>SEQ ID NO 19: SNK0002AR_H

MGRLTSSFLLLIVPAYVLSQVTLKESGPGILQPSQTLSLTCSFSGFSLSTFGMGVGWIR

QPSGKGLEWLAHIWWDDDKYYNPALKSRLTISKDTSKNQVFLKIANVDTADTATYYCAR

IAGFDYWGQGTTLTVSSESARNPTIYPLTLPRALSSDPVIIGCLIHDYFPSGTMNVTWG

KSGKDITTVNFPPALASGGGYTMSSQLTLPAVECPEGESVKCSVQHDSNAVQELDVKCS

GPPPPCPPCPPSCHPSLSLQRPALEDLLLGSDASLTCTLNGLRNPEGAVFTWEPSTGKD

AVQKKAVQNSCGCYSVSSVLPGCAERWNSGASFKCTVTHPESDTLTGTIAKITVNTFPP

QVHLLPPPSEELALNELVSLTCLVRAFNPKEVLVRWLHGNEELSPESYLVFEPLKEPGE

GATTYLVTSVLRVSAELWKQGDQYSCMVGHEALPMNFTQKTIDRLSGKPTNVSVSVIMS

EGDGICY

(SNK0002AR Antibody light chain amino acid sequence)

>SEQ ID NO 20: SNK0002AR_L

MHFQVQIFSFLLISASVIMSRGQIVLTQSPAIMSASPGEKVTITCSASSSVSYMHWFQQ

KPGTSPKLWIYSTSNLASGVPARFSGSGSGTSYSLTISRMEAEDAATYYCQQRSSYPYT

FGGGTKLEIKRADAAPTVSIFPPSSEQLTSGGASVVCFLNNFYPKDINVKWKIDGSERQ

NGVLNSWTDQDSKDSTYSMSSTLTLTKDEYERHNSYTCEATHKTSTSPIVKSFNRNEC

>SEQ ID NO 21: SNK0003_HCDR1
SYNIH

>SEQ ID NO 22: SNK0003_HCDR2
AIYSGNGATSHNQKFK

>SEQ ID NO 23: SNK0003_HCDR3
FCTRVGLR

>SEQ ID NO 24: SNK0003_LCDR1
KASQSVGTSVA

>SEQ ID NO 25: SNK0003_LCDR2
SASYRYS

>SEQ ID NO 26: SNK0003_LCDR3
QQYNNYPYT

>SEQ ID NO 27: SNK0003_HV

MGFSRIFLFLLSVTTGVHSQAYLQQSGAELVRPGASVRMSCKASDYTFTSYNIHWVKQT

PRQGLEWIGAIYSGNGATSHNQKFKGRATLTVDKSSSTAYMQLSSLTSEDSAVYFCTRV

GLRSPFDFWGQGTTLTVSS

>SEQ ID NO 28: SNK0003_LV

MESQTQVFVYMLLWLSGVDGDIVMTQSLKFMSTSGGDRVSVTCKASQSVGTSVAWYQQK

PGQSPKPLIYSASYRYSGVPDRFTGSGSGTDFSLTISNVQSEDLAEYFCQQYNNYPYTF

GGGTKLEIK

(SNK0003A Antibody heavy chain amino acid sequence)

>SEQ ID NO 29:SNK0003A H

MGFSRIFLFLLSVTTGVHSQAYLQQSGAELVRPGASVRMSCKASDYTFTSYNIHWVKQT

PRQGLEWIGAIYSGNGATSHNQKFKGRATLTVDKSSSTAYMQLSSLTSEDSAVYFCTRV

GLRSPFDFWGQGTTLTVSSESARNPTIYPLTLPRALSSDPVIIGCLIHDYFPSGTMNVT

WGKSGKDITTVNFPPALASGGGYTMSSQLTLPAVECPEGESVKCSVQHDSNAVQELDVK

CSGPPPPCPPCPPSCHPSLSLQRPALEDLLLGSDASLTCTLNGLRNPEGAVFTWEPSTG

KDAVQKKAVQNSCGCYSVSSVLPGCAERWNSGASFKCTVTHPESDTLTGTIAKITVNTF

PPQVHLLPPPSEELALNELVSLTCLVRAFNPKEVLVRWLHGNEELSPESYLVFEPLKEP

GEGATTYLVTSVLRVSAELWKQGDQYSCMVGHEALPMNFTQKTIDRLSGKPTNVSVSVI

MSEGDGICY

(SNK0003A Antibody light chain amino acid sequence)

>SEQ ID NO 30: SNK0003A L

MESQTQVFVYMLLWLSGVDGDIVMTQSLKFMSTSGGDRVSVTCKASQSVGTSVAWYQQK

PGQSPKPLIYSASYRYSGVPDRFTGSGSGTDFSLTISNVQSEDLAEYFCQQYNNYPYTF

GGGTKLEIKRADAAPTVSIFPPSSEQLTSGGASVVCFLNNFYPKDINVKWKIDGSERQN

GVLNSWTDQDSKDSTYSMSSTLTLTKDEYERHNSYTCEATHKTSTSPIVKSFNRNEC

>SEQ ID NO 31: W27G2_HCDR1
DYYIH

>SEQ ID NO 32: W27G2_HCDR2
RIDPENDETTYAPKFQ

>SEQ ID NO 33: W27G2_HCDR3
YCARSTVL

>SEQ ID NO 34: W27G2_LCDR1
RASQSIVHTNG

>SEQ ID NO 35: W27G2_LCDR2
KLLIYKV

>SEQ ID NO 36: W27G2_LCDR3
GVYYCFQGS

>SEQ ID NO 37:W27G2_HV

MKCSWIIFFLMAVVTGVNSEVQLQQSGSELVKSGASVKLSCTVSGFNFTDYYIHWVRQR

TEQGLEWIGRIDPENDETTYAPKFQGKATMTADTSSNTAYLQLTSLTSEDTAVYYCARS

TVLDYWGHGTTLTVSS

>SEQ ID NO 38:W27G2_LV

MKLPVRLLVLMFWIPGFSSDVLMTQTPLSLPVSLGDQASISCRASQSIVHTNGNTYLEW

YLQKPGQSPKLLIYKVSNRFSGVPDRFSGSGSGTDFILKISRVEAEDLGVYYCFQGSHV

PPTFGGGTKLEVK

(Mouse W27G2 hybridoma-produced antibody heavy chain amino acid sequence)

>SEQ ID NO 39:W27G2_H

MKCSWIIFFLMAVVTGVNSEVQLQQSGSELVKSGASVKLSCTVSGFNFTDYYIHWVRQR

TEQGLEWIGRIDPENDETTYAPKFQGKATMTADTSSNTAYLQLTSLTSEDTAVYYCARS

TVLDYWGHGTTLTVSSESARNPTIYPLTLPRALSSDPVIIGCLIHDYFPSGTMNVTWGK

SGKDITTVNFPPALASGGGYTMSSQLTLPAVECPEGESVKCSVQHDSNAVQELDVKCSG

PPPPCPPCPPSCHPSLSLQRPALEDLLLGSDASLTCTLNGLRNPEGAVFTWEPSTGKDA

VQKKAVQNSCGCYSVSSVLPGCAERWNSGASFKCTVTHPESDTLTGTIAKITVNTFPPQ

VHLLPPPSEELALNELVSLTCLVRAFNPKEVLVRWLHGNEELSPESYLVFEPLKEPGEG

ATTYLVTSVLRVSAELWKQGDQYSCMVGHEALPMNFTQKTIDRLSGKPTNVSVSVIMSE

GDGICY

(Mouse W27G2 hybridoma-produced antibody light chain amino acid sequence)

>SEQ ID NO 40:W27G2_L

MKLPVRLLVLMFWIPGFSSDVLMTQTPLSLPVSLGDQASISCRASQSIVHTNGNTYLEW

YLQKPGQSPKLLIYKVSNRFSGVPDRFSGSGSGTDFILKISRVEAEDLGVYYCFQGSHV

PPTFGGGTKLEVKRADAAPTVSIFPPSSEQLTSGGASVVCFLNNFYPKDINVKWKIDGS

ERQNGVLNSWTDQDSKDSTYSMSSTLTLTKDEYERHNSYTCEATHKTSTSPIVKSFNRN

EC

>SEQ ID NO 41:RS_HCDR1_D50N
NYYIH

>SEQ ID NO 42:RS_HCDR2_D75N
RIDPENNETTYAPKFQ

>SEQ ID NO 43:RS_HCDR2_E76Q
RIDPENDQTTYAPKFQ

>SEQ ID NO 44:RS_HCDR2_D75N_E76Q
RIDPENNQTTYAPKFQ

>SEQ ID NO 45:RS_HV001

MKCSWIIFFLMAVVTGVNSEVQLQQSGSELVKSGASVKLSCTVSGFNFTNYYIHWVRQR

TEQGLEWIGRIDPENDETTYAPKFQGKATMTADTSSNTAYLQLTSLTSEDTAVYYCARS

TVLDYWGHGTTLTVSS

>SEQ ID NO 46:RS_HV002

MKCSWIIFFLMAVVTGVNSEVQLQQSGSELVKSGASVKLSCTVSGFNFTDYYIHWVRQR

TEQGLEWIGRIDPENNETTYAPKFQGKATMTADTSSNTAYLQLTSLTSEDTAVYYCARS

TVLDYWGHGTTLTVSS

>SEQ ID NO 47:RS_HV003

MKCSWIIFFLMAVVTGVNSEVQLQQSGSELVKSGASVKLSCTVSGFNFTDYYIHWVRQR

TEQGLEWIGRIDPENDQTTYAPKFQGKATMTADTSSNTAYLQLTSLTSEDTAVYYCARS

TVLDYWGHGTTLTVSS

>SEQ ID NO 48:RS_HV004

MKCSWIIFFLMAVVTGVNSEVQLQQSGSELVKSGASVKLSCTVSGFNFTNYYIHWVRQR

TEQGLEWIGRIDPENNETTYAPKFQGKATMTADTSSNTAYLQLTSLTSEDTAVYYCARS

TVLDYWGHGTTLTVSS

>SEQ ID NO 49:RS_HV005

MKCSWIIFFLMAVVTGVNSEVQLQQSGSELVKSGASVKLSCTVSGFNFTNYYIHWVRQR

TEQGLEWIGRIDPENDQTTYAPKFQGKATMTADTSSNTAYLQLTSLTSEDTAVYYCARS

TVLDYWGHGTTLTVSS

>SEQ ID NO 50:RS_HV006

MKCSWIIFFLMAVVTGVNSEVQLQQSGSELVKSGASVKLSCTVSGFNFTDYYIHWVRQR

TEQGLEWIGRIDPENNQTTYAPKFQGKATMTADTSSNTAYLQLTSLTSEDTAVYYCARS

TVLDYWGHGTTLTVSS

>SEQ ID NO 51:RS_HV007

MKCSWIIFFLMAVVTGVNSEVQLQQSGSELVKSGASVKLSCTVSGFNFTNYYIHWVRQR

TEQGLEWIGRIDPENNQTTYAPKFQGKATMTADTSSNTAYLQLTSLTSEDTAVYYCARS

TVLDYWGHGTTLTVSS

>SEQ ID NO 52: RS_LCDR1_A44S
RSSQSIVHTNG

>SEQ ID NO 53: RS_LFR1_L001
SPASLSVSLGDRA

>SEQ ID NO 54: RS_LFR1_L002

SPASLSVSLGDRV

>SEQ ID NO 55: RS_LFR1_L003
SPASMSVSLGDRA

>SEQ ID NO 56: RS_LFR1_L000
TPLSLPVSLGDQA

>SEQ ID NO 57:RS_LV001

MKLPVRLLVLMFWIPGFSSDVLMTQSPASLSVSLGDRATISCRASQSIVHTNGNTYLEW

YLQKPGQSPKLLIYKVSNRFSGVPDRFSGSGSGTDFILKISRVEAEDLGVYYCFQGSHV

PPTFGGGTKLEVK

>SEQ ID NO 58:RS_LV002

MKLPVRLLVLMFWIPGFSSDVLMTQSPASLSVSLGDRVTISCRASQSIVHTNGNTYLEW

YLQKPGQSPKLLIYKVSNRFSGVPDRFSGSGSGTDFILKISRVEAEDLGVYYCFQGSHV

PPTFGGGTKLEVK

>SEQ ID NO 59:RS_LV003

MKLPVRLLVLMFWIPGFSSDVLMTQSPASLSVSLGDRVTISCRSSQSIVHTNGNTYLEW

YLQKPGQSPKLLIYKVSNRFSGVPDRFSGSGSGTDFILKISRVEAEDLGVYYCFQGSHV

PPTFGGGTKLEVK

>SEQ ID NO 60:RS_LV004

MKLPVRLLVLMFWIPGFSSDVLMTQSPASMSVSLGDRATISCRSSQSIVHTNGNTYLEW

YLQKPGQSPKLLIYKVSNRFSGVPDRFSGSGSGTDFILKISRVEAEDLGVYYCFQGSHV

PPTFGGGTKLEVK

>SEQ ID NO 61:RS_LV005

MKLPVRLLVLMFWIPGFSSDVLMTQSPASLSVSLGDRATISCRSSQSIVHTNGNTYLEW

YLQKPGQSPKLLIYKVSNRFSGVPDRFSGSGSGTDFILKISRVEAEDLGVYYCFQGSHV

PPTFGGGTKLEVK

>SEQ ID NO 62: RS_HCDR1_X
XYYIH

>SEQ ID NO 63: RS_HCDR2_X
RIDPENXXTTYAPKFQ

>SEQ ID NO 64: RS_LCDR1_X
RXSQSIVHTNG
(SNK0001AR Antibody heavy chain cDNA sequence)

>SEQ ID NO 65: SNK0001AR_H_CDNA

ATGGAATGGATCTGGATCTTTCTCTTCATCCTGTCAGGAACTGCAGGTGTCCAATCCCA

GGTTCAGCTGCAGCAGTCTGGAGCTGAGCTGGCGAGGCCTGGGGCTTCAGTGAAGCTGT

CCTGCAAGGCTTCTGGCTACACCTTCACAAGCTATGGTATAAGCTGGGTGAAGCAGAGA

ACTGGACAGGGCCTTGAGTGGATTGGAGAGATTTATCCTAGAAGTGGTAATACTTACTA

CAATGAGAAGTTCAAGGGCAAGGCCACACTGACTGCAGACAAATCCTCCAGCACAGCGT

ACATGGAGCTCCGCAGCCTGACATCTGAGGACTCTGCGGTCTATTTCTGTGCAAGATTG

GCATCGAGCTACTACGGTAGTAGCTACGACTGGTACTTCGATGTCTGGGGCACAGGGAC

CACGGTCACCGTCTCCTCAGAGTCTGCGAGAAATCCCACCATCTACCCACTGACACTCC

CACGAGCTCTGTCAAGTGACCCAGTGATAATCGGCTGCCTGATTCACGATTACTTCCCT

TCCGGCACGATGAATGTGACCTGGGGAAAGAGTGGGAAGGATATAACCACCGTAAACTT

CCCACCTGCCCTGGCCTCTGGGGGAGGGTACACCATGAGCAGCCAGTTGACCCTGCCAG

CTGTCGAGTGCCCAGAAGGAGAATCCGTGAAATGTTCCGTGCAACATGACTCTAACGCC

GTCCAAGAATTGGATGTGAAGTGCTCTGGTCCTCCTCCTCCTTGTCCTCCTTGTCCTCC

TTCCTGCCATCCCAGCCTGTCACTGCAGCGGCCAGCTCTTGAGGACCTGCTCCTGGGTT

CAGATGCCAGCCTCACATGTACTCTGAATGGCCTGAGAAATCCTGAGGGAGCTGTCTTC

ACCTGGGAGCCCTCCACTGGGAAGGATGCAGTGCAGAAGAAAGCTGTGCAGAATTCCTG

CGGCTGCTACAGTGTGTCCAGCGTCCTGCCTGGCTGTGCTGAGCGCTGGAACAGTGGCG

CATCATTCAAGTGCACAGTTACCCATCCTGAGTCTGACACCTTAACTGGCACAATTGCC

AAAATCACAGTGAACACCTTCCCACCCCAGGTCCACCTGCTACCGCCGCCGTCGGAGGA

GCTGGCCCTGAATGAGCTCGTGTCCCTGACATGCCTGGTGCGAGCTTTCAACCCTAAAG

AAGTGCTGGTGCGATGGCTGCATGGAAATGAGGAGCTGTCCCCAGAAAGCTACCTAGTG

TTTGAGCCCCTAAAGGAGCCAGGCGAGGGAGCCACCACCTACCTGGTGACAAGCGTGTT

GCGTGTATCAGCTGAACTCTGGAAACAGGGTGACCAGTACTCCTGCATGGTGGGCCACG

AGGCCTTGCCCATGAACTTCACCCAGAAGACCATCGACCGTCTGTCGGGTAAACCCACC

AATGTCAGCGTGTCTGTGATCATGTCAGAGGGAGATGGCATCTGCTACTGA

(SNK0001AR Antibody light chain cDNA sequence)

>SEQ ID NO 66:SNK0001AR_L_CDNA

ATGAGGACCCCTGCTCAGTTTCTTGGAATCTTGTTGCTCTGGTTTCCAGGTATCAAATG

TGACATCAAGATGACCCAGTCTCCATCTTCCATGTATGCATCTCTAGGAGAGAGAGTCA

CTATCACTTGCAAGGCGAGTCAGGACATTAATAGCTATTTAAGCTGGTTCCAGCAGAAA

CCAGGGAAATCTCCTAAGACCCTGATCTATCGTGCAAACAGATTGGTAGATGGGGTCCC

ATCAAGGTTCAGTGGCAGTGGATCTGGGCAAGATTATTCTCTCACCATCAGCAGCCTGG

AGTATGAAGATATGGGAATTTATTATTGTCTACAGTATGATGAGTTTCCGCTCACGTTC

GGTGCTGGGACCAAGCTGGAGCTGAAACGGGCTGATGCTGCACCAACTGTATCCATCTT

CCCACCATCCAGTGAGCAGTTAACATCTGGAGGTGCCTCAGTCGTGTGCTTCTTGAACA

ACTTCTACCCCAAAGACATCAATGTCAAGTGGAAGATTGATGGCAGTGAACGACAAAAT

GGCGTCCTGAACAGTTGGACTGATCAGGACAGCAAAGACAGCACCTACAGCATGAGCAG

CACCCTCACGTTGACCAAGGACGAGTATGAACGACATAACAGCTATACCTGTGAGGCCA

CTCACAAGACATCAACTTCACCCATTGTCAAGAGCTTCAACAGGAATGAGTGTTGA

(SNK0002AR Antibody heavy chain cDNA sequence)

>SEQ ID NO 67: SNK0002AR_H_CDNA

ATGGGCAGGCTTACTTCTTCATTCCTGTTACTGATTGTCCCTGCATATGTCCTGTCCCA

GGTTACTCTGAAAGAGTCTGGCCCTGGGATATTGCAGCCCTCCCAGACCCTCAGTCTGA

CTTGTTCTTTCTCTGGGTTTTCACTGAGCACTTTTGGTATGGGTGTAGGCTGGATTCGT

CAGCCTTCAGGGAAGGGTCTGGAGTGGCTGGCACACATTTGGTGGGATGATGATAAGTA

CTATAACCCAGCCCTGAAGAGTCGGCTCACAATCTCCAAGGATACCTCCAAAAACCAGG

TATTCCTCAAGATCGCCAATGTGGACACTGCAGATACTGCCACATACTACTGTGCTCGA

ATAGCTGGGTTTGACTACTGGGGCCAAGGCACCACTCTCACAGTCTCCTCAGAGTCTGC

GAGAAATCCCACCATCTACCCACTGACACTCCCACGAGCTCTGTCAAGTGACCCAGTGA

TAATCGGCTGCCTGATTCACGATTACTTCCCTTCCGGCACGATGAATGTGACCTGGGGA

AAGAGTGGGAAGGATATAACCACCGTAAACTTCCCACCTGCCCTGGCCTCTGGGGGAGG

GTACACCATGAGCAGCCAGTTGACCCTGCCAGCTGTCGAGTGCCCAGAAGGAGAATCCG

TGAAATGTTCCGTGCAACATGACTCTAACGCCGTCCAAGAATTGGATGTGAAGTGCTCT

GGTCCTCCTCCTCCTTGTCCTCCTTGTCCTCCTTCCTGCCATCCCAGCCTGTCACTGCA

GCGGCCAGCTCTTGAGGACCTGCTCCTGGGTTCAGATGCCAGCCTCACATGTACTCTGA

ATGGCCTGAGAAATCCTGAGGGAGCTGTCTTCACCTGGGAGCCCTCCACTGGGAAGGAT

GCAGTGCAGAAGAAAGCTGTGCAGAATTCCTGCGGCTGCTACAGTGTGTCCAGCGTCCT

GCCTGGCTGTGCTGAGCGCTGGAACAGTGGCGCATCATTCAAGTGCACAGTTACCCATC

CTGAGTCTGACACCTTAACTGGCACAATTGCCAAAATCACAGTGAACACCTTCCCACCC

CAGGTCCACCTGCTACCGCCGCCGTCGGAGGAGCTGGCCCTGAATGAGCTCGTGTCCCT

GACATGCCTGGTGCGAGCTTTCAACCCTAAAGAAGTGCTGGTGCGATGGCTGCATGGAA

ATGAGGAGCTGTCCCCAGAAAGCTACCTAGTGTTTGAGCCCCTAAAGGAGCCAGGCGAG

GGAGCCACCACCTACCTGGTGACAAGCGTGTTGCGTGTATCAGCTGAACTCTGGAAACA

GGGTGACCAGTACTCCTGCATGGTGGGCCACGAGGCCTTGCCCATGAACTTCACCCAGA

AGACCATCGACCGTCTGTCGGGTAAACCCACCAATGTCAGCGTGTCTGTGATCATGTCA

GAGGGAGATGGCATCTGCTACTGA

(SNK0002AR Antibody light chain cDNA sequence)

>SEQ ID NO 68: SNK0002AR_L_CDNA

ATGCATTTTCAAGTGCAGATTTTCAGCTTCCTGCTAATCAGTGCCTCAGTCATAATGTC

CAGAGGACAAATTGTTCTCACCCAGTCTCCAGCAATCATGTCTGCATCTCCAGGGGAGA

AGGTCACCATAACCTGCAGTGCCAGCTCAAGTGTAAGTTACATGCACTGGTTCCAGCAG

AAGCCAGGCACTTCTCCCAAACTCTGGATTTATAGCACATCCAACCTGGCTTCTGGAGT

CCCTGCTCGCTTCAGTGGCAGTGGATCTGGGACCTCTTACTCTCTCACAATCAGCCGAA

TGGAGGCTGAAGATGCTGCCACTTATTACTGCCAGCAAAGGAGTAGTTACCCGTACACG

TTCGGAGGGGGGACCAAGCTGGAAATAAAACGGGCTGATGCTGCACCAACTGTATCCAT

CTTCCCACCATCCAGTGAGCAGTTAACATCTGGAGGTGCCTCAGTCGTGTGCTTCTTGA

ACAACTTCTACCCCAAAGACATCAATGTCAAGTGGAAGATTGATGGCAGTGAACGACAA

AATGGCGTCCTGAACAGTTGGACTGATCAGGACAGCAAAGACAGCACCTACAGCATGAG

CAGCACCCTCACGTTGACCAAGGACGAGTATGAACGACATAACAGCTATACCTGTGAGG

CCACTCACAAGACATCAACTTCACCCATTGTCAAGAGCTTCAACAGGAATGAGTGTTGA

(SNK0003A Antibody heavy chain cDNA sequence)

>SEQ ID NO 69: SNK0003A_H_CDNA

ATGGGATTCAGCAGGATCTTTCTCTTCCTCCTGTCAGTAACTACAGGTGTCCACTCCCA

GGCTTATCTACAGCAGTCTGGGGCTGAGCTGGTGAGGCCTGGGGCCTCAGTGAGGATGT

CCTGCAAGGCTTCTGACTACACATTTACCAGTTACAATATCCACTGGGTAAAGCAGACA

CCTAGACAGGGCCTGGAATGGATTGGAGCTATTTATTCAGGAAATGGTGCTACTTCCCA

CAATCAGAAGTTCAAGGGCAGGGCCACACTGACTGTAGACAAATCCTCCAGCACAGCCT

ACATGCAACTCAGCAGCCTGACATCTGAAGACTCTGCGGTCTATTTCTGTACAAGAGTG

GGACTCCGGTCCCCTTTTGACTTCTGGGGCCAAGGCACCACTCTCACAGTCTCCTCAGA

GTCTGCGAGAAATCCCACCATCTACCCACTGACACTCCCACGAGCTCTGTCAAGTGACC

CAGTGATAATCGGCTGCCTGATTCACGATTACTTCCCTTCCGGCACGATGAATGTGACC

TGGGGAAAGAGTGGGAAGGATATAACCACCGTAAACTTCCCACCTGCCCTGGCCTCTGG

GGGAGGGTACACCATGAGCAGCCAGTTGACCCTGCCAGCTGTCGAGTGCCCAGAAGGAG

AATCCGTGAAATGTTCCGTGCAACATGACTCTAACGCCGTCCAAGAATTGGATGTGAAG

TGCTCTGGTCCTCCTCCTCCTTGTCCTCCTTGTCCTCCTTCTGCCATCCCAGCCTGTC

ACTGCAGCGGCCAGCTCTTGAGGACCTGCTCCTGGGTTCAGATGCCAGCCTCACATGTA

CTCTGAATGGCCTGAGAAATCCTGAGGGAGCTGTCTTCACCTGGGAGCCCTCCACTGGG

AAGGATGCAGTGCAGAAGAAAGCTGTGCAGAATTCCTGCGGCTGCTACAGTGTGTCCAG

CGTCCTGCCTGGCTGTGCTGAGCGCTGGAACAGTGGCGCATCATTCAAGTGCACAGTTA

CCCATCCTGAGTCTGACACCTTAACTGGCACAATTGCCAAAATCACAGTGAACACCTTC

CCACCCCAGGTCCACCTGCTACCGCCGCCGTCGGAGGAGCTGGCCCTGAATGAGCTCGT

GTCCCTGACATGCCTGGTGCGAGCTTTCAACCCTAAAGAAGTGCTGGTGCGATGGCTGC

ATGGAAATGAGGAGCTGTCCCCAGAAAGCTACCTAGTGTTTGAGCCCCTAAAGGAGCCA

GGCGAGGGAGCCACCACCTACCTGGTGACAAGCGTGTTGCGTGTATCAGCTGAACTCTG

GAAACAGGGTGACCAGTACTCCTGCATGGTGGGCCACGAGGCCTTGCCCATGAACTTCA

CCCAGAAGACCATCGACCGTCTGTCGGGTAAACCCACCAATGTCAGCGTGTCTGTGATC

ATGTCAGAGGGAGATGGCATCTGCTACTGA

(SNK0003A Antibody light chain cDNA sequence)

>SEQ ID NO 70: SNK0003A_L_CDNA

ATGGAGTCACAGACTCAGGTCTTTGTATACATGTTGCTGTGGTTGTCTGGTGTTGATGG

AGACATTGTGATGACCCAGTCTCTAAAATTCATGTCCACATCGGGAGGAGACAGGGTCA

GCGTCACCTGCAAGGCCAGTCAGAGTGTGGGTACAAGTGTAGCCTGGTATCAGCAGAAA

CCAGGACAATCTCCTAAACCACTGATTTATTCGGCATCTTATCGGTACAGTGGAGTCCC

TGATCGCTTCACAGGCAGTGGATCTGGGACAGATTTCAGTCTCACCATCAGCAATGTGC

AGTCTGAAGACTTGGCAGAATATTTCTGTCAGCAATATAACAACTATCCGTACACGTTC

GGAGGGGGGACCAAGCTGGAGATAAAACGGGCTGATGCTGCACCAACTGTATCCATCTT

CCCACCATCCAGTGAGCAGTTAACATCTGGAGGTGCCTCAGTCGTGTGCTTCTTGAACA

ACTTCTACCCCAAAGACATCAATGTCAAGTGGAAGATTGATGGCAGTGAACGACAAAAT

GGCGTCCTGAACAGTTGGACTGATCAGGACAGCAAAGACAGCACCTACAGCATGAGCAG

CACCCTCACGTTGACCAAGGACGAGTATGAACGACATAACAGCTATACCTGTGAGGCCA

CTCACAAGACATCAACTTCACCCATTGTCAAGAGCTTCAACAGGAATGAGTGTTGA

(Mouse W27G2 hybridoma-produced antibody heavy chain cDNA sequence)

>SEQ ID NO 71:W27G_MOUSE_IGA_H_CDNA

ATGAAATGCAGCTGGATCATCTTCTTCCTGATGGCAGTGGTTACAGGGGTCAATTCAGA

GGTTCAGCTGCAGCAGTCTGGGTCAGAGCTTGTGAAGTCTGGGGCCTCAGTCAAGTTGT

CCTGCACAGTTTCTGGGTTCAACTTTACAGACTACTATACACTGGGTGAGGCAGAGG

ACTGAACAGGGCCTGGAATGGATTGGAAGGATTGATCCTGAGAATGATGAAACTACATA

```
TGCCCCGAAATTCCAGGGCAAGGCCACTATGACAGCAGACACATCTTCCAACACAGCCT

ACCTGCAGCTCACCAGCCTGACATCTGAAGACACTGCCGTCTATTACTGTGCTAGATCT

ACGGTCCTTGACTACTGGGGCCACGGCACCACTCTCACAGTCTCCTCAGAGTCTGCGAG

AAATCCCACCATCTACCCACTGACACTCCCACGAGCTCTGTCAAGTGACCCAGTGATAA

TCGGCTGCCTGATTCACGATTACTTCCCTTCCGGCACGATGAATGTGACCTGGGGAAAG

AGTGGGAAGGATATAACCACCGTAAACTTCCCACCTGCCCTGGCCTCTGGGGGAGGGTA

CACCATGAGCAGCCAGTTGACCCTGCCAGCTGTCGAGTGCCCAGAAGGAGAATCCGTGA

AATGTTCCGTGCAACATGACTCTAACGCCGTCCAAGAATTGGATGTGAAGTGCTCTGGT

CCTCCTCCTCCTTGTCCTCCTTGTCCTCCTTCCTGCCATCCCAGCCTGTCACTGCAGCG

GCCAGCTCTTGAGGACCTGCTCCTGGGTTCAGATGCCAGCCTCACATGTACTCTGAATG

GCCTGAGAAATCCTGAGGGAGCTGTCTTCACCTGGGAGCCCTCCACTGGGAAGGATGCA

GTGCAGAAGAAAGCTGTGCAGAATTCCTGCGGCTGCTACAGTGTGTCCAGCGTCCTGCC

TGGCTGTGCTGAGCGCTGGAACAGTGGCGCATCATTCAAGTGCACAGTTACCCATCCTG

AGTCTGACACCTTAACTGGCACAATTGCCAAAATCACAGTGAACACCTTCCCACCCCAG

GTCCACCTGCTACCGCCGCCGTCGGAGGAGCTGGCCCTGAATGAGCTCGTGTCCCTGAC

ATGCCTGGTGCGAGCTTTCAACCCTAAAGAAGTGCTGGTGCGATGGCTGCATGGAAATG

AGGAGCTGTCCCCAGAAAGCTACCTAGTGTTTGAGCCCCTAAAGGAGCCAGGCGAGGGA

GCCACCACCTACCTGGTGACAAGCGTGTTGCGTGTATCAGCTGAACTCTGGAAACAGGG

TGACCAGTACTCCTGCATGGTGGGCCACGAGGCCTTGCCCATGAACTTCACCCAGAAGA

CCATCGACCGTCTGTCGGGTAAACCCACCAATGTCAGCGTGTCTGTGATCATGTCAGAG

GGAGATGGCATCTGCTACTGA
```

(Mouse W27G2 hybridoma-produced antibody light chain cDNA sequence)

>SEQ ID NO 72:W27G MOUSE_IGA_L_CDNA

ATGAAGTTGCCTGTTAGGCTGTTGGTGCTGATGTTCTGGATTCCTGGATTCAGCAGTGA

TGTTTTGATGACCCAAACTCCACTCTCCCTGCCTGTCAGTCTTGGAGATCAAGCCTCCA

TCTCTTGTAGAGCTAGTCAGAGCATTGTACACACTAATGGGAACACCTATTTAGAATGG

TACCTGCAGAAACCAGGCCAGTCTCCAAAGCTCCTGATCTACAAAGTTTCCAACCGATT

TTCTGGGGTCCCAGACAGGTTCAGTGGCAGTGGATCTGGGACAGATTTCATACTCAAGA

TCAGCAGAGTGGAGGCTGAGGATCTGGGAGTTTATTACTGCTTTCAAGGTTCACATGTT

CCTCCGACGTTCGGTGGAGGCACCAAGCTGGAAGTCAAACGGGCTGATGCTGCACCAAC

TGTATCCATCTTCCCACCATCCAGTGAGCAGTTAACATCTGGAGGTGCCTCAGTCGTGT

GCTTCTTGAACAACTTCTACCCCAAAGACATCAATGTCAAGTGGAAGATTGATGGCAGT

GAACGACAAAATGGCGTCCTGAACAGTTGGACTGATCAGGACAGCAAAGACAGCACCTA

CAGCATGAGCAGCACCCTCACGTTGACCAAGGACGAGTATGAACGACATAACAGCTATA

CCTGTGAGGCCACTCACAAGACATCAACTTCACCCATTGTCAAGAGCTTCAACAGGAAT

GAGTGTTAG

(Mouse W27G2 IGG antibody heavy chain cDNA sequence)

>SEQ ID NO 73:W27G2 MOUSE_IGG_H_CDNA

ATGAAATGCAGCTGGATCATCTTCTTCCTGATGGCAGTGGTTACAGGGGTCAATTCAGA

GGTTCAGCTGCAGCAGTCTGGGTCAGAGCTTGTGAAGTCTGGGGCCTCAGTCAAGTTGT

CCTGCACAGTTTCTGGGTTCAACTTTACAGACTACTATACACTGGGTGAGGCAGAGG

ACTGAACAGGGCCTGGAATGGATTGGAAGGATTGATCCTGAGAATGATGAAACTACATA

TGCCCCGAAATTCCAGGGCAAGGCCACTATGACAGCAGACACATCTTCCAACACAGCCT

ACCTGCAGCTCACCAGCCTGACATCTGAAGACACTGCCGTCTATTACTGTGCTAGATCT

ACGGTCCTTGACTACTGGGGCCACGGCACCACTCTCACAGTCTCCTCAAAAACGACACC

CCCATCTGTCTATCCACTGGCCCCTGGATCTGCTGCCCAAACTAACTCCATGGTGACCC

TGGGATGCCTGGTCAAGGGCTATTTCCCTGAGCCAGTGACAGTGACCTGGAACTCTGGA

TCCCTGTCCAGCGGTGTGCACACCTTCCCAGCTGTCCTGCAGTCTGACCTCTACACTCT

GAGCAGCTCAGTGACTGTCCCCTCCAGCACCTGGCCCAGCGAGACCGTCACCTGCAACG

TTGCCCACCCGGCCAGCAGCACCAAGGTGGACAAGAAAATTGTGCCCAGGGATTGTGGT

TGTAAGCCTTGCATATGTACAGTCCCAGAAGTATCATCTGTCTTCATCTTCCCCCCAAA

GCCCAAGGATGTGCTCACCATTACTCTGACTCCTAAGGTCACGTGTGTTGTGGTAGACA

TCAGCAAGGATGATCCCGAGGTCCAGTTCAGCTGGTTTGTAGATGATGTGGAGGTGCAC

ACAGCTCAGACGCAACCCCGGGAGGAGCAGTTCAACAGCACTTTCCGCTCAGTCAGTGA

ACTTCCCATCATGCACCAGGACTGGCTCAATGGCAAGGAGTTCAAATGCAGGGTCAACA

GTGCAGCTTTCCCTGCCCCCATCGAGAAAACCATCTCCAAAACCAAAGGCAGACCGAAG

GCTCCACAGGTGTACACCATTCCACCTCCCAAGGAGCAGATGGCCAAGGATAAAGTCAG

TCTGACCTGCATGATAACAGACTTCTTCCCTGAAGACATTACTGTGGAGTGGCAGTGGA

ATGGGCAGCCAGCGGAGAACTACAAGAACACTCAGCCCATCATGGACACAGATGGCTCT

TACTTCGTCTACAGCAAGCTCAATGTGCAGAAGAGCAACTGGGAGGCAGGAAATACTTT

CACCTGCTCTGTGTTACATGAGGGCCTGCACAACCACCATACTGAGAAGAGCCTCTCCC

ACTCTCCTGGTAAAtaa

(Human chimeric W27G2 IgG antibody heavy chain cDNA sequence)

>SEQ ID NO 74: W27G2_HUMAN_CHIMERA_IGG_H_CDNA

ATGAAATGCAGCTGGATCATCTTCTTCCTGATGGCAGTGGTTACAGGGGTCAATTCAGA

GGTTCAGCTGCAGCAGTCTGGGTCAGAGCTTGTGAAGTCTGGGGCCTCAGTCAAGTTGT

CCTGCACAGTTTCTGGGTTCAACTTTACAGACTACTATACACTGGGTGAGGCAGAGG

ACTGAACAGGGCCTGGAATGGATTGGAAGGATTGATCCTGAGAATGATGAAACTACATA

TGCCCCGAAATTCCAGGGCAAGGCCACTATGACAGCAGACACATCTTCCAACACAGCCT

ACCTGCAGCTCACCAGCCTGACATCTGAAGACACTGCCGTCTATTACTGTGCTAGATCT

ACGGTCCTTGACTACTGGGGCCACGGCACCACTCTCACAGTCTCCTCAGCATCCCCGAC

TAGTTCCACCAAGGGCCCATCGGTCTTCCCCCTGGCACCCTCCTCCAAGAGCACCTCTG

GGGGCACAGCGGCCCTGGGCTGCCTGGTCAAGGACTACTTCCCCGAACCGGTGACGGTG

TCGTGGAACTCAGGCGCCCTGACCAGCGGCGTGCACACCTTCCCGGCTGTCCTACAGTC

CTCAGGACTCTACTCCCTCAGCAGCGTGGTGACCGTGCCCTCCAGCAGCTTGGGCACCC

AGACCTACATCTGCAACGTGAATCACAAGCCCAGCAACACCAAGGTGGACAAGAAAGTT

GAGCCCAAATCTTGTGACAAAACTCACACATGCCCACCGTGCCCAGCACCTGAACTCCT

GGGGGGACCGTCAGTCTTCCTCTTCCCCCCAAAACCCAAGGACACCCTCATGATCTCCC

GGACCCCTGAGGTCACATGCGTGGTGGTGGACGTGAGCCACGAAGACCCTGAGGTCAAG

TTCAACTGGTACGTGGACGGCGTGGAGGTGCATAATGCCAAGACAAAGCCGCGGGAGGA

GCAGTACAACAGCACGTACCGTGTGGTCAGCGTCCTCACCGTCCTGCACCAGGACTGGC

TGAATGGCAAGGAGTACAAGTGCAAGGTCTCCAACAAAGCCCTCCCAGCCCCTATCGAG

AAAACCATCTCCAAAGCCAAAGGGCAGCCCCGAGAACCACAGGTGTACACCCTGCCCCC

ATCCCGGGATGAGCTGACCAAGAACCAGGTCAGCCTGACCTGCCTGGTCAAAGGCTTCT

ATCCCAGCGACATCGCCGTGGAGTGGGAGAGCAATGGGCAGCCGGAGAACAACTACAAG

ACCACGCCTCCCGTGCTGGACTCCGACGGCTCCTTCTTCCTCTACAGCAAGCTCACCGT

GGACAAGAGCAGGTGGCAGCAGGGGAACGTCTTCTCATGCTCCGTGATGCATGAGGGTC

TGCACAACCACTACACGCAGAAGAGCCTCTCCCTGTCTCCGGGTAAATGA

(Human chimeric W27G2 IgG antibody light chain cDNA sequence)

>SEQ ID NO 75: W27G2_HUMAN_CHIMERA_IGG_L_CDNA

ATGGACATGAAGTTGCCTGTTAGGCTGTTGGTGCTGATGTTCTGGATTCCTGGATTCAG

CAGTGATGTTTTGATGACCCAAACTCCACTCTCCCTGCCTGTCAGTCTTGGAGATCAAG

CCTCCATCTCTTGTAGAGCTAGTCAGAGCATTGTACACTAATGGGAACACCTATTTA

GAATGGTACCTGCAGAAACCAGGCCAGTCTCCAAAGCTCCTGATCTACAAAGTTTCCAA

CCGATTTTCTGGGGTCCCAGACAGGTTCAGTGGCAGTGGATCTGGGACAGATTTCATAC

TCAAGATCAGCAGAGTGGAGGCTGAGGATCTGGGAGTTTATTACTGCTTTCAAGGTTCA

CATGTTCCTCCGACGTTCGGTGGAGGCACCAAGCTGGAGTCAAACGGGCTGATGCTGC

ACCAACTGTATCCATCTTCCACCATCCAGTGAGCAGTTAACATCTGGAGGTGCCTCTG

TTGTGTGCCTGCTGAATAACTTCTATCCTCGAGAGGCCAAAGTACAGTGGAAGGTGGAT

AACGCCCTCCAATCGGGTAACTCCCAGGAGAGTGTCACAGAGCAGGACAGCAAGGACAG

CACCTACAGCCTCAGCAGCACCCTGACGCTGAGCAAAGCAGACTACGAGAACACAAAG

TCTACGCCTGCGAAGTCACCCATCAGGGCCTGAGCTCGCCCGTCACAAAGAGCTTCAAC

AGGGGAGAGTGTTAG

(Mouse W27G2 IGG antibody heavy chain amino acid sequence)

>SEQ ID NO 76: W27G2_MOUSE_IGG_H

MKCSWIIFFLMAVVTGVNSEVQLQQSGSELVKSGASVKLSCTVSGFNFTDYYIHWVRQR

TEQGLEWIGRIDPENDETTYAPKFQGKATMTADTSSNTAYLQLTSLTSEDTAVYYCARS

TVLDYWGHGTTLTVSSKTTPPSVYPLAPGSAAQTNSMVTLGCLVKGYFPEPVTVTWNSG

SLSSGVHTFPAVLQSDLYTLSSSVTVPSSTWPSETVTCNVAHPASSTKVDKKIVPRDCG

CKPCICTVPEVSSVFIFPPKPKDVLTITLTPKVTCVVVDISKDDPEVQFSWFVDDVEVH

TAQTQPREEQFNSTFRSVSELPIMHQDWLNGKEFKCRVNSAAFPAPIEKTISKTKGRPK

APQVYTIPPPKEQMAKDKVSLTCMITDFFPEDITVEWQWNGQPAENYKNTQPIMDTDGS

YFVYSKLNVQKSNWEAGNTFTCSVLHEGLHNHHTEKSLSHSPGK

(Human chimeric W27G2 IgG antibody heavy chain amino acid sequence)

>SEQ ID NO 77:W27G2_HUMAN _CHIMERA_IGG_H

MKCSWIIFFLMAVVTGVNSEVQLQQSGSELVKSGASVKLSCTVSGFNFTDYYIHWVRQR

TEQGLEWIGRIDPENDETTYAPKFQGKATMTADTSSNTAYLQLTSLTSEDTAVYYCARS

TVLDYWGHGTTLTVSSASPTSSTKGPSVFPLAPSSKSTSGGTAALGCLVKDYFPEPVTV

SWNSGALTSGVHTFPAVLQSSGLYSLSSVVTVPSSSLGTQTYICNVNHKPSNTKVDKKV

EPKSCDKTHTCPPCPAPELLGGPSVFLFPPKPKDTLMISRTPEVTCVVVDVSHEDPEVK

FNWYVDGVEVHNAKTKPREEQYNSTYRVVSVLTVLHQDWLNGKEYKCKVSNKALPAPIE

KTISKAKGQPREPQVYTLPPSRDELTKNQVSLTCLVKGFYPSDIAVEWESNGQPENNYK

TTPPVLDSDGSFFLYSKLTVDKSRWQQGNVFSCSVMHEGLHNHYTQKSLSLSPGK

(Human chimeric W27G2 IgG antibody light chain amino acid sequence)

>SEQ ID NO 78:W27G2_HUMAN _CHIMERA_IGG_L

MDMKLPVRLLVLMFWIPGFSSDVLMTQTPLSLPVSLGDQASISCRASQSIVHTNGNTYL

EWYLQKPGQSPKLLIYKVSNRFSGVPDRFSGSGSGTDFILKISRVEAEDLGVYYCFQGS

HVPPTFGGGTKLEVKRADAAPTVSIFPPSSEQLTSGGASVVCLLNNFYPREAKVQWKVD

NALQSGNSQESVTEQDSKDSTYSLSSTLTLSKADYEKHKVYACEVTHQGLSSPVTKSFN

RGEC

(Gastrointestinal contents or excretions)

The gastrointestinal contents or excretions used in the method of the present disclosure are not particularly limited, and one of the ordinary skill in the art can appropriately select specific gastrointestinal contents or excretions to be used in consideration of the purpose and workability. In a preferred aspect, the gastrointestinal contents or excretions used in the methods of the present disclosure are feces. The gastrointestinal contents or excretions may be obtained by a method known in the art, for example, may be obtained from the lumen of the gastrointestinal tract using an endoscope, or a part of the excretions may be obtained.

(Modification of gastrointestinal contents or excretions by IgA antibody)

In the method of the present disclosure, the modification of the gastrointestinal contents or excretions by the IgA antibody includes various processes including a step of contacting the IgA antibody with the gastrointestinal contents or excretions.
In one aspect, the gastrointestinal contents or excretions are modified by contacting with an IgA antibody that restores the bacterial flora in the gastrointestinal tract of IBD patients, before administration to subjects. In one aspect, after the step of contacting the IgA antibody with gastrointestinal contents or excretions, the IgA antibody is separated from the gastrointestinal contents or excretions with the conjugate thereof. In one aspect, after contacting the IgA antibody with gastrointestinal contents or excretions, the IgA antibody is used without being separated from the gastrointestinal contents or excretions. In a preferred aspect, after contacting the IgA antibody with the gastrointestinal contents or excretions, bacteria that bind to the IgA antibody are excluded from the gastrointestinal contents or excretions together with the IgA antibody. The gastrointestinal contents or excretions can be further processed before or after modification with the IgA antibody. For example, a bacterial flora is purified and acquired from the gastrointestinal contents or excretions, and the bacterial flora modified with an IgA antibody can be used as the modified gastrointestinal contents or excretions. In another aspect, after modifying the gastrointestinal contents or excretions with IgA antibodies, a bacterial flora is purified and obtained from the modified product and can be used as modified gastrointestinal contents or excretions.

In one aspect, the gastrointestinal contents or excretions are modified by contacting with an IgA antibody that restores the bacterial flora in the gastrointestinal tract of IBD patients, after administration to subjects. For example, concurrently administering a composition including gastrointestinal contents or excretions and a composition including an IgA antibody allows to modify the gastrointestinal contents or excretions after being administered to subjects with the IgA antibody. The composition including gastrointestinal contents or excretions and the composition including an IgA antibody may each be included in a separate dosage unit or may be included in a single dosage unit. When a composition including gastrointestinal contents or excretions and a composition including an IgA antibody are included in separate dosage units, these compositions can be administered in any combination of order of administration, frequency of administration, schedule of administration, and dose, depending on the purpose. In a preferred aspect, a composition including an IgA antibody is administered after administering a composition including gastrointestinal contents or excretions.

The amount of the IgA antibody used for modifying the gastrointestinal contents or excretions is not particularly limited, and is adjusted by one of the ordinary skill in the art in consideration of the purpose, workability, and the like. For example, in the case of application to human excretion, the IgA antibody can be applied in a range of 1 mg to 1 g per 1 g of excretion. The amount of IgA antibody to be applied can be further adjusted according to the bacterial count included in the excretion.

(Method for administering modified gastrointestinal contents or excretions)

In the method of the present disclosure, the method for administering the gastrointestinal contents or excretions modified with the IgA antibody to the IBD patient is not particularly limited as long as it is a method capable of affecting the intestinal flora of the patient. For example, administration is performed by common fecal transplantation therapy. The gastrointestinal contents or excretions modified with the IgA antibody of the present disclosure can be administered, for example, by rectal administration with a colonic endoscope, a suppository, an enema, an upper endoscope, or an upper push small intestinal endoscope, or nasal or oral intubation via a nasogastric, nasoenteric, or nasojejunal tubenasogastric tube.

In addition, the gastrointestinal contents or excretions modified with the IgA antibody of the present disclosure can be administered orally by solids such as pills, tablets, suspensions, gels, gel-tabs, semi-solids, tablets, sachets, troches, or capsules or microcapsules, or as intestinal formulations, or in the form of compositions such as liquids, suspensions, gels, gel-tabs, semi-solids, tablets, sachets, troches, or capsules.

The composition including the gastrointestinal contents or excretions modified by the IgA antibody of the present disclosure may undergo additional processing before being administered. In addition, the composition including the gastrointestinal contents or excretions modified with the IgA antibody of the present disclosure may be lyophilized, freeze dried, frozen, or processed into a powder.

The composition of the present disclosure, in one aspect, contains one or more types of pharmaceutically acceptable carriers. As the carrier to be used, those known in the art can be used, and the carrier is appropriately selected according to the dosage form and administration mode of the composition. For example, as the carrier, a diluent or an excipient, for example, a filler, a binder, a wetting agent, a disintegrant, a surfactant, a glidant, a lubricant, or the like may be used. In general, the carrier may be a solid (including a powder), a liquid, or a combination thereof. Each carrier is preferably acceptable from the viewpoint of being compatible with the other ingredients in the composition and harmless to the subject. The carrier may be biologically acceptable and inert. For example, the carrier is formulated to allow the composition to maintain the viability of the biomaterial thereof until delivered to the appropriate site.

When the composition of the present disclosure is made into an oral composition, an inert diluent or an edible carrier may be included. For example, a sweetening agent such as sucrose or saccharin, and a flavoring agent such as peppermint, methyl salicylate, or orange flavor may be included. In addition, the oral composition may be prepared by combining a food product with the gastrointestinal contents or excretions modified with the IgA antibody of the present disclosure. In one embodiment, the food product used for administration is cooled and provided in the form of, for example, ice cream. In one embodiment, an oral composition including bacterial flora purified from gastrointestinal contents or excretions can be provided as a food composition.

The amount of the gastrointestinal contents or excretions contained per unit of administration in the composition including the gastrointestinal contents or excretions modified by the IgA antibody of the present disclosure can be set by one of the ordinary skill in the art in consideration of the administration route, administration form, patient condition, administration frequency, and the like. For example, but not limited to, about 0.001 to 1000 milligrams (mg)/kilogram (kg) of body weight can be used as a daily dose of the active ingredient (modified gastrointestinal contents or excretions). For example, the use is possible in an amount of 0.01 to 100 mg/kg body weight, 0.1 to 50 mg/kg body weight, 1 to 10 mg/kg body weight, or the like.

2. A composition for use in a method for treating IBD using gastrointestinal contents or excretions modified with IgA antibody.

The present disclosure provides, in one aspect, a composition for treating IBD including gastrointestinal contents or excretions modified with an IgA antibody, and a method for producing the same.

The composition for treating IBD of the present disclosure, in one aspect, is a composition including the gastrointestinal contents or excretions modified with an IgA antibody, as described above in "1. Method for treating IBD with gastrointestinal contents or excretions modified with IgA antibody".

The gastrointestinal contents or excretions included in the composition for treating IBD of the present disclosure are those described in (Gastrointestinal contents or excretions) of "1. Method for treating IBD with gastrointestinal contents or excretions modified with IgA antibody" above. The gastrointestinal contents or excretions are modified with an IgA antibody that can restore the bacterial flora in the gastrointestinal tract of the IBD patient, according to the description of (IgA antibody) and (Modification of gastrointestinal contents or excretions by IgA antibody) described above.

In one aspect, the gastrointestinal contents or excretions modified with an IgA antibody are included in the composition as purified and separated bacterial flora.

The composition for treating IBD of the present disclosure, in one aspect, is a composition including an IgA antibody that modifies gastrointestinal contents or excretions after administration, as described above in "1. Method for treating IBD with gastrointestinal contents or excrements modified with IgA antibody".

The composition for treating IBD of the present disclosure is mixed with a carrier or formulated as it is without being mixed with a carrier so as to have properties suitable for various administration routes and dosages described above in (Method for administrating modified gastrointestinal contents or excretions and composition containing the same) of "1. Methods for treating IBD with gastrointestinal contents or excretions modified with IgA antibody".

3. Method for obtaining IgA antibody that restores bacterial flora in gastrointestinal tract of IBD patient.

In one aspect, the present disclosure provides a method for screening an IgA antibody that restores the bacterial flora in the gastrointestinal tract of IBD patients.

In one aspect, the screening method of the present disclosure includes the following steps:

(B1) a step of preparing gastrointestinal contents or excretions of an IBD patient;
(B2) a step of modifying the gastrointestinal contents or excretions with a candidate IgA antibody; and
(B3) a step of confirming that the modified gastrointestinal contents or excretions are capable of restoring the bacterial flora in the gastrointestinal tract.

In the method of the present disclosure, (B1) the step of preparing gastrointestinal contents or excretions of IBD patients is performed as described above in (Gastrointestinal contents or excretions) of "1. Method for treating IBD using gastrointestinal contents or excretions modified by IgA antibody".

In the method of the present disclosure, the candidate IgA antibody used in (B2) the step of modifying the gastrointestinal contents or excretions with a candidate IgA antibody may have an amino acid sequence derived from the same species as the IBD patient, or may have an amino acid derived from a different species, such as a closely related different organism. Specific examples thereof include human-derived, mouse-derived, rat-derived, hamster-derived, rabbit-derived, goat-derived, donkey-derived, but-derived, bovine-derived, equine-derived, chicken derived, simian-derived, chimpanzee-derived, camel-derived, and llama-derived.

The candidate IgA antibody may further be conjugated to other compounds or fused to heterologous peptides.

In the method of the present disclosure, the modification in (B2) the step of modifying the gastrointestinal contents or excretions with a candidate IgA antibody is performed as described in the above (Modification of gastrointestinal contents or excretions with IgA antibody) of "1. Method for treating IBD using gastrointestinal contents or excretions modified by IgA antibody".

In the method of the present disclosure, (B3) the step of confirming that the modified gastrointestinal contents or excretions can restore bacterial flora in the gastrointestinal tract is performed by the various methods described in the method for confirming that the IgA antibody can restore bacterial flora in the gastrointestinal tract of the IBD patient as described above in (IgA antibody) of "1. Method for treating IBD using gastrointestinal contents or excretions modified by IgA antibody".

In one aspect, the present disclosure provides a method for producing an IgA antibody, including each step of the above-described screening method.

4. Method for processing gastrointestinal contents or excretions from IBD patient in vitro using IgA antibody

The present disclosure provides, in one aspect, a method for modifying the gastrointestinal contents or excretions of IBD patients using an IgA antibody.

The method of the present disclosure, in one aspect, includes the following steps:

(C1) a step of preparing the gastrointestinal contents or excretions of an IBD patient; and
(C2) a step of contacting the gastrointestinal contents or excretions with an IgA antibody that restores bacterial flora in the gastrointestinal tract in vitro.

(C1) The step of preparing gastrointestinal contents or excretions of an IBD patient in the method of the present disclosure is performed as described above in (Gastrointestinal contents or excretions) in "1. Method for treating IBD using gastrointestinal contents or excretions modified by IgA antibody".

The IgA antibody used in (C2) the step of, in vitro, contacting the gastrointestinal contents or excretions with an IgA antibody that restores bacterial flora in the gastrointestinal tract of IBD patients is an IgA antibody that has been confirmed to be capable of restoring the bacterial flora in the gastrointestinal tract of IBD patients by the various methods described for the method for confirming that an IgA antibody is capable of restoring the bacterial flora in the gastrointestinal tract of IBD patients, in (IgA antibody) of "1. Method for treating IBD using gastrointestinal contents or excretions modified by IgA antibody".

Contact of the gastrointestinal contents or excretions with the IgA antibody is typically achieved by mixing the two. Before mixing, the gastrointestinal contents or excretions may or may not be diluted with a diluent.

The gastrointestinal contents or excretions of IBD patients modified by the IgA antibody obtained as a result of the method of the present disclosure can be administered to IBD patients as an active ingredient of a pharmaceutical drug for improving IBD.

5. Composition containing IgA antibody for modifying gastrointestinal contents or excretions of IBD patient in vitro

In one aspect, the present disclosure provides a composition containing an IgA antibody for processing the gastrointestinal contents or excretions of IBD patients in vitro, and a method for producing the same.

The IgA antibody contained in the composition of the present disclosure has been confirmed to be capable of restoring the bacterial flora in the gastrointestinal tract of IBD patients by the various methods described in the method for confirming that the IgA antibody can restore the bacterial flora in the gastrointestinal tract of IBD patients, in the above (IgA antibody) of "1. Method for treating IBD using gastrointestinal contents or excretions modified by IgA antibody".

The composition of the present disclosure, in one aspect, is formulated with one or more types of pharmaceutically acceptable carriers in a variety of forms suitable for modifying the gastrointestinal contents or excretions of IBD patients. For example, formulation is performed in the form of a solution, suspension, gel, capsule, or microcapsule. As the carrier, a diluent or excipient, for example, a filler, binder, wetting agent, disintegrant, surfactant, glidant, lubricant, or the like may be used.

The amount of IgA antibody contained in the composition of the present disclosure is not particularly limited, and can be adjusted by one of the ordinary skill in the art taking into consideration the purpose, workability, and the like. For example, when applied to human excretion, the formulation can be made such that the IgA antibody is applied in the range of 1 mg to 1 g per gram of the excrement. The amount of IgA antibody to be applied can be further adjusted according to the bacterial count included in the excretion.

6. Method for testing subjects with a pharmaceutical drug containing an IgA antibody to diagnose presence or absence of IBD or risk of developing IBD

The present disclosure provides a method for testing subjects using the disclosed IgA antibody that restores bacterial flora in a gastrointestinal tract to diagnose the presence or absence of IBD in the subject or the risk of developing IBD.

The method of the present disclosure, in one aspect, includes the following steps:

(D1) a step of preparing IgA antibody that restores bacterial flora in a gastrointestinal tract of an IBD patient;
(D2) a step of contacting the gastrointestinal contents or excretions of a subject with the IgA antibody; and
(D3) a step of identifying bacteria that bind to the IgA antibody.

Then, if the IgA antibody and the IBD-related bacteria exhibit binding, the subject is processed as suffering from IBD or having a risk of developing IBD.

The IgA antibody used in this test is an IgA antibody confirmed to be capable of restoring the bacterial flora in the gastrointestinal tract of IBD patients in IBD patients. The fact that the IgA antibody can restore the bacterial flora in the gastrointestinal tract can be confirmed, for example, according to the same method as the described method for confirming that the IgA antibody can restore the bacterial flora in the gastrointestinal tract of IBD patients, regarding to the above (Method for treating IBD using gastrointestinal contents or excretions modified with the IgA antibody).

(D2) The step of bringing the gastrointestinal contents or excretions into contact with the IgA antibody in the present disclosure is typically performed by mixing the two. Before mixing, the gastrointestinal contents or excretions may or may not be diluted with a diluent. The amount of an IgA antibody to be used can be appropriately set by one of the ordinary skill in the art according to the characteristics of the sample, desired sensitivity, and the like. For example, for example, in the case of application to human excretion, the IgA antibody can be applied in a range of 1 mg to 1 g per 1 g of the excretion. The amount of IgA antibody to be applied can be further adjusted according to the bacterial count included in the excretion.

(D3) The step of identifying a bacterium that binds to the IgA antibody in the present disclosure is performed, for example, by comparing the amount of binding of the IgA antibody with enterobacteria that is known to exacerbate IBD between the gastrointestinal contents or excretions derived from the patient and the gastrointestinal contents or excretions derived from healthy persons. In another aspect, the analysis step can be performed by, for example, comparing a binding profile created on the basis of the type and amount of bacteria bound to the IgA antibody by bringing the gastrointestinal contents or excretions derived from the patient into contact with the IgA antibody and a binding profile created on the basis of the type and amount of bacteria bound to the IgA antibody by bringing the gastrointestinal contents or excretions derived from healthy persons into contact with the IgA antibody. In one example, the binding profile can be shown by quantitative data with the IgA Index, which is determined from the amounts of binding and no binding to IgA for each bacterium (Andrew L. Kau, et al Sci Transl Med 7, 2015, Hirosuke Sugahara, et al., Frontiers in Microbiology 8, 1757, 2017).

For example, the IgA Index is calculated according to the following formula:

$$IgA^{+}_{taxon\ abundance} = IgA^{+}_{taxon\ abundance} \times IgA\ binding\ ability\ /100$$

$$IgA^{-}_{taxon\ abundance} = IgA^{-}_{taxon\ abundance} \times (1- IgA\ binding\ ability\ /100)$$

$$IgA\ index = - \frac{[\log (IgA^{+}_{taxon\ abundance}) - \log (IgA^{-}_{taxon\ abundance})]}{[\log (IgA^{+}_{taxon\ abundance}) + \log (IgA^{-}_{taxon\ abundance})]}$$

A plurality of types of IgA antibodies can be used in the methods for testing a subject for IBD treatment of the present disclosure. In one aspect, the subject is processed as suffering from IBD or having a risk of developing IBD if a test of the present disclosure is performed using a plurality of IgA antibodies each of which has been confirmed to be capable of restoring the bacterial flora in the gastrointestinal tract in different IBD patients, and one or more types thereof show specific binding to IBD-related bacteria. If, of a plurality of IgA antibodies tested, a plurality of IgA show specific binding to IBD-related bacteria, it is more reliably presumed that the subject is suffering from IBD or having a risk of developing IBD. For example, the subjects are processed as suffering from IBD or having a risk of developing IBD if one or more types, two or more types, three or more types, four or more types, and five or more types of IgA that can restore the bacterial flora in the gastrointestinal tract of IBD patients bind to IBD-related bacteria.

Whether subjects are suffering from IBD or having a risk of developing IBD can be expressed as a qualitative or quantitative indicator. For example, it is possible to perform quantification by using an index that account for the binding amount between the IgA antibody and IBD-related bacteria or binding specificity, or it is possible to be shown qualitatively by classifying the quantified data.

If subjects are processed as suffering from IBD or having a risk of developing IBD, the processing includes any processing related to maintaining or improving the health of the subjects. For example, the information processing system includes, but is not limited to, classifying and tagging the patient as an IBD patient or a person having a risk of developing IBD, guiding the patient to improve his/her life as an IBD patient or a person having a risk of developing IBD, and treating and preventing IBD.

When subjects are treated for IBD treatment of the present disclosure, the treatment method is not particularly limited, and known IBD treatment methods can be used. In one aspect, the method for treating IBD includes administering to the subject gastrointestinal contents or excretions modified with an IgA antibody that has shown binding to IBD-related bacteria in the test.

7. Pharmaceutical drug for testing subject to determine whether IBD treatment is necessary, including IgA antibody

The present disclosure provides, in one aspect, a pharmaceutical drug for diagnosing the presence or absence of IBD morbidity or the risk of developing IBD, the drug including an IgA antibody that restores the bacterial flora in the gastrointestinal tract of IBD patients.

The diagnostic pharmaceutical drug of the present disclosure is used for "6. Method for testing subjects for IBD treatment using diagnostic pharmaceutical drug containing IgA antibody", and contains an IgA antibody having the properties described for the method.

The composition of the present disclosure, in one aspect, is formulated with one or more types of pharmaceutically acceptable carriers in a variety of forms suitable for testing the gastrointestinal contents or excretions of IBD patients. For example, formulation is performed in the form of a solution, suspension, gel, capsule, or microcapsule. As the carrier, a diluent or excipient, for example, a filler, binder, wetting agent, disintegrant, surfactant, glidant, lubricant, or the like may be used.

The amount of IgA antibody contained in the composition of the present disclosure is not particularly limited, and can be adjusted by one of the ordinary skill in the art taking into consideration the purpose, workability, and the like. For example, when applied to human excretion, the formulation can be made such that the IgA antibody is applied in the range of 1 mg to 1 g per gram of the excrement. The amount of IgA antibody to be applied can be further adjusted according to the bacterial count included in the excretion.

8. Method for testing patients treated with IBD therapeutic agent containing gastrointestinal contents or excretions using a diagnostic pharmaceutical drug containing IgA antibody

In one aspect, the present disclosure provides a method for testing a diagnostic pharmaceutical drug containing IgA antibody in patients treated with an IBD therapeutic agent containing gastrointestinal contents or excretions.

The method of the present disclosure, in one aspect, includes the following steps:

(E1) a step of obtaining gastrointestinal contents or excretions of a patient who is, is to be, or has been administered with an IBD therapeutic agent containing gastrointestinal contents or excretions;
(E2) a step of contacting the gastrointestinal contents or excretions with an IgA antibody; and
(E3) a step of analyzing bacteria that bind to the IgA antibody.

If the analysis results indicate that the bacterial flora in a gastrointestinal tract is restored, treatment with the IBD therapeutic agent is continued.

In the method of the present disclosure, the test subject is a patient who is receiving, will be receiving, or has previously received an IBD therapeutic agent that contains gastrointestinal contents or excretions.

The IBD therapeutic agent containing gastrointestinal contents or excretions of the present disclosure is preferably an IBD therapeutic agent containing gastrointestinal contents or excretions modified by the IgA antibody used in this test. In one aspect, the gastrointestinal contents or excretions contained in the IBD therapeutic agent of the present disclosure are gastrointestinal contents or excretions obtained from the patient himself/herself. Preferably, the IgA antibody used in this test is an IgA antibody that has been confirmed to be capable of restoring bacterial flora in a gastrointestinal tract of the patient being tested.

(D2) The step of bringing the gastrointestinal contents or excretions into contact with the IgA antibody in the present disclosure is typically performed by mixing the two. Before mixing, the gastrointestinal contents or excretions may or may not be diluted with a diluent. The amount of an IgA antibody to be used can be appropriately set by one of the ordinary skill in the art according to the characteristics of the sample, desired sensitivity, and the like. For example, for example, in the case of application to human excretion, the IgA antibody can be applied in a range of 1 mg to 1 g per 1 g of the excretion. The amount of IgA antibody to be applied can be further adjusted according to the bacterial count included in the excretion.

(D3) The step of the present disclosure of analyzing bacteria that bind to the IgA antibody is performed, for example, by comparing the amount of binding of the IgA antibody to enterobacteria known to exacerbate IBD in the gastrointestinal contents or excretions derived from the patient with that in the gastrointestinal contents or excretions derived from healthy persons. In another aspect, the analysis step can be performed by, for example, comparing a binding profile created on the basis of the type and amount of bacteria bound to the IgA antibody by bringing the gastrointestinal contents or excretions derived from the patient into contact with the IgA antibody and a binding profile created on the basis of the type and amount of bacteria bound to the IgA antibody by bringing the gastrointestinal contents or excretions derived from healthy persons into contact with the IgA antibody.

In the method of the present disclosure, the analysis results indicate that bacteria that bind to an IgA antibody restore bacterial flora in a gastrointestinal tract, meaning, for example, that when comparing the amount of binding of the IgA antibody to enterobacteria known to exacerbate IBD in the gastrointestinal contents or excretions derived from the patient with that in the gastrointestinal contents or excretions derived from healthy persons, no significant difference is observed between the two. In another aspect, the analysis results indicate that the bacteria that bind to an IgA antibody restore bacterial flora in a gastrointestinal tract, meaning, for example, that when comparing a binding profile created based on the type and amount of bacteria that bind to the IgA antibody by contacting the gastrointestinal contents or excretions from the patient with the IgA antibody with a binding profile created based on the type and amount of bacteria that bind to the IgA antibody by contacting the gastrointestinal contents or excretions from healthy persons with the IgA antibody, no significant difference is observed in the profiles related to IBD-related bacteria.

9. Diagnostic pharmaceutical drug containing IgA antibody for testing patients treated with IBD therapeutic agent containing gastrointestinal contents or excretions

In one aspect, the present disclosure provides a diagnostic pharmaceutical drug containing an IgA antibody for testing patients treated with an IBD therapeutic agent containing gastrointestinal contents or excretions.

The diagnostic pharmaceutical drug of the present disclosure is used in "8. Method for testing patients treated with IBD therapeutic agent containing gastrointestinal contents or excretions using a diagnostic pharmaceutical drug containing IgA antibody" and contains an IgA antibody having the properties described for the method.

The composition of the present disclosure, in one aspect, is formulated with one or more types of pharmaceutically acceptable carriers in a variety of forms suitable for testing the gastrointestinal contents or excretions of IBD patients. For example, formulation is performed in the form of a solution, suspension, gel, capsule, or microcapsule. As the carrier, a diluent or excipient, for example, a filler, binder, wetting agent, disintegrant, surfactant, glidant, lubricant, or the like may be used.

The amount of IgA antibody contained in the composition of the present disclosure is not particularly limited, and can be adjusted by one of the ordinary skill in the art taking into consideration the purpose, workability, and the like. For example, when applied to human excretion, the formulation can be made such that the IgA antibody is applied in the range of 1 mg to 1 g per gram of the excrement. The amount of IgA antibody to be applied can be further adjusted according to the bacterial count included in the excretion.

In one aspect, "1. Method for treating IBD using gastrointestinal contents or excretions modified by IgA antibody", "6. Method for testing subjects for IBD treatment using a diagnostic pharmaceutical drug containing IgA antibody", and "8. Method for testing patients treated with IBD therapeutic agent containing gastrointestinal contents or excretions with diagnostic therapeutic agent containing IgA antibody" of the present disclosure, as well as the compositions used in these methods, can be used in combination in a part or in whole. For example, patients who have been selected for IBD treatment by "6. Method for testing subjects for IBD treatment using diagnostic pharmaceutical drug containing IgA antibody" can be treated by "1. Method for treating IBD using gastrointestinal contents or excretions modified by IgA antibody", and the progress of the treatment can be diagnosed by "8. Method for testing patients treated with IBD therapeutic agent containing gastrointestinal contents or excretions with a diagnostic pharmaceutical drug containing IgA antibody". The IgA antibody used in these methods may be the same or different.

Examples

The present invention will be described in more detail below with reference to examples, but these are merely illustrative and do not limit the present disclosure.

Example 1: Binding characteristics of endogenous IgA antibody

(Methods and materials)

Bacterial extraction from human feces samples

Human feces samples from patients with inflammatory bowel disease (n = 12, of the 12 cases, 7 were ulcerative colitis patients and 5 were Crohn's disease patients, P1 to P12) and healthy persons (n = 12, C1 to C12) were provided by Shiga University of Medical Science and Kanazawa University.

Healthy persons were defined as volunteers who showed no abnormal values in their medical checkups. Fecal samples were immediately placed in anaerobic packs after collection, stored frozen (-80°C), and sent to the University of Tokyo. In addition, a human feces sample from one patient (P13) with ulcerative colitis was provided by the Department of Gastroenterology at the University of Tokyo and used in the experiment on the same day.

The provided sample was placed in an anaerobic chamber (80% N2, 10% H2, 10% CO2, Coy Laboratory Products) to prepare a bacterial solution as follows, aliquoted, and stored at -80°C for subsequent experiments. All reagents used in the anaerobic chamber were sterilized through a 0.22 $\mu$m filter and left to stand overnight in the anaerobic chamber to be anaerobic. A frozen fecal sample was thawed in an anaerobic chamber by adding 30 ml of PBS, then suspended and centrifuged at 50 g for 15 min at 4°C. The sample provided by the University of Tokyo was weighed, suspended in 9-fold volumes of PBS, and centrifuged at 50 g for 15 minutes at 4°C. The supernatant was collected in another 50 ml tube, suspended well, and then dispensed at 1 ml into 1.5 ml tubes and stored at -80°C as bacterial frozen stocks of the human feces sample.

Measurement of bacterial count concentration in bacterial frozen stocks of human feces sample

Bacterial frozen stocks of the human feces sample was thawed in an anaerobic chamber. 10 $\mu$l of bacterial frozen stocks was taken and diluted 1,000-fold with PBS. Bacterial counts were measured according to the Cell Viability kit (BD) protocol. In the present study, in order to target all bacteria contained in a human feces sample, the bacterial count was measured using only thiazole orange (TO), which detects bacteria by staining nucleic acid, without using propidium iodide (PI), which detects dead bacteria. PBS, Liquid Counting Beads (BD), and thiazole orange (final concentration 42 nM, BD) were added to the bacterial dilution and reacted on ice for 10 minutes. The solution for bacterial count was subjected to flow cytometry analysis using a SONY SA3800 Analyzer (Sony Group Corporation). The bacterial count concentration of the bacterial dilution was calculated based on the number of Counting Beads detected in the analysis.

## EP 4 523 694 A1

16S rRNA gene amplicon sequence analysis of bacterial DNA

Bacterial DNA was subjected to polymerase chain reaction (PCR) targeting the V3 to V4 region of the variable region of the 16S rRNA gene. A primer was prepared for next-generation sequencing using Miseq, and index sequences were added to distinguish the samples to be analyzed (refer to the following). After the PCR reaction, 10 × Loading Buffer (Takara Bio Inc.) was added and agarose electrophoresis was performed. The gel used for electrophoresis of the PCR sample was a 1.5% agarose gel (VWR life science) containing LED StainG (LABTAS+). The DNA size marker used was 100 bp DNA Ladder (Takara Bio Inc.). Electrophoresis was performed at 100 V for 30 min. After electrophoresis, the PCR product of about 500 bp was excised and purified using a Fast Gene gel extraction kit (NIPPON Genetics Co, Ltd). The purified PCR product was subjected to electrophoresis again to confirm whether or not purification was successful. Sequence analysis was commissioned to TAK-Circulator Corporation and performed using Mi-seq Reagent Kit V3 (Illumina, Inc.). The sequence data obtained by sequence analysis was used for bacterial flora analysis using Qiime2 software (ver. 2020-2).

The primer sequences for 16S rRNA gene amplicon sequence analysis are shown below.

| Primer name | Base sequence (5' >3') |
|---|---|
| 342F | AATGATACGGCGACCACCGAGATCTACACXXXXXXXXXTACACGACGCTCTTCCGATCTCTACGGGGGGCAGCAG |
| 806R | CAAGCAGAAGACGGCATACGAGATXXXXXXGTGACTGGAGTTCAGACGTGTGCTCTTCCGATCTGGACTACCGGGGTATCT |

The XXX represents the index sequence, and the gray indicates the sequence that targets the template 16S rRNA.

Method for calculating IgA Index

A high IgA Index value indicates that the bacterium is strongly bound by the IgA antibody, whereas a low value indicates that the bacterium is weakly bound by the IgA antibody. The IgA Index for each individual was calculated taking into consideration the fact that the binding ratio of the IgA antibody to the total bacterial flora differs from person to person. IgA binding ability is the ratio of bacteria that bind to the IgA antibody to the total bacterial flora. Using this, the ratio of IgA antibody-binding bacteria or antibody non-binding bacteria (IgA+ taxon abundance, IgA- taxon abundance) was calculated for each individual, and then the IgA index was calculated (Andrew L. Kau, et al Sci Transl Med 7,2015, Hirosuke Sugahara, et al., Frontiers in Microbiology 8, 1757, 2017).

$$IgA^+_{taxon\ abundance} = IgA^+_{taxon\ abundance} \times IgA\ binding\ ability\ /100$$

$$IgA^-_{taxon\ abundance} = IgA^-_{taxon\ abundance} \times (1- IgA\ binding\ ability\ /100)$$

$$IgA\ index = -\frac{[log\ (IgA^+_{taxon\ abundance})-log\ (IgA^-_{taxon\ abundance})]}{[log\ (IgA^+_{taxon\ abundance})+log\ (IgA^-_{taxon\ abundance})]}$$

Specifically, the taxon abundance of IgA antibody-binding bacteria was calculated by multiplying the relative abundance of IgA-binding bacteria obtained by 16S rRNA analysis by the IgA binding ability (the ratio of IgA antibody-binding bacteria to the total bacterial flora). On the other hand, for antibody non-binding bacteria, the taxon abundance of IgA antibody non-binding bacteria was calculated by multiplying the relative abundance by 1-IgA binding ability (number of 100% minus the ratio of IgA antibody-binding bacteria). The IgA index was determined using each of the taxon abundance.

Separation of RS_H000_L001 IgA antibody-binding bacteria and non-binding bacteria from human feces sample using Magnetic Cell Sorter (MACS) and cell sorter

The procedure was performed in an anaerobic chamber. After measuring the bacterial count, the bacteria in the human feces sample were prepared to $6 \times 10^7$ cells/ml by adding PBS. Then, centrifugation was performed at 8,000 g for 5 minutes at 4°C, and the supernatant was removed. 100 $\mu$l of PBS containing 20% normal rat serum (FUJIFILM Wako Pure Chemical Corporation) was added to the cells with the supernatant removed, which were then allowed to react on ice for 20 minutes. This prevented non-specific binding of the antibody used. FACS buffer was added to the bacterial solution, which was then centrifuged at 8,000 g for 5 minutes at 4°C, and the supernatant was removed. 15 $\mu$g of biotinylated RS_H000_L001 IgA antibody was added per $6 \times 10^6$ bacteria, and the mixture was allowed to react on ice for 20 minutes. FACS buffer was added to the antibody reaction solution, which was then centrifuged at 8,000 g for 5 minutes at 4°C, and the supernatant was removed. 15 $\mu$l of microbeads-streptavidin (Miltenyi Biotec) was added per $6 \times 10^6$ bacteria, and was allowed to react on ice for 20 minutes. The bacterial isolation procedure was performed in accordance with the protocol of the MACS column (LS column, Miltenyi Biotec) kit. The bacteria adsorbed to the MACS column were collected as RS_H000_L001 IgA antibody-binding bacteria, and the bacteria that passed through the column were collected as RS_H000_L001IgA antibody-non-binding bacteria. For the RS_H000_L001 IgA antibody-binding bacteria, the RS_H000_L001IgA antibody-binding bacteria were concentrated again using a MACS column. The collected RS_H000_L001 IgA antibody-binding bacteria and non-binding bacteria were centrifuged at 8,000 g for 5 minutes at 4°C, and the supernatant was removed. FACS buffer, TO (final concentration 42 nM, BD), and Phycoerythrin (PE)-anti mouse IgA antibody (final concentration 5 $\mu$g/ml, BioLegend, Inc.) were added to the RS_H000_L001 IgA antibody-binding bacteria and non-binding bacteria collected by MACS, and the reaction was performed for 10 minutes while shielding from light on ice. Then, RS_H000_L001 IgA antibody-binding bacteria and non-binding bacteria were separated by cell sorting using a SONY Cell Sorter SH800 (Sony Group Corporation). The bacteria from each fraction collected by cell sorting were reanalyzed using a SONY Cell Sorter SH800, and it was confirmed that the desired fractions were collected at a ratio of 90% or more.

Separation of human endogenous IgA antibody-binding bacteria and non-binding bacteria from human feces sample using magnetic cell sorter (MACS) and cell sorter

Bacteria from a human feces sample was thawed in an anaerobic chamber. All reagents used in the anaerobic chamber were sterilized through a 0.22 $\mu$m filter and left to stand overnight in the anaerobic chamber to be anaerobic. After measuring the bacteria count, PBS was added to adjust the concentration to $6 \times 10^7$ cells/ml. Then, centrifugation was performed at 8,000 g for 5 minutes at 4°C, and the supernatant was removed. 100 µl of PBS containing 20% normal rat serum (FUJIFILM Wako Pure Chemical Corporation) was added to the cells with the supernatant removed, which were then allowed to react on ice for 20 minutes. This prevented non-specific binding of the antibody used. FACS buffer was added to the bacterial solution, which was then centrifuged at 8,000 g for 5 minutes at 4°C, and the supernatant was removed. 15 µl of Anti-IgA-PE human antibody (Miltenyi Biotec) was added to $6 \times 10^6$ of bacteria count, and was reacted on ice for 20 minutes. FACS buffer was added to the antibody reaction solution, which was then centrifuged at 8,000 g for 5 minutes at 4°C, and the supernatant was removed. 15 µl of Anti-PE Microbeads (Miltenyi Biotec) was added to $6 \times 10^6$ bacteria, and was reacted on ice for 20 minutes. The bacterial isolation procedure was performed in accordance with the protocol of the MACS column (LS column, Miltenyi Biotec) kit. The bacteria adsorbed on the MACS column were collected as human endogenous IgA antibody-binding bacteria, and the bacteria passing through the column were collected as human endogenous IgA antibody-non-binding bacteria. The human endogenous IgA antibody-binding bacteria were concentrated using the MACS column again. The collected human endogenous IgA antibody-binding bacteria and non-binding bacteria were centrifuged at 8,000 g for 5 minutes at 4°C, and the supernatant was removed. FACS buffer and TO (final concentration 42 nM, BD) were added to the human endogenous IgA antibody-binding bacteria and non-binding bacteria collected by MACS, and the reaction was performed for 10 minutes while shielding from light on ice. Thereafter, human endogenous IgA antibody-binding bacteria and non-binding bacteria were separated by cell sorting using SONY Cell Sorter SH800 (Sony Group Corporation). The bacteria from each fraction collected by cell sorting were reanalyzed using a SONY Cell Sorter SH800, and it was confirmed that the desired fractions were collected at a ratio of 90% or more.

(Results)

Endogenous polyclonal IgA antibody-binding bacteria were collected from healthy persons or IBD patients, and binding characteristics to various enterobacteria contained in feces collected from healthy persons or IBD patients were tested.

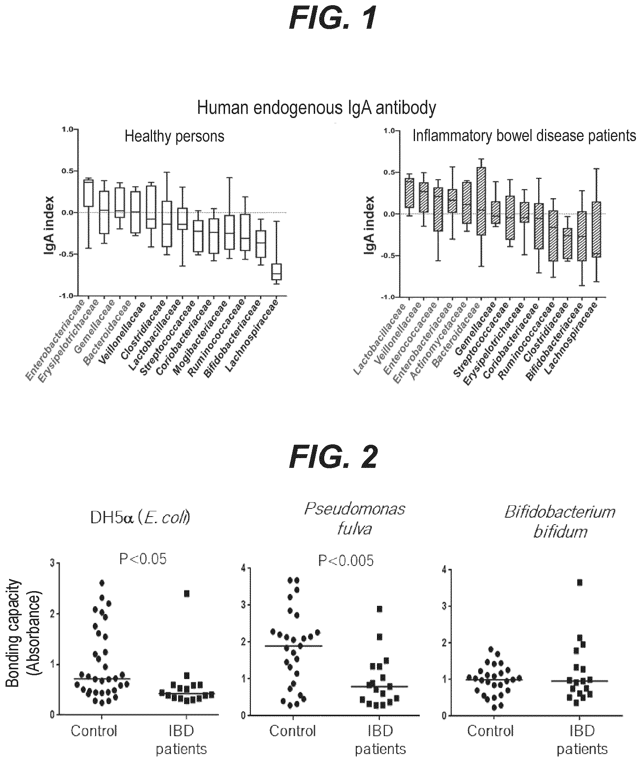

As a result, as shown in Fig. 1, endogenous polyclonal IgA of healthy persons showed a median value of IgA index >0 with respect to Enterobacteriaceae, Erysipelotrichaceae, Gemellaceae, and Bacteroidaceae, and was confirmed to bind to these bacteria. In particular, this showed high binding to Enterobacteriaceae. This bacterium is a bacterium that has been pointed out to be associated with the deterioration of the intestinal environment such as the provocation of enteritis, and it has been suggested that endogenous IgA of healthy persons binds to these bacteria that exacerbate IBD and suppresses the activity thereof, thereby performing a biophylaxis function. On the other hand, endogenous polyclonal IgA of IBD showed a median IgA index >0 against Lactobacillaceae, Veillonellaceae, Enterococcaceae, Enterobacteriaceae, Actinomycetaceae, and Bacteroidaceae, and was confirmed to bind to these bacteria. It is noted that endogenous polyclonal IgA of IBD patients exhibits a binding characteristics significantly different from that of endogenous polyclonal IgA of healthy persons from the viewpoint of exhibiting the highest specificity to Lactobacillaceae, fermentative bacteria that are typically considered not to be associated with deterioration of the intestinal environment. This result has been suggested that the biophylaxis mechanism by endogenous polyclonal IgA does not function well in IBD patients.

Example 2: Reduced binding capacity of IgA derived from IBD patients to IBD-related bacteria

(Methods and materials)

Method for preparing human IgA antibody derived from feces sample

An appropriate amount of PBS was added to a feces sample derived from healthy persons or patients (cryopreserved at -80°C after collection), and the mixture was dissolved and suspended at room temperature. All operations were performed under aerobic conditions. The solution after suspension was centrifuged at 2,000 g for 10 minutes at 4°C, and the supernatant was collected. The supernatant was further centrifuged at 8,000 g for 10 minutes at 4°C, and the supernatant was collected. This operation was repeated once more. The same volume of saturated ammonium sulfate was added to the subsequent supernatant under stirring on ice, and then the mixture was allowed to stand at 4°C overnight. On the next day, centrifugation was performed at 10,000 g for 10 minutes at 4°C to provide an ammonium sulfate precipitate. A 50% saturated ammonium sulfate solution was added thereto, the suspension was obtained, and centrifugal washing was performed at 10,000 g for 10 minutes at 4°C. The subsequent precipitates were dissolved in PBS, and further dialyzed in PBS to cryopreserve in small portions as human fecal derived IgA antibody solutions at -20°C.

Measurement of human IgA antibody titer derived from fecal sample

The IgA antibody concentration in the IgA antibody solution extracted from the feces sample was measured by typical sandwich ELISA. Goat anti-human IgA INLB (final concentration 2 $\mu$g/ml, SouthernBiotech) was used as an anti-human IgA antibody coated on an ELISA plate (C96 MaxiSorp Nunc Immuno Plate (Thermo Fisher Scientific Inc.)). The ELISA plate immobilized with an anti-human IgA antibody was washed 3 times with PBS, 150 $\mu$l of PBS containing 1% bovine serum albumin (BSA, FUJIFILM Wako Pure Chemical Corporation) was added to each well, and the plate was allowed to stand at 4°C overnight to perform blocking. Using human IgA isotype control (Genway, 1 mg/ml) as a control IgA antibody, a dilution series of a control IgA antibody and a human endogenous IgA antibody extracted from healthy persons or patients was prepared in a 96 well plate. The dilution was performed by serial dilution up to 1-fold, 1/3 fold, 1/10 fold, 1/30 fold, 1/100 fold, 1/300 fold, 1/1,000 fold, and 1/3,000 fold using PBS containing 1% BSA. After blocking, PBS containing 1% BSA of the ELISA plate was removed, 50 $\mu$l of the serially diluted antibody was added to each well, and the mixture was reacted at room temperature for 1 hour. Then, the ELISA plate was washed 3 times with PBS containing 0.05% Tween 20 (Chem Cruz), then alkaline phosphatase-conjugated goat anti-human IgA (final concentration 0.5 $\mu$g/ml, SouthernBiotech) was added as a secondary antibody, and the mixture was reacted at room temperature for 1 hour. As a substrate reaction solution for color development, magnesium chloride (final concentration 2 mM) and a phosphatase substrate (Sigma-Aldrich Co. LLC) were dissolved in a sodium hydrogen carbonate (final concentration 6.5 mM) solution and a sodium carbonate (final concentration 18.5 mM) solution, respectively. The plate reacted with the secondary antibody was washed again with PBS containing 0.05% Tween 20, then 50 $\mu$l of the substrate reaction solution was added to each well, and the plate was shielded from light by aluminum foil to develop color, and the absorbance (O.D. 405 nm) was measured using Tristar2 LB942 (Berthold Technologies GmbH & Co.KG). The IgA antibody concentration of each sample was calculated from comparison with the reaction curve of the control IgA antibody.

Measurement of binding capacity of human IgA antibody to bacteria (by ELISA method)

Each bacteria was either aerobically or anaerobically cultured overnight at optimal conditions. Then, PBS was added and centrifuged at 8,000 g for 5 minutes at 4°C to remove the supernatant. After measuring the bacterial count, each bacteria was suspended in a 50 mM sodium carbonate solution such that the bacterial count was $1.0 \times 10^7/50$ nl/well in an ELISA plate (C96 MaxiSorp Nunc Immuno Plate (Thermo Fisher Scientific Inc.)). 50 $\mu$l/well was added to the ELISA plate, and the plate was allowed to stand at 4°C overnight to immobilize the bacteria.

The ELISA plate was washed using a plate washer (Vaccu-Pette/96 multiwell pipetter, Sigma-Aldrich Co. LLC). The ELISA plate immobilized with bacteria was washed 3 times with PBS, 150 $\mu$l of PBS containing 1% bovine serum albumin (BSA, FUJIFILM Wako Pure Chemical Corporation) was added to each well, and the plate was allowed to stand at 4°C overnight to perform blocking. A dilution series of human endogenous IgA antibody samples (concentration adjusted to 30 $\mu$g/ml) extracted from healthy persons or patients was prepared in 96 well plates. The dilution was performed by serial dilution up to 1-fold, 1/3 fold, 1/10 fold, 1/30 fold, 1/100 fold, 1/300 fold, 1/1,000 fold, and 1/3,000 fold using PBS containing 1% BSA. After blocking, PBS containing 1% BSA of the ELISA plate was removed, 50 $\mu$l of the serially diluted antibody was added to each well, and the mixture was reacted at room temperature for 1 hour. Then, washing was performed 3 times with PBS containing 0.05% Tween 20 (Chem Cruz), then alkaline phosphatase-conjugated goat anti-human IgA (final concentration 0.5 $\mu$g/ml, SouthernBiotech) was added as a secondary antibody, and the mixture was reacted at room temperature for 1 hour. As a substrate reaction solution for color development, magnesium chloride (final concentration 2 mM) and a phosphatase substrate (Sigma-Aldrich Co. LLC) were dissolved in a sodium hydrogen carbonate (final concentration 6.5 mM) solution and a sodium carbonate (final concentration 18.5 mM) solution, respectively. The plate reacted with the secondary antibody was washed again with 0.05% Tween 20 added PBS, then 50 $\mu$l of the substrate reaction solution was added to each well, and the color was developed while being shielded from light. Absorbance (O.D. 405 nm) was measured using Tristar2 LB942 (Berthold Technologies GmbH & Co.KG). After confirming that each sample developed color along the dilution series, Fig. 2 plotted the value of the absorbance (405 nm) when the IgA antibody concentration of each sample was 10 mg/ml. The horizontal axis represents the median value. P-values were determined by two-tailed Student's T-test.

(Results)

As a result of measuring the binding capacity of the human IgA antibody to bacteria by the ELISA method, the binding capacity of the IgA antibody to probiotics (good bacteria) such as Bifidobacterium bifidum was not different between healthy persons and patients, but the reaction of the IgA antibody to bacteria belonging to Proteobacteria such as E. coli and Pseudomonas fulva was significantly reduced in IBD patients (Fig. 2).

Example 3: IgA antibody with high binding capacity to IBD-related bacteria

(Methods and materials)

Bacterial extraction from human feces samples

The sample provided in the same manner as in Example 1 was placed in an anaerobic chamber (80% N2, 10% H2, 10% CO2, Coy Laboratory Products) to prepare a bacterial solution as follows, aliquoted, and stored at -80°C for subsequent experiments. All reagents used in the anaerobic chamber were sterilized through a 0.22 $\mu$m filter and left to stand overnight in the anaerobic chamber to be anaerobic. A frozen fecal sample was thawed in an anaerobic chamber by adding 30 ml of PBS, then suspended and centrifuged at 50 g for 15 min at 4°C. The sample provided by the University of Tokyo was weighed, suspended in 9-fold volumes of PBS, and centrifuged at 50 g for 15 minutes at 4°C. The supernatant was collected in another 50 ml tube, suspended well, and then dispensed at 1 ml into 1.5 ml tubes and stored at -80°C as bacterial frozen stocks of the human feces sample.

Measurement of bacterial count concentration in bacterial frozen stocks of human feces sample

Bacterial frozen stocks of the human feces sample was thawed in an anaerobic chamber in the same manner as in Example 1. 10 $\mu$l of bacterial frozen stocks was taken and diluted 1,000-fold with PBS. Bacterial counts were measured according to the Cell Viability kit (BD) protocol. In the present study, in order to target all bacteria contained in a human feces sample, the bacterial count was measured using only thiazole orange (TO), which detects bacteria by staining nucleic acid, without using propidium iodide (PI), which detects dead bacteria. PBS, Liquid Counting Beads (BD), and thiazole orange (final concentration 42 nM, BD) were added to the bacterial dilution and reacted on ice for 10 minutes. The solution for bacterial count was subjected to flow cytometry analysis using a SONY SA3800 Analyzer (Sony Group Corporation). The bacterial count concentration of the bacterial dilution was calculated based on the number of Counting Beads detected in the analysis.

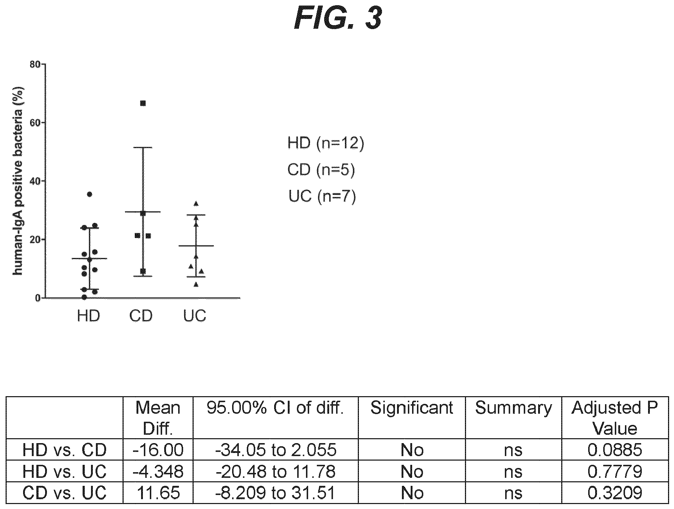

Analysis of ratio of human endogenous IgA antibody-binding bacteria (Fig. 3) to bacteria in human feces sample

The procedure was performed in an anaerobic chamber. After measuring the bacterial count, bacteria derived from human feces were prepared to be $6 \times 10^7$ bacteria/ml by adding PBS. Then, centrifugation was performed at 8,000 g for 5 minutes at 4°C, and the supernatant was removed. 100 $\mu$l of PBS containing 20% normal rat serum (FUJIFILM Wako Pure Chemical Corporation) was added to the cells with the supernatant removed, which were then allowed to react on ice for 30 minutes. This prevented non-specific binding of the antibody. FACS buffer was added to the bacterial solution, which was then centrifuged at 8,000 g for 5 minutes at 4°C, and the supernatant was removed. 15 $\mu$l of Anti-IgA-PE human antibody (Miltenyi Biotec) was added to $6 \times 10^6$ of bacteria count, and was reacted on ice for 20 minutes. FACS buffer was added to the antibody reaction solution, which was then centrifuged at 8,000 g for 5 minutes at 4°C, and the supernatant was removed. FACS buffer and Thiazole Orange (final concentration 42 nM, BD) were added to the bacteria, and the mixture was reacted on ice for 10 minutes. Thereafter, the ratio of total endogenous IgA antibody-binding bacteria was analyzed with SONY Cell Sorter SH800 (Sony Group Corporation).

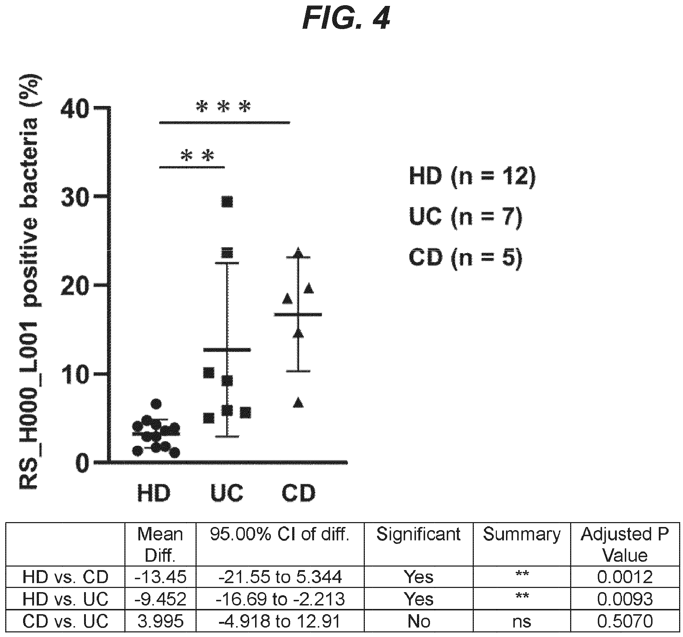

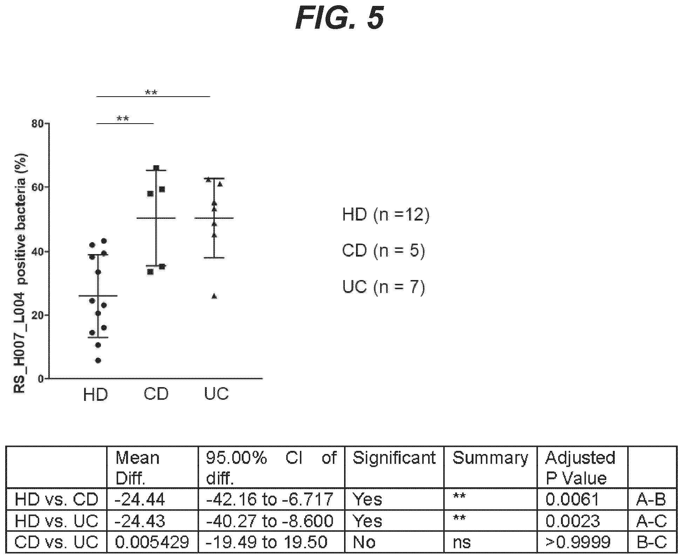

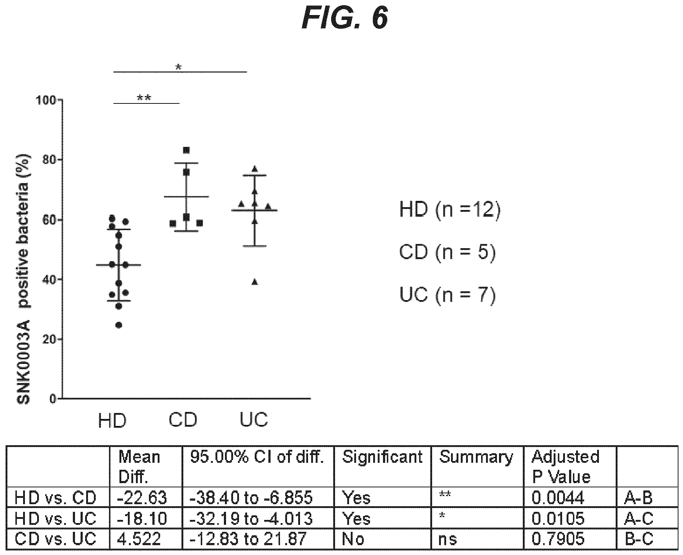

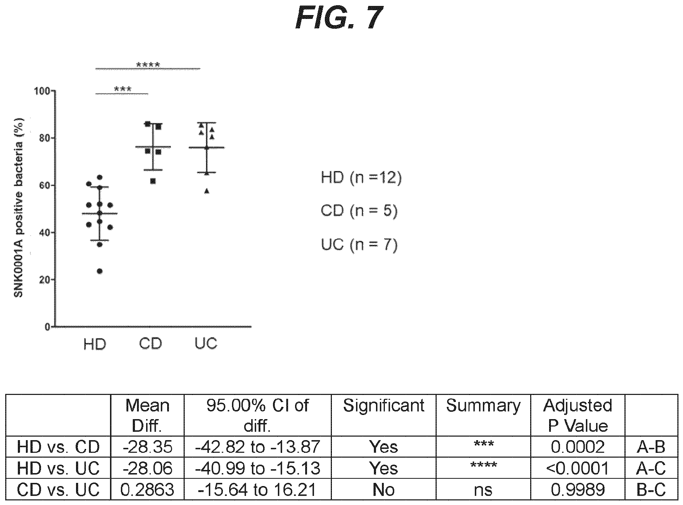

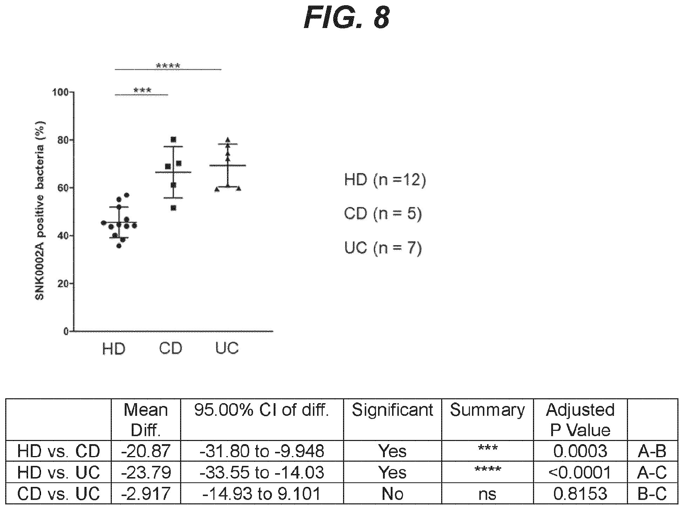

Analysis of ratios of RS_H000_L001 IgA antibody-binding bacteria (Fig. 4), RS_H007_L004 antibody-binding bacteria (Fig. 5), SNK0003A antibody-binding bacteria (Fig. 6), SNK0001A antibody-binding bacteria (Fig. 7), and SNK0002A antibody-binding bacteria (Fig. 8) to bacteria in human feces sample

The procedure was performed in an anaerobic chamber. After measuring the bacterial count, PBS was added to the bacteria in the human feces sample to prepare $6 \times 107$ bacteria/ml. Then, centrifugation was performed at 8,000 g for 5 minutes at 4°C, and the supernatant was removed. 100 $\mu$l of PBS containing 20% normal rat serum (FUJIFILM Wako Pure Chemical Corporation) was added to the cells with the supernatant removed, which were then allowed to react on ice for 30 minutes. This prevented non-specific binding of the antibody. Fluorescence activated cell sorter (FACS) buffer for antibody staining was prepared by adding 10% fetal bovine serum (FBS, NICHIREI BIOSCIENCES INC.) and EDTA (final concentration 5 $\mu$M, NACALAI TESQUE, INC.) to PBS, and sterilizing the mixture through a 0.22 $\mu$m filter. FACS buffer was added to the bacterial solution, which was then centrifuged at 8,000 g for 5 minutes at 4°C, and the supernatant was removed. 15 $\mu$g of each biotinylated antibody was added to $6 \times 106$ bacteria, and the mixture was reacted on ice for 20 minutes. FACS buffer was added to the antibody reaction solution, which was then centrifuged at 8,000 g for 5 minutes at 4°C, and the supernatant was removed. 20 $\mu$l of PE/Cyanine7-Streptavidin(10 $\mu$g/ml final concentration, BioLegend, Inc.) was added to the bacterial cells, and the reaction was performed for 20 minutes while shielding from light on ice. FACS buffer was added to the reaction solution, and the mixture was centrifuged at 8,000 g for 5 minutes at 4°C to remove the supernatant. FACS buffer and Thiazole Orange (final concentration 42 nM, BD) were added to the bacteria, and the mixture was reacted on ice for 10 minutes. Thereafter, the ratio of each antibody-binding bacteria was analyzed with SONY Cell Sorter SH800 (Sony Group Corporation).

Analysis of ratio of RS_H000_L001 antibody-binding bacteria to bacteria in human feces sample

The procedure was performed in an anaerobic chamber. After measuring the bacterial count, PBS was added to the bacteria in the human feces sample to prepare $6 \times 10^7$ bacteria/ml. Then, centrifugation was performed at 8,000 g for 5 minutes at 4°C, and the supernatant was removed. 100 $\mu$l of PBS containing 20% normal rat serum (FUJIFILM Wako Pure Chemical Corporation) was added to the cells with the supernatant removed, which were then allowed to react on ice for 30 minutes. This prevented non-specific binding of the antibody. Fluorescence activated cell sorter (FACS) buffer for antibody staining was prepared by adding 10% fetal bovine serum (FBS, NICHIREI BIOSCIENCES INC.) and EDTA (final concentration 5 $\mu$M, NACALAI TESQUE, INC.) to PBS, and sterilizing the mixture through a 0.22 $\mu$m filter. FACS buffer was added to the bacterial solution, which was then centrifuged at 8,000 g for 5 minutes at 4°C, and the supernatant was removed. 15 $\mu$g of biotinylated RS_H000_L001 antibody was added per $6 \times 10^6$ bacteria, and was allowed to react on ice for 20 minutes. FACS buffer was added to the antibody reaction solution, which was then centrifuged at 8,000 g for 5 minutes at 4°C, and the supernatant was removed. 20 $\mu$l of PE/Cyanine7-Streptavidin(10 $\mu$g/ml final concentration, BioLegend, Inc.) was added to the bacterial cells, and the reaction was performed for 20 minutes while shielding from light on ice. FACS buffer was added to the reaction solution, and the mixture was centrifuged at 8,000 g for 5 minutes at 4°C to remove the supernatant. FACS buffer and Thiazole Orange (final concentration 42 nM, BD) were added to the bacteria, and the mixture was reacted on ice for 10 minutes. Thereafter, the ratio of RS_H000_L001 antibody-binding bacteria was analyzed with SONY Cell Sorter SH800 (Sony Group Corporation).

Gemella morbillorum growth inhibition test

The procedure was performed in an anaerobic chamber. For culturing Gemella morbillorum (G. morbillorum, ATCC27824 strain), a medium prepared by adding mutton defibrated blood (Japan Bioserum Co., Ltd., final concentration 5%) to Tryptic Soy Broth (BD) was used. G. morbillorum was raised from a glycerol stock and cultured in 10 ml of medium overnight at 37°C. 450 $\mu$l of the medium was added to 50 $\mu$l of the bacterial solution, and the mixture was centrifuged at 8,000 g for 5 minutes at room temperature to remove the supernatant. After 500 $\mu$l of the culture medium was added and the bacterial count was measured, the culture medium was added to prepare a bacterial solution to $2 \times 10^3$ cells/ul. 25 $\mu$l of PBS or RS_H000_L001 IgA antibody (1 mg/ml) was added to 5 $\mu$l of the bacterial solution, and the mixture was reacted at 37°C for 1 hour. After the reaction, 30 $\mu$l of the medium was added, and the mixture was cultured at 37°C for 8 hours. After the culturing, the bacterial solution was seeded on the agar medium, and cultured overnight at 37°C, and the number of colonies was measured.

(Results)

As a result of searching for an IgA antibody having high binding capacity to IBD-related bacteria in feces of IBD patients, it has been found that RS_H000_L001 antibody and the variants thereof (RS_H007_L004), SNK0003A, and SNK0001A SNK0002A have high binding capacity to IBD-related bacteria with respect to bacteria obtained from feces of IBD patients (Figs. 3 to 8). Fig. 3 shows results obtained by FACS analysis of the ratio of bacteria that bind to total endogenous IgA antibodies (the number of binding bacteria/the number of total bacteria in a feces sample) among bacteria obtained from feces of healthy persons or IBD patients (Crohn's disease: CD, ulcerative colitis: UC). Regarding the ratio of bacteria that bind to total endogenous IgA antibody, no significant difference was observed in the binding ratio to the antibody sample between bacteria derived from healthy person feces and bacteria derived from IBD patient feces (Fig. 3). On the other hand, when RS_H000_L001 and the variants thereof (RS_H007_L004), SNK0003A, SNK0001A, and SNK0002A were used as antibody samples, the binding ratio of bacteria derived from healthy person feces was low, whereas bacteria derived from IBD patient feces showed a significantly high binding ratio to these antibody samples (Figs. 4 to 8).

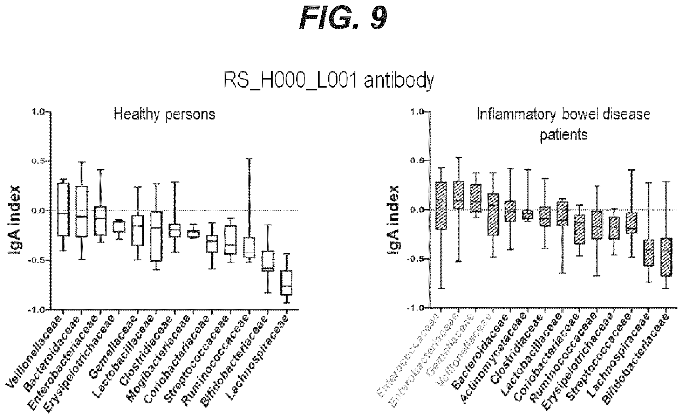

As a result of confirming the binding characteristics of the RS_H000_L001 IgA antibody to bacteria in feces obtained from healthy persons or IBD patients, for bacteria in feces derived from healthy persons, bacteria showing particularly specific binding was not confirmed, whereas for bacteria collected from feces obtained from IBD patients, binding was shown to Enterococcaceae and Enterobacteriaceae, Gemellaceae, and Veillonellaceae known to be associated with the induction of enteritis (Fig. 9).

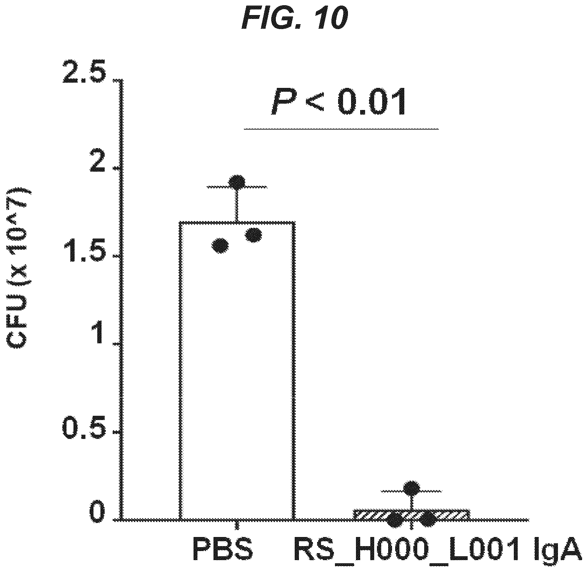

Gemellaceae is one of bacteria to which an IgA antibody derived from healthy persons binds in feces derived from healthy persons (Fig. 1), but RS_H000_L001 antibody showed a strong growth inhibitory effect on Gemella morbillorum in an in vitro growth inhibitory test (Fig. 10). From these results, it has been suggested that the IgA antibody having a high binding capacity to IBD-related bacteria can improve the intestinal flora and improve IBD disease by suppressing the growth of IBD-related bacteria in the gastrointestinal tract of IBD patients.

Example 4: IBD therapeutic effect of feces processed with IgA antibody having high binding capacity to IBD-related bacteria and oral administration of IgA antibody

(Methods and materials)

Mice used for human feces sample transplantation

BALB/c background activation-induced cytidine deaminase (AID) knockout sterile mice deficient in endogenous IgA were used. Mice were housed in sterile vinyl isolators. Male mice aged 5 to 19 weeks were used. At the time of sample collection after the end of the experiment, mice were euthanized with cervical dislocation.

Transplantation of bacteria into mice by oral administration of enterobacteria from IBD patient samples (P10 and P13)

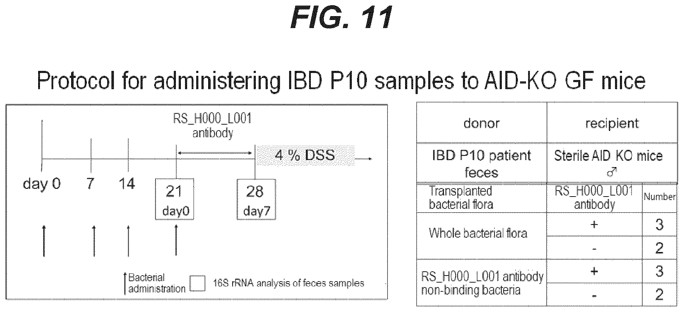

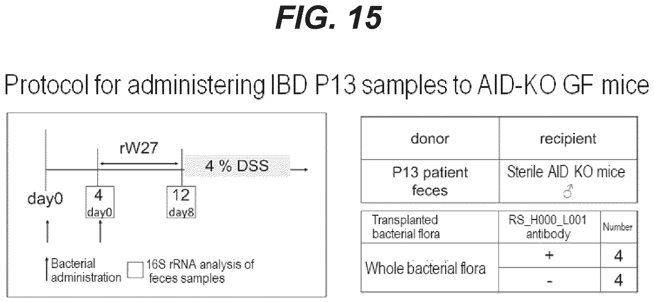

The procedure was performed in an anaerobic chamber in the same manner as in Example 3. After measuring the bacterial count, PBS was added to the bacterial suspension to prepare $3 \times 10^8$ cells/tube. Then, centrifugation was performed at 8,000 g for 5 minutes at 4°C, and the supernatant was removed. 100 $\mu$l of sterile PBS was added thereto, and the mixture was orally administered to a mouse. The number and interval of oral administration of the bacterial solution were different between P10 and P13. A schematic diagram and details of the experiment are shown in Figs. 11 and 15, respectively.

Removal of RS_H000_L001 antibody-binding bacteria in the IBD patient sample (P10) by column

The procedure was performed in an anaerobic chamber. After measuring the bacteria count, PBS was added to the bacteria of the human feces sample to prepare $3 \times 10^8$ bacteria/tube. Then, centrifugation was performed at 8,000 g for 5 minutes at 4°C, and the supernatant was removed. 100 $\mu$l of PBS containing 20% normal rat serum (FUJIFILM Wako Pure Chemical Corporation) was added to the cells with the supernatant removed, which were then allowed to react on ice for 30 minutes to block nonspecific binding. FACS buffer was added to the bacterial solution, which was then centrifuged at 8,000 g for 5 minutes at 4°C, and the supernatant was removed. 15 $\mu$g of biotinylated RS_H000_L001 IgA antibody was added per $6 \times 10^6$ bacteria, and the mixture was allowed to react on ice for 40 minutes. FACS buffer was added to the antibody reaction solution, which was then centrifuged at 8,000 g for 5 minutes at 4°C, and the supernatant was removed. RS_H000_L001 antibody-binding bacteria were removed using a magnetic bead separation (MACS) kit of VERITAS Corporation. Myone C1-streptavidin beads (VERITAS Corporation) were used to remove RS_H000_L001 IgA antibody-binding bacteria from the P10 feces sample. 15 $\mu$l of beads prepared according to the bead washing protocol of VERITAS Corporation was added to 15 $\mu$g of biotinylated RS_H000_L001 IgA antibody, and the mixture was allowed to react on ice for 30 minutes. Bacterial isolation procedures were performed according to the MACS bacterial isolation protocol of VERITAS Corporation. The bacteria adsorbed to the magnet were collected as RS_H000_L001 IgA antibody-binding bacteria, and the bacteria collected as the flow-through were collected as RS_H000_L001 antibody-non-binding bacteria, and were suspended in 500 $\mu$l of FACS buffer. 5 $\mu$l of RS_H000_L001 antibody non-binding bacteria was collected, and FACS buffer, TO (final concentration 42 nM, BD), and Phycoerythrin (PE)-anti mouse IgA antibody (final concentration 5 $\mu$g/ml, BioLegend, Inc.) were added, and the reaction was performed for 10 minutes while shielding from light on ice. Subsequent FACS analysis using a SONY Cell Sorter SH800 (Sony Group Corporation) confirmed that only non-binding bacteria were collected at a ratio of 90% or more. The remaining RS_H000_L001 antibody non-binding bacteria were centrifuged at 8,000 g for 5 minutes at 4°C, and the supernatant was removed. The solution was adjusted to 100 $\mu$l with PBS and orally administered to mice.

RS_H000_L001 Oral administration of IgA antibody

From the final day of administration of the bacterial solution, the RS_H000_L001 antibody was orally administered to mice via a stomach tube once a day for 7 days (P10) or 8 days (P13). A single dosage was prepared in 200 $\mu$l of PBS so as to be 100 $\mu$g/mice of RS_H000_L001 antibody.

Dextran sulfate sodium (DSS) induced enteritis model mouse

From the day after the last day of RS_H000_L001 antibody administration, 4% dextran sulfate sodium (DSS (MP BIOMEDICALS)) was added to a water supply bottle, and orally administered to mice to induce enteritis. This was added to sterile water so as to have a final concentration of DSS of 4%, and administered in free drinking for 7 days. Body weight and feces scores of mice were measured daily.

Freezing and tissue staining of large intestinal tissue

On 7th day of DSS administration, mice were euthanized by cervical dislocation, and the mice were dissected and large intestines were removed. The contents of large intestine tissues were washed, frozen with liquid nitrogen using optimum cutting temperature (OCT) compound (Sakura Finetek Japan Co., Ltd.), and then stored at -80°C.

A tissue section having a thickness of 6 μm was prepared from the large intestine tissue of the mouse cryopreserved with OCT compound. The tissue section was fixed with acetone for 15 minutes, subjected to hematoxylin-eosin staining and alcian blue staining, and observed with an optical microscope. For hematoxylin and eosin staining, Hematoxylin solution modified acc. to Gill III (Merck KGaA), an eosin alcohol solution, and an acid extract (FUJIFILM Wako Pure Chemical Corporation) were used. As the alcian blue staining, an alcian blue solution (pH 2.5 (FUJIFILM Wako Pure Chemical Corporation)) and a Kernechtrot solution (MUTO PURE CHEMICALS CO., LTD.) were used.

Method for extracting bacterial DNA derived from mouse feces

Mouse feces samples were collected before (0 day) and after administration of RS_H000_L001 IgA antibody. Mouse feces samples were weighed and the 9-fold amount of sterile PBS was added. The suspension was performed by vortexing and centrifuged at 50 g for 15 minutes at 4°C. The supernatant was collected in a 1.5 ml tube and centrifuged at 8,000 g for 5 minutes at 4°C to remove the supernatant. DNA Lysis Buffer for DNA extraction was prepared by adding Tris-HCl (final concentration 50 mM, NACALAI TESQUE, INC.), NaCl (final concentration 300 mM, NACALAI TESQUE, INC.), EDTA (final concentration 1 mM, NACALAI TESQUE, INC.), and 0.5% SDS (NACALAI TESQUE, INC.) to ultrapure water. 500 μl of DNA Lysis Buffer was added to the bacterial cells to be suspended, then the suspension was transferred to a sterilized 2.0 ml tube (TM-625S, TOMY SEIKO CO., LTD.) containing glass beads (GB-01, 0.1Φ, TOMY SEIKO CO., LTD.), and bead disruption was performed at 3,500 rpm for 1 minute with a bead type cell disruptor (MS-100, TOMY SEIKO CO., LTD.). Protein kinase K (final concentration 0.5 mg/ml, NACALAI TESQUE, INC.) was added to the bacterial suspension with beads crushed, and the mixture was vortexed and allowed to stand at 55°C overnight to completely dissolve the bacterial cells. The temperature was returned from 55°C to room temperature, and 500 μl of phenol chloroform-isoamyl alcohol = 24 : 25 : 1 (NACALAI TESQUE, INC.) was added to the cell lysate, mixed by vortexing, and centrifuged at 13,000 rpm, 4°C for 5 minutes, and the upper layer was collected in another 1.5 ml tube. Chloroform (NACALAI TESQUE, INC.) was added in an amount equivalent to the amount of the collected solution, mixed by vortexing, and centrifuged at 13,000 rpm for 5 minutes at 4°C. Again, the upper layer was collected in another 1.5 ml tube, isopropanol (NACALAI TESQUE, INC.) and glycogen (final concentration 0.04 mg/ml, NACALAI TESQUE, INC.) were added in amounts equivalent to the collected amounts, and the mixture was mixed by inversion. The DNA solution mixed by inversion was allowed to stand at -20°C for 20 minutes, and then centrifuged at 13,000 rpm for 10 minutes at 4°C to remove the supernatant. 500 μl of 70% ethanol was added to the precipitated DNA, and the mixture was centrifuged at 13,000 rpm for 5 minutes at 4°C to remove the supernatant. The precipitated DNA was air-dried, and then ultra pure distilled water (Invitrogen) was added to dissolve the DNA. The concentration of DNA was measured using Bio Drop DUO (Biochrome).

(Results)

For a specific IBD patient (P10), RS_H000_L001 IgA antibody non-binding bacteria from which bacteria binding to RS_H000_L001 IgA antibody exhibiting high binding capacity to the total bacterial flora obtained from the feces of the patient or IBD-related bacteria were removed were orally administered to IBD model mice a plurality of times. When the RS_H000_L001 IgA antibody-binding bacteria in the bacterial flora of the IBD patient (P10) were analyzed, a high IgA Index for Enterobacteriaceae and the like and a low IgA Index for Bifidobacteriaceae and the like were shown, and in addition to oral administration of bacteria, the RS_H000_L001 IgA antibody was orally administered to some of these mice. That is, the test groups were 4 groups of (1) total bacterial flora/antibody non-administration group, (2) total bacterial flora/RS_H000_L001 IgA antibody oral administration group, (3) RS_H000_L001 IgA antibody non-binding bacteria/antibody non-administration group, and (4) RS_H000_L001 IgA antibody non-binding bacteria/RS_H000_L001 IgA antibody oral administration group (Fig. 11).

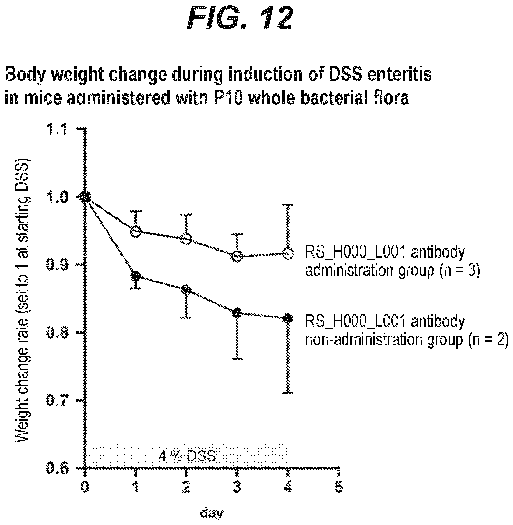

As a result, (1) in the total bacterial flora/antibody non-administration group, the body weight loss considered to be associated with the onset of DSS-induced enteritis after oral administration of bacteria was shown, whereas (2) in the total bacterial flora/RS _H000_L001 IgA antibody oral administration group, the body weight loss was significantly suppressed (Fig. 12).

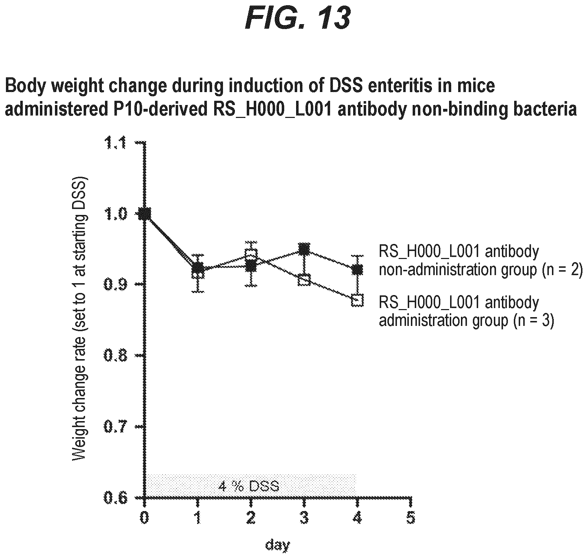

On the other hand, in the (3) RS_H000_L001 IgA antibody non-binding bacteria/antibody non-administration group, weight loss considered to be associated with the onset of DSS-induced enteritis after oral administration of bacteria was hardly observed, and it has been suggested that modifying the bacterial flora of IBD patient feces with RS_H000_L001 IgA antibody reduces the enteritis onset action. Further, the body weight change of the (4) RS_H000_L001 IgA antibody non-binding bacteria/RS_H000_L001 IgA antibody oral administration group in which the oral administration of the RS_H000_L001 IgA antibody was combined was almost the same as that of the (3) RS_H000_L001 IgA antibody non-binding bacteria/antibody non-administration group (Fig. 13).

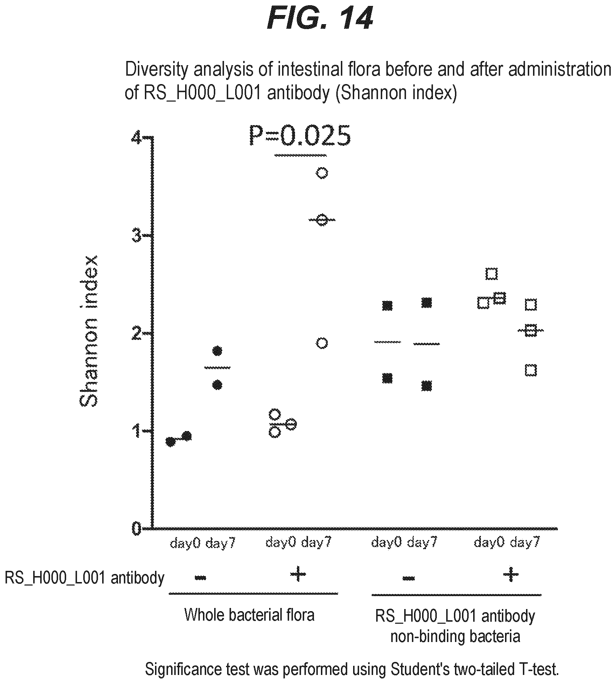

In addition, when the diversity analysis of the intestinal flora of the mice after the treatment was performed, compared with the (1) total bacterial flora/antibody non-administration group, the (2) total bacterial flora/RS_H000_L001 IgA antibody oral administration group, the (3) RS_H000_L001 IgA antibody non-binding bacteria/antibody non-administration group, and the (4) RS_H000_L001 IgA antibody non-binding bacteria/RS_H000_L001 IgA antibody oral administration group showed higher diversity (Fig. 14).

From these results, it has become clear that when treating IBD patients with autologous fecal transplantation (bacterial flora transplantation), concurrently orally administering an IgA antibody having a high binding capacity to IBD-related bacteria or modifying bacterial flora contained in the autologous feces with an IgA antibody having a high binding capacity to IBD-related bacteria allows the intestinal flora to be improved and IBD disease symptoms can be improved in IBD patients.

A similar test was performed using a bacterial flora derived from feces of the IBD patient (P13) different from the IBD patient (P10). As shown in Fig. 15, the purpose of this test was to test the antibody combined effect of the entire bacterial flora and the RS_H000_L001 IgA antibody in more detail.

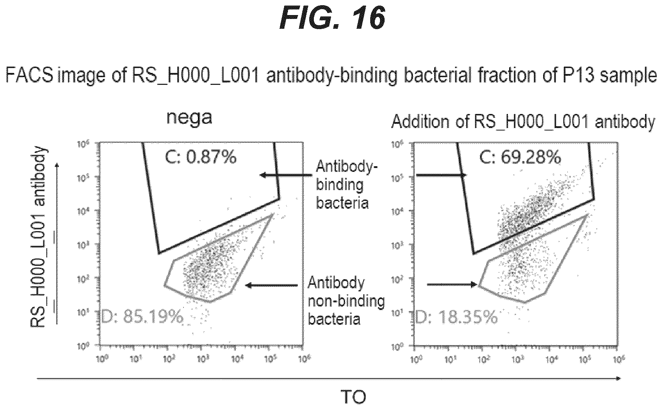

As a result of FACS analysis, it has been observed that about 70% of the bacterial flora from the feces of the IBD patient (P13) binds to the RS_H000_L001 antibody (Fig. 16).

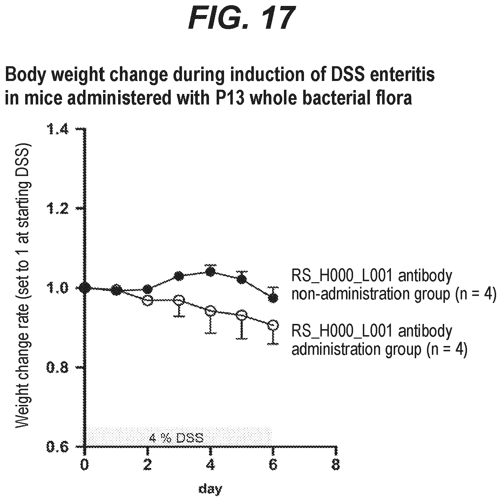

Regarding the (1) total bacterial flora/antibody non-administration group and the (2) total bacterial flora/RS_H000_L001 IgA antibody oral administration group, the body weight change after oral administration of bacteria was observed, and as for the bacterial flora derived from the feces of the IBD patient (P13), the body weight change was hardly observed also for the (1) total bacterial flora/antibody non-administration group, and the body weight change was almost the same for the (2) total bacterial flora/RS_H000_L001 IgA antibody oral administration group (Fig. 17).

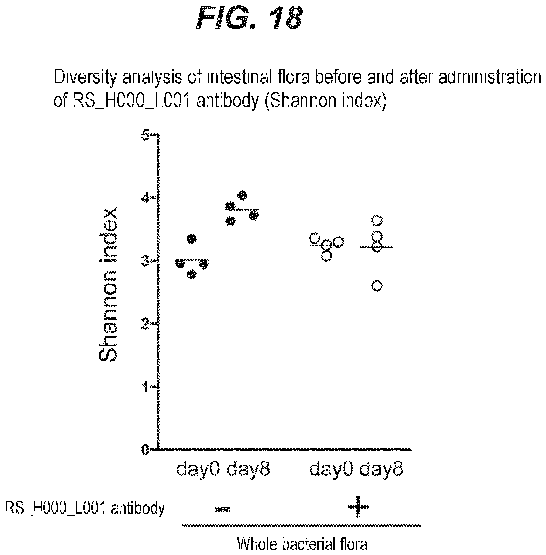

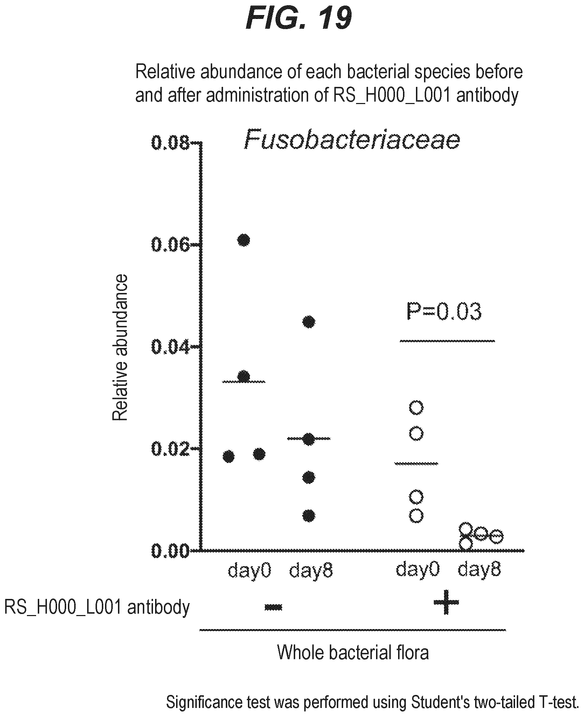

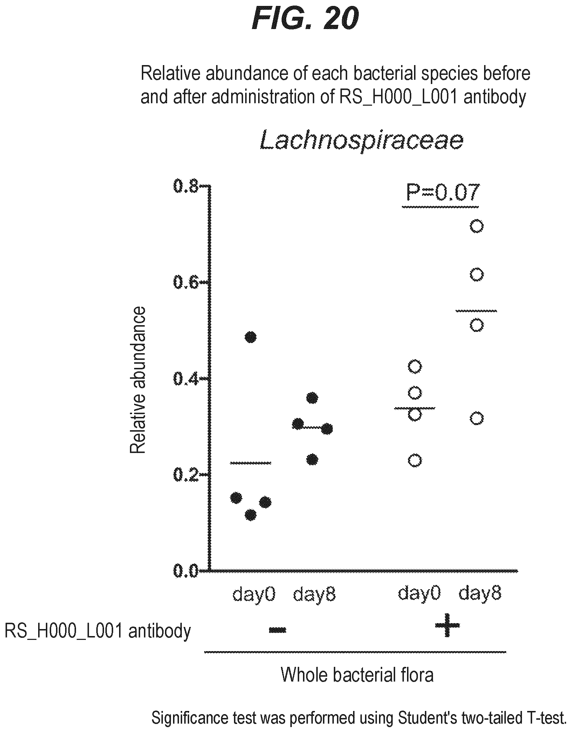

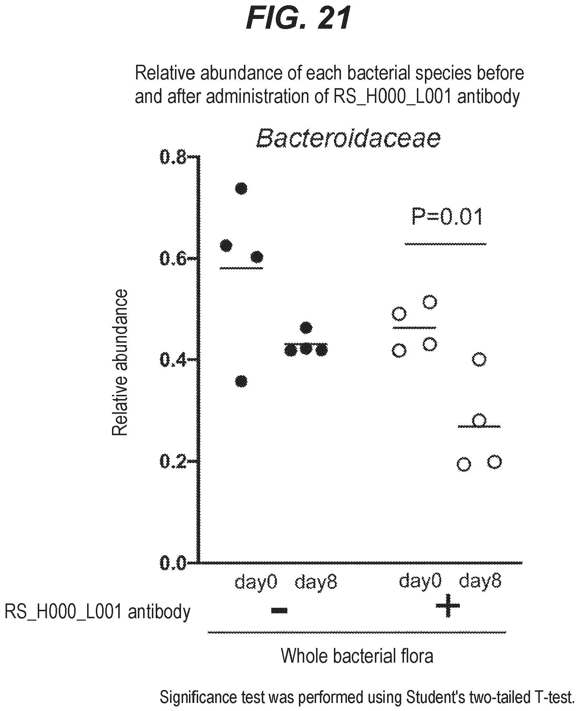

In addition, when diversity analysis of the intestinal flora after treatment was performed (Figs. 18 to 21), there was almost no change in Shannon index (Fig. 18). On the other hand, when the relative abundance ratios of various bacteria were confirmed, a decrease in the relative abundance ratios of Fusobacteriaceae and Bacteroidaceae that have been indicated to be associated with IBD was observed in both of the (1) total bacterial flora/antibody-non-administered group and the (2) total bacterial flora/RS_H000_L001 IgA antibody orally administration group (Fig. 19, Fig. 21), and on the contrary, an increase in the relative abundance ratio of Lachnospiraceae was observed (Fig. 20).

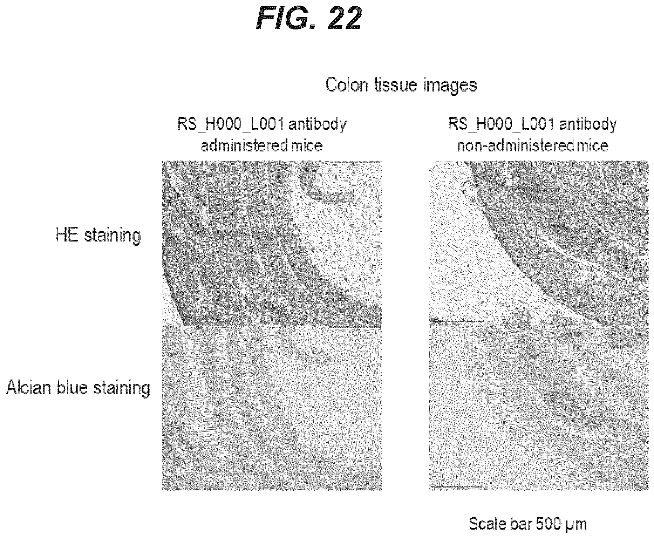

Further, when the large intestine tissue section after the treatment was observed, in the (1) total bacterial flora/antibody non-administration group, abnormalities were observed in the structure of tissues and cells, whereas in the (2) total bacterial flora/RS_H000_L001 IgA antibody oral administration group, normal structures of tissues and cells were observed (Fig. 22).

These results also support that, when treating IBD patients with autologous fecal transplantation (bacterial flora transplantation), concomitant oral administration of an IgA antibody having a high binding capacity to IBD-related bacteria can improve the intestinal flora and improve IBD disease symptoms in IBD patients. In addition, in the mouse administration test using the bacterial flora derived from the feces of the IBD patient (P10), weight loss considered to be associated with the onset of DSS-induced enteritis markedly occurred when the IgA antibody was not used in combination, whereas in the mouse administration test using the bacterial flora derived from the feces of the IBD patient (P13), the remarkable weight loss was not observed, suggesting that the bacterial flora aspect involved in the onset of IBD patients is different in each of the IBD patients. In the present invention, it is possible to obtain a more effective IBD improving effect by using an IgA antibody that improves the bacterial flora in the gastrointestinal tract of an individual patient.

Example 5: Further IgA antibody with high binding capacity to IBD-related bacteria

Further IgA antibodies with high binding capacity for IBD-related bacteria were tested in a manner similar to that of Example 3.

The binding ratio of bacteria derived from feces of healthy persons was compared with that of bacteria derived from feces of IBD patients for the SNK0004 and SNK0005 antibodies, which are IgA antibody clones isolated from mouse intestinal mucosa.

The amino acid sequences of the heavy and light chains of SNK0004 and SNK0005 are shown below.

| SNK0004 amino acid sequence | | |
|---|---|---|
| Heavy chain | Full-length sequence | MKFSWVIFFLMAVVTGVNSEVQLQQSLAELVRPGASVKLSCTASGFNIK NTYMHWVKQRPEQDLEWIGRIDPANGYTKYAPKFQGKATITADTSSNAA YLQLSSLTSEDTAIYYCGRGYSNFDYWGQGTTLTVSSESARNPTIYPLT LPRALSSDPVIIGCLIHDYFPSGTMNVTWGKSGKDITTVNFPPALASGG GYTMSSQLTLPAVECPEGESVKCSVQHDSNAVQELDVKCSGPPPPCPPC PPSCHPSLSLQRPALEDLLLGSDASLTCTLNGLRNPEGAVFTWEPSTGK DAVQKKAVQNSCGCYSVSSVLPGCAERWNSGASFKCTVTHPESDTLTGT IAKITVNTFPPQVHLLPPPSEELALNELVSLTCLVRAFNPKEVLVRWLH GNEELSPESYLVFEPLKEPGEGATTYLVTSVLRVSAELWKQGDQYSCMV GHEALPMNFTQKTIDRLSGKPTNVSVSVIMSEGDGICY |
| | Variable region | MKFSWVIFFLMAVVTGVNSEVQLQQSLAELVRPGASVKLSCTASGFNIK NTYMHWVKQRPEQDLEWIGRIDPANGYTKYAPKFQGKATITADTSSNAA YLQLSSLTSEDTAIYYCGRGYSNFDYWGQGTTLTVSS |
| | CDR1 | GFNIKNTY |
| | CDR2 | IDPANGYT |
| | CDR3 | GRGYSNFDY |
| Light chain | Full-length sequence | MDMRTPAQFLGILLLWFPGFKCDIKMTQSPSSMYASLGERVTITCKASQ DINSYLSWFQQKPGKSPKTLTYRANRLVDGVPSKFSGSGSGQDYSLTIS SLEYEDMGIYYCLQYDEFPLTFGAGTKLELKRADAAPTVSIFPPSSEQL TSGGASVVCFLNNFYPKDINVKWKIDGSERQNGVLNSWTDQDSKDSTYS MSSTLTLTKDEYERHNSYTCEATHKTSTSPIVKSFNRNEC |
| | Variable region | MDMRTPAQFLGILLLWFPGFKCDIKMTQSPSSMYASLGERVTITCKASQ DINSYLSWFQQKPGKSPKTLTYRANRLVDGVPSKFSGSGSGQDYSLTIS SLEYEDMGIYYCLQYDEFPLTFGAGTKLELK |
| | CDR1 | QDINSY |
| | CDR2 | RAN |
| | CDR3 | LQYDEFPLT |

| SNK0005 amino acid sequence | | |
|---|---|---|
| Heavy chain | Full-length sequence | MGWSWIFLLFLSGTAGVLSEVQLQQSGPEVVEPGASVKIPCKASGYTFT DYNMDWVKQSHGKSLEWIGDINPNNGGTIYNQKFKGQATLTVDKSSSTA YMELRSLTSEDTAVYYCAKSGYYGSGRYFDVWGTGTTVTVSSESARNPT IYPLTLPRALSSDPVIIGCLIHDYFPSGTMNVTWGKSGKDITTVNFPPA LASGGGYTMSSQLTLPAVECPEGESVKCSVQHDSNAVQELDVKCSGPPP PCPPCPPSCHPSLSLQRPALEDLLLGSDASLTCTLNGLRNPEGAVFTWE PSTGKDAVQKKAVQNSCGCYSVSSVLPGCAERWNSGASFKCTVTHPESD TLTGTIAKITVNTFPPQVHLLPPPSEELALNELVSLTCLVRAFNPKEVL VRWLHGNEELSPESYLVFEPLKEPGEGATTYLVTSVLRVSAELWKQGDQ YSCMVGHEALPMNFTQKTIDRLSGKPTNVSVSVIMSEGDGICY |
| | Variable region | MGWSWIFLLFLSGTAGVLSEVQLQQSGPEVVEPGASVKIPCKASGYTFT DYNMDWVKQSHGKSLEWIGDINPNNGGTIYNQKFKGQATLTVDKSSSTA YMELRSLTSEDTAVYYCAKSGYYGSGRYFDVWGTGTTVTVSS |
| | CDR1 | GYTFTDYN |
| | CDR2 | INPNNGGT |
| | CDR3 | ARSGYYGSGRYFDV |

(continued)

| SNK0005 amino acid sequence | | |
|---|---|---|
| Light chain | Full-length sequence | MVSTPQFLVFLLFWIPASRGDILLTQSPVILSVSPGERVSFSCRASQSI GSSIHWYQQRTNGSPRLLIKYASESISGIPSRFSGSGSGTDFTLTINSV ASEDIADYYCQQSKSWPWTFGGGTKLEIKRADAAPTVSIFPPSSEQLTS GGASVVCFLNNFYPKDINVKWKIDGSERQNGVLNSWTDQDSKDSTYSMS STLTLTKDEYERHNSYTCEATHKTSTSPIVKSFNRNEC |
| | Variable region | MVSTPQFLVFLLFWIPASRGDILLTQSPVILSVSPGERVSFSCRASQSI GSSIHWYQQRTNGSPRLLIKYASESISGIPSRFSGSGSGTDFTLTINSV ASEDIADYYCQQSKSWPWTFGGGTKLEIK |
| | CDR1 | QSIGSS |
| | CDR2 | YAS |
| | CDR3 | QQSKSWPWT |

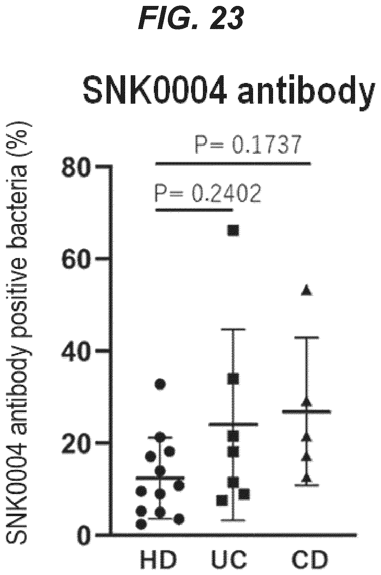

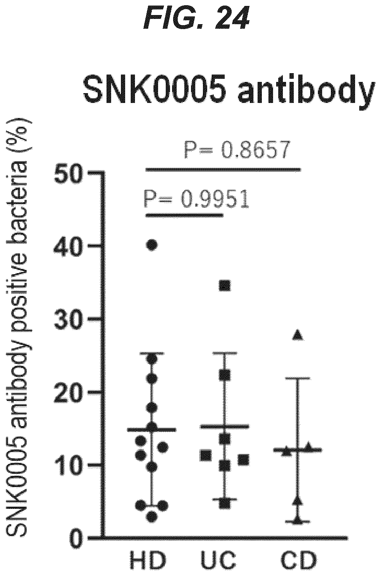

The results are shown in Figs. 23 and 24. For the SNK0004 antibody, the binding ratio of bacteria derived from feces of IBD patients that bound to the antibody was higher than the binding ratio of bacteria derived from feces of healthy persons that bound to the antibody. On the other hand, for SNK0005, there was no clear difference between the binding ratio of bacteria derived from feces of healthy persons and that derived from feces of IBD patients.

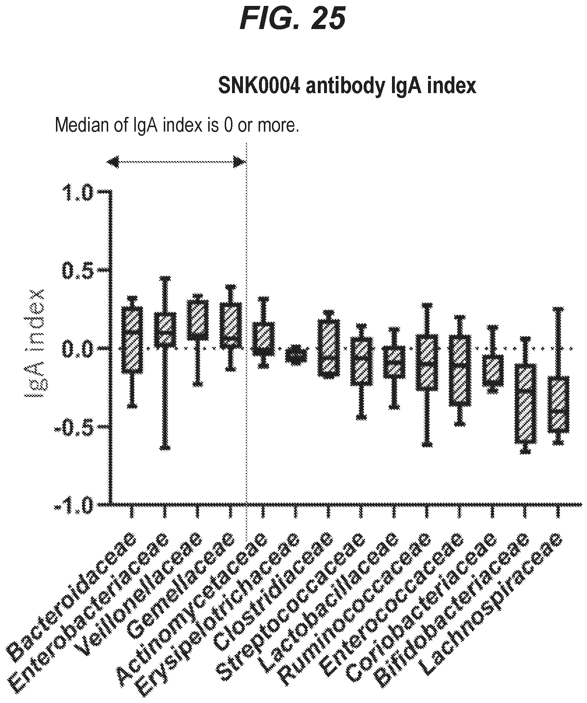

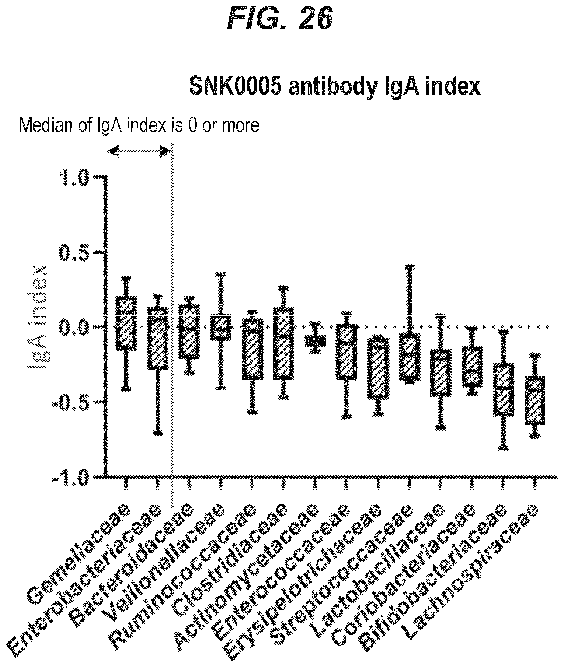

On the other hand, confirmation of the binding characteristics of individual bacterial species for the bacteria isolated from BD patient feces that bound to these antibodies has revealed that both SNK0004 and SNK0005 exhibited specific binding to Enterobacteriaceae, while other bacteria exhibited different binding characteristics (Figs. 25 and 26).

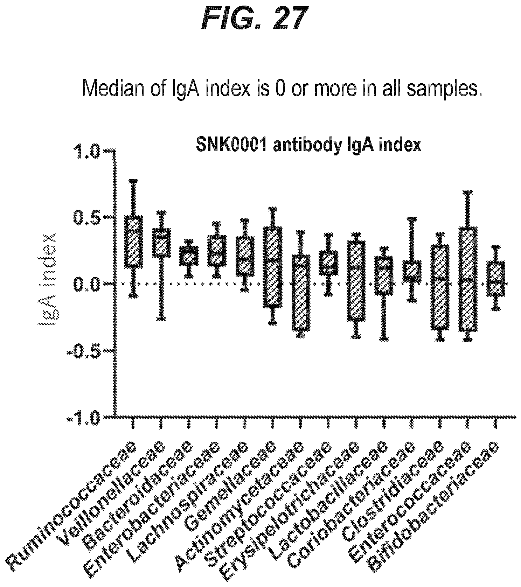

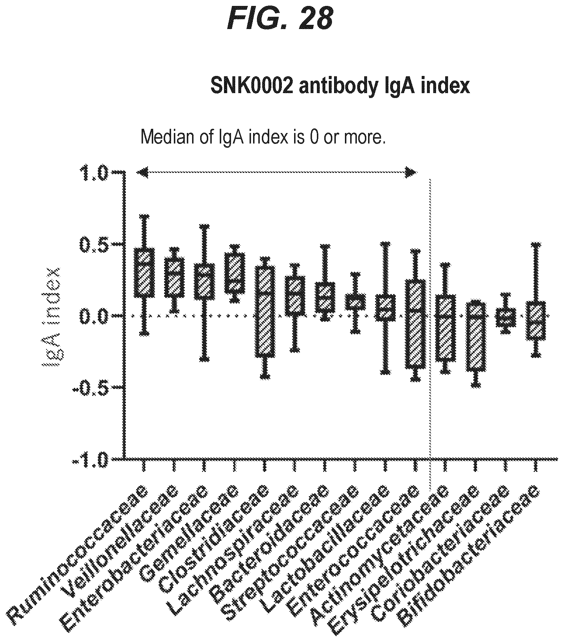

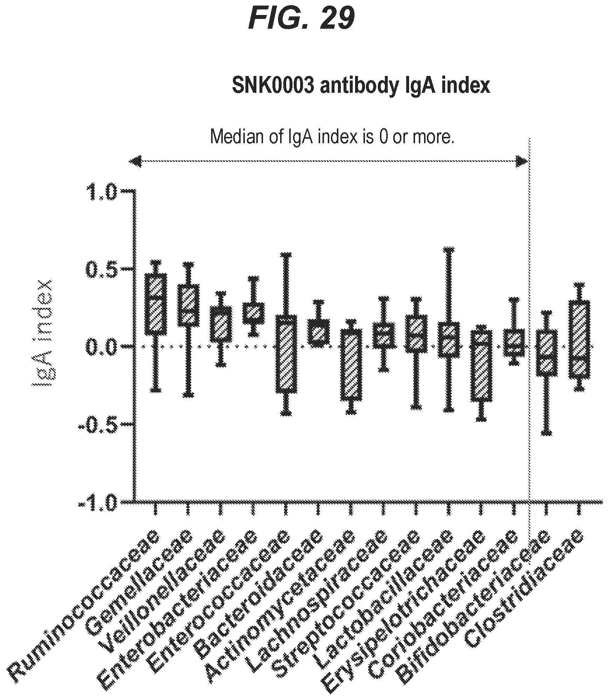

For the SNK0001, SNK0002, and SNK0003 antibodies used in Example 3, bacteria derived from feces samples of IBD patients that specifically bound to these antibodies were also confirmed (Figs. 27 to 29). These results indicate that even among antibodies that have high binding ratio of bacteria derived from the feces of IBD patients, the bacteria that exhibits specific binding to each antibody are different, compared to bacteria derived from the feces of healthy persons.

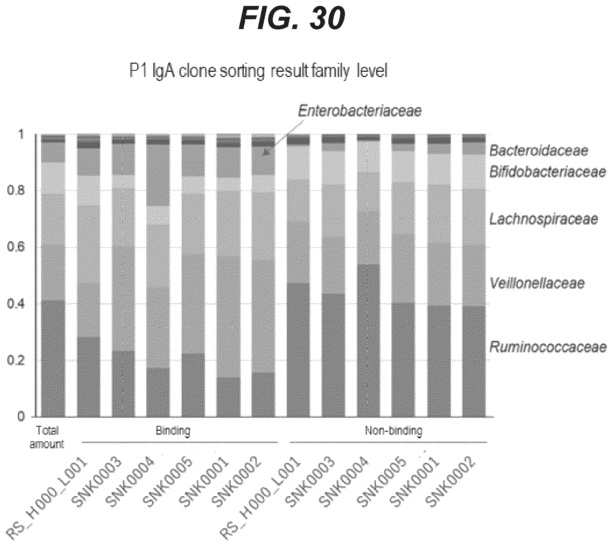

Fig. 30 shows the results of comparing the ratios of bacteria derived from feces samples of IBD patients that bind to the RS_H000_L001, SNK0001, SNK0002, SNK0003, SNK0004, and SNK0005 antibodies. These results indicate that all of these antibodies bind to the Enterobacteriaceae, and thus are considered to have a healthy effect on the intestinal flora, but that each antibody binds to different bacterial species.

It is considered that each IBD patient has different IBD-related bacteria having abnormal abundance ratio. These results demonstrate that different types of IgA antibodies can be used to test for the status of bacteria that exhibit abnormal abundance ratios in these IBD patients, and that depending on the test results, the IgA antibody more suitable for the patient can be used as a drug for restoring bacterial flora in a gastrointestinal tract and as an IBD therapeutic agent.

Industrial Applicability

In a treatment method for administering the gastrointestinal contents or excretions to treat IBD, the present disclosure has extremely high industrial value by providing a method for obtaining gastrointestinal contents or excretions that improves bacterial flora in a gastrointestinal tract by processing the gastrointestinal contents or excretions with an IgA antibody, a method for obtaining an IgA antibody related to the method, and a pharmaceutical composition for use in these methods.

**Claims**

1. A pharmaceutical composition for treating inflammatory bowel disease (IBD), comprising gastrointestinal contents or excretions processed with an IgA antibody that restores bacterial flora in a gastrointestinal tract.

2. A pharmaceutical composition for treating IBD, comprising an IgA antibody that is used in combination with a composition containing gastrointestinal contents or excretions and restores bacterial flora in a gastrointestinal tract.

3. The pharmaceutical composition according to claim 1 or 2, wherein an IgA antibody that restores the bacterial flora in a gastrointestinal tract has one or more of the following properties:

(1) an IgA antibody that binds to more IBD-related bacteria when brought into contact with gastrointestinal contents or excretions derived from an IBD patient, when the IgA antibody is brought into contact with gastrointestinal contents or excretions derived from an IBD patient or is brought into contact with gastrointestinal contents or excretions derived from a healthy person;

(2) an IgA antibody wherein when a first binding profile created by bringing gastrointestinal contents or excretions derived from an IBD patient into contact with the IgA antibody and based on a type and amount of bacteria bound to the IgA antibody is compared with a second binding profile created by bringing gastrointestinal contents or excretions derived from a healthy person into contact with the IgA antibody and based on a type and amount of bacteria bound to the IgA antibody, binding to more types of IBD-related bacteria or a greater amount of binding to IBD-related bacteria is observed in the first binding profile;

(3) an IgA antibody that increases bacterial flora diversity of gastrointestinal contents or excretions after processing with the IgA antibody when bacterial flora diversity of gastrointestinal contents or excretions derived from an IBD patient before processing with the IgA antibody is compared with bacterial flora diversity of gastrointestinal contents or excretions after processing with IgA antibody;

(4) an IgA antibody that increases bacterial flora diversity of gastrointestinal contents or excretions after administration when gastrointestinal contents or excretions modified with the IgA antibody are administered to a patient and bacterial flora diversity in a gastrointestinal tract of the patient before administration is compared with bacterial flora diversity of gastrointestinal contents or excretions after administration;

(5) an IgA antibody that increases a content of a short-chain fatty acid after administration when gastrointestinal contents or excretions modified with the IgA antibody are administered to a patient and a content of a short-chain fatty acid in a gastrointestinal tract of the patient before administration is compared with a content of a short-chain fatty acid in gastrointestinal contents or excretions of the patient after administration; and

(6) an IgA antibody that decreases a content of Enterobacteriaceae in gastrointestinal contents or excretions after administration when gastrointestinal contents or excretions modified with the IgA antibody are administered to a patient, and a content of Enterobacteriaceae in gastrointestinal contents or excretions of a subject before administration is compared with a content of Enterobacteriaceae in gastrointestinal contents or excretions after administration.

4. The pharmaceutical composition according to claim 1 or 2, wherein the gastrointestinal contents or excretions are derived from a subject to be administered with the composition.

5. A method for screening for IgA antibodies that restore bacterial flora in a gastrointestinal tract, the method comprising:

(B1) a step of preparing gastrointestinal contents or excretions of an IBD patient;
(B2) a step of modifying the gastrointestinal contents or excretions with a candidate IgA antibody; and
(B3) a step of confirming that the modified gastrointestinal contents or excretions are capable of restoring the bacterial flora in the gastrointestinal tract.

6. The method according to claim 5, wherein (B3) the step of confirming that modified gastrointestinal contents or excretions are capable of restoring bacterial flora in a gastrointestinal tract is performed by any of followings:

(1) confirming that the IgA antibody binds to more IBD-related bacteria when the IgA antibody is brought into contact with gastrointestinal contents or excretions derived from an IBD patient, when the IgA antibody is brought into contact with gastrointestinal contents or excretions derived from the IBD patient or is brought into contact with gastrointestinal contents or excretions derived from a healthy person;

(2) confirming that when a first binding profile created by bringing gastrointestinal contents or excretions derived from an IBD patients into contact with the IgA antibody and based on a type and amount of bacteria bound to the IgA antibody is compared with a second binding profile created by bringing gastrointestinal contents or excretions derived from a healthy person into contact with the IgA antibody and based on a type and amount of bacteria bound to the IgA antibody, binding to more types of IBD-related bacteria or a greater amount of binding to IBD-related bacteria is observed in the first binding profile;

(3) confirming that bacterial flora diversity of gastrointestinal contents or excretions after processing with the IgA antibody is increased when bacterial flora diversity of gastrointestinal contents or excretions derived from an IBD patient before processing with the IgA antibody is compared with bacterial flora diversity of gastrointestinal contents or excretions after processing with the IgA antibody;

(4) confirming that bacterial flora diversity of gastrointestinal contents or excretions after administration is increased when gastrointestinal contents or excretions modified with the IgA antibody are administered to a patient and bacterial flora diversity in a gastrointestinal tract of the patient before administration is compared with bacterial flora diversity of gastrointestinal contents or excretions after administration;

(5) increasing a content of a short-chain fatty acid after administration when gastrointestinal contents or excretions modified with the IgA antibody are administered to a patient and a content of a short-chain fatty acid in a gastrointestinal tract of the patient before administration is compared with a content of a short-chain fatty acid in gastrointestinal contents or excretions of the patient after administration; and

(6) decreasing a content of Enterobacteriaceae in gastrointestinal contents or excretions after administration when gastrointestinal contents or excretions modified with the IgA antibody are administered to a patient, and a content of Enterobacteriaceae in gastrointestinal contents or excretions of a subject before administration is compared with a content of Enterobacteriaceae in gastrointestinal contents or excretions after administration.

7.  A method for processing gastrointestinal contents or excretions of an IBD patient, the method comprising:

    (C1) a step of preparing gastrointestinal contents or excretions of an IBD patient; and
    (C2) a step of bringing the gastrointestinal contents or excretions into contact with, in vitro, IgA that restores bacterial flora in a gastrointestinal tract.

8.  A composition for modifying bacterial flora of gastrointestinal contents or excretions in vitro, comprising IgA that restores bacterial flora of a gastrointestinal tract.

9.  A method for testing subjects for presence or absence of IBD morbidity, or a risk of developing IBD for diagnosis, the method comprising:

    (D1) a step of preparing IgA antibody that restores bacterial flora in a gastrointestinal tract of an IBD patient;
    (D2) a step of contacting the gastrointestinal contents or excretions of a subject with the IgA antibody; and
    (D3) a step of identifying bacteria that bind to the IgA antibody,
    wherein if the IgA antibody and IBD-related bacteria are bonded, the subject is processed as suffering from IBD or having a risk of developing IBD.

10. A pharmaceutical drug for diagnosing presence or absence of IBD morbidity or a risk of developing IBD, the drug comprising an IgA antibody that restores bacterial flora in a gastrointestinal tract of an IBD patient.

11. A method for testing therapeutic effect of an IBD therapeutic agent, the method comprising:

    (E1) a step of obtaining gastrointestinal contents or excretions of a patient who is, is to be, or has been administered with an IBD therapeutic agent containing gastrointestinal contents or excretions;
    (E2) a step of contacting the gastrointestinal contents or excretions with an IgA antibody; and
    (E3) a step of analyzing bacteria that bind to the IgA antibody,
    wherein treatment with the IBD therapeutic agent is continued if the analysis result indicates restored bacterial flora in a gastrointestinal tract.

12. The method according to claim 11, wherein the gastrointestinal contents or excretions are modified with the IgA.

13. A diagnostic pharmaceutical drug containing an IgA antibody for testing therapeutic effect of an IBD therapeutic agent in a patient who is, is to be, or has been administered with an IBD therapeutic agent containing gastrointestinal contents or excretions.

14. The diagnostic pharmaceutical drug according to claim 13, wherein the gastrointestinal contents or excretions are modified with the IgA.

# FIG. 1

Human endogenous IgA antibody

# FIG. 2

## FIG. 3

HD (n=12)
CD (n=5)
UC (n=7)

| | Mean Diff. | 95.00% CI of diff. | Significant | Summary | Adjusted P Value |
|---|---|---|---|---|---|
| HD vs. CD | -16.00 | -34.05 to 2.055 | No | ns | 0.0885 |
| HD vs. UC | -4.348 | -20.48 to 11.78 | No | ns | 0.7779 |
| CD vs. UC | 11.65 | -8.209 to 31.51 | No | ns | 0.3209 |

# FIG. 4

HD (n = 12)

UC (n = 7)

CD (n = 5)

|  | Mean Diff. | 95.00% CI of diff. | Significant | Summary | Adjusted P Value |
|---|---|---|---|---|---|
| HD vs. CD | -13.45 | -21.55 to 5.344 | Yes | ** | 0.0012 |
| HD vs. UC | -9.452 | -16.69 to -2.213 | Yes | ** | 0.0093 |
| CD vs. UC | 3.995 | -4.918 to 12.91 | No | ns | 0.5070 |

# FIG. 5

HD (n =12)

CD (n = 5)

UC (n = 7)

| | Mean Diff. | 95.00% CI of diff. | Significant | Summary | Adjusted P Value | |
|---|---|---|---|---|---|---|
| HD vs. CD | -24.44 | -42.16 to -6.717 | Yes | ** | 0.0061 | A-B |
| HD vs. UC | -24.43 | -40.27 to -8.600 | Yes | ** | 0.0023 | A-C |
| CD vs. UC | 0.005429 | -19.49 to 19.50 | No | ns | >0.9999 | B-C |

## FIG. 6

HD (n =12)

CD (n = 5)

UC (n = 7)

| | Mean Diff. | 95.00% CI of diff. | Significant | Summary | Adjusted P Value | |
|---|---|---|---|---|---|---|
| HD vs. CD | -22.63 | -38.40 to -6.855 | Yes | ** | 0.0044 | A-B |
| HD vs. UC | -18.10 | -32.19 to -4.013 | Yes | * | 0.0105 | A-C |
| CD vs. UC | 4.522 | -12.83 to 21.87 | No | ns | 0.7905 | B-C |

# FIG. 7

HD (n =12)

CD (n = 5)

UC (n = 7)

| | Mean Diff. | 95.00% CI of diff. | Significant | Summary | Adjusted P Value | |
|---|---|---|---|---|---|---|
| HD vs. CD | -28.35 | -42.82 to -13.87 | Yes | *** | 0.0002 | A-B |
| HD vs. UC | -28.06 | -40.99 to -15.13 | Yes | **** | <0.0001 | A-C |
| CD vs. UC | 0.2863 | -15.64 to 16.21 | No | ns | 0.9989 | B-C |

# FIG. 8

HD (n =12)

CD (n = 5)

UC (n = 7)

| | Mean Diff. | 95.00% CI of diff. | Significant | Summary | Adjusted P Value | |
|---|---|---|---|---|---|---|
| HD vs. CD | -20.87 | -31.80 to -9.948 | Yes | *** | 0.0003 | A-B |
| HD vs. UC | -23.79 | -33.55 to -14.03 | Yes | **** | <0.0001 | A-C |
| CD vs. UC | -2.917 | -14.93 to 9.101 | No | ns | 0.8153 | B-C |

# FIG. 9

RS_H000_L001 antibody

## FIG. 10

## FIG. 11

### Protocol for administering IBD P10 samples to AID-KO GF mice

| donor | recipient | |
|---|---|---|
| IBD P10 patient feces | Sterile AID KO mice ♂ | |
| Transplanted bacterial flora | RS_H000_L001 antibody | Number |
| Whole bacterial flora | + | 3 |
| | - | 2 |
| RS_H000_L001 antibody non-binding bacteria | + | 3 |
| | - | 2 |

# FIG. 12

**Body weight change during induction of DSS enteritis
in mice administered with P10 whole bacterial flora**

RS_H000_L001 antibody
administration group (n = 3)

RS_H000_L001 antibody
non-administration group (n = 2)

# FIG. 13

Body weight change during induction of DSS enteritis in mice
administered P10-derived RS_H000_L001 antibody non-binding bacteria

# FIG. 14

Diversity analysis of intestinal flora before and after administration
of RS_H000_L001 antibody (Shannon index)

Significance test was performed using Student's two-tailed T-test.

# FIG. 15

## Protocol for administering IBD P13 samples to AID-KO GF mice

| donor | recipient |
|---|---|
| P13 patient feces | Sterile AID KO mice ♂ |

| Transplanted bacterial flora | RS_H000_L001 antibody | Number |
|---|---|---|
| Whole bacterial flora | + | 4 |
| | − | 4 |

# FIG. 16

FACS image of RS_H000_L001 antibody-binding bacterial fraction of P13 sample

# FIG. 17

**Body weight change during induction of DSS enteritis in mice administered with P13 whole bacterial flora**

# FIG. 18

Diversity analysis of intestinal flora before and after administration
of RS_H000_L001 antibody (Shannon index)

# FIG. 19

Relative abundance of each bacterial species before and after administration of RS_H000_L001 antibody

Significance test was performed using Student's two-tailed T-test.

# FIG. 20

Relative abundance of each bacterial species before
and after administration of RS_H000_L001 antibody

## Lachnospiraceae

RS_H000_L001 antibody

Whole bacterial flora

Significance test was performed using Student's two-tailed T-test.

# FIG. 21

Relative abundance of each bacterial species before
and after administration of RS_H000_L001 antibody

*Bacteroidaceae*

Significance test was performed using Student's two-tailed T-test.

# FIG. 22

Colon tissue images

RS_H000_L001 antibody
administered mice

RS_H000_L001 antibody
non-administered mice

HE staining

Alcian blue staining

Scale bar 500 µm

## FIG. 23

SNK0004 antibody

# *FIG. 24*

## SNK0005 antibody

# FIG. 25

## SNK0004 antibody IgA index

Median of IgA index is 0 or more.

# FIG. 26

## SNK0005 antibody IgA index

## FIG. 27

Median of IgA index is 0 or more in all samples.

SNK0001 antibody IgA index

# FIG. 28

## SNK0002 antibody IgA index

Median of IgA index is 0 or more.

## FIG. 29

### SNK0003 antibody IgA index

Median of IgA index is 0 or more.

# FIG. 30

P1 IgA clone sorting result family level

| | INTERNATIONAL SEARCH REPORT | International application No. |
|---|---|---|
| | | **PCT/JP2023/018019** |

**A. CLASSIFICATION OF SUBJECT MATTER**

*A61K 35/741*(2015.01)i; *A61K 39/395*(2006.01)i; *A61P 1/04*(2006.01)i; *G01N 33/15*(2006.01)i
FI:    A61K35/741; A61P1/04; A61K39/395 R; A61K39/395 D; G01N33/15 Z

According to International Patent Classification (IPC) or to both national classification and IPC

**B. FIELDS SEARCHED**

Minimum documentation searched (classification system followed by classification symbols)

A61K35/741; A61K39/395; A61P1/04; G01N33/15

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | SHINKURA, R. Therapeutic immunoglobulin A antibody for dysbiosis-related diseases. Int. Immunol. 2021, vol. 33, no. 12, pp. 787-790 <br> abstract, p. 788, right column, third paragraph to p. 789, right column, second paragraph | 1-14 |
| Y | JP 2020-121996 A (CRESTOVO HOLDINGS LLC) 13 August 2020 (2020-08-13) <br> paragraphs [0110], [0111] | 1-14 |
| Y | JP 2019-520340 A (CRESTOVO HOLDINGS LLC) 18 July 2019 (2019-07-18) <br> claims | 1-14 |
| Y | WO 2021/077107 A1 (CRESTOVO HOLDINGS LLC) 22 April 2021 (2021-04-22) <br> claims, paragraph [0002] | 1-14 |

☐ Further documents are listed in the continuation of Box C.     ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **07 July 2023** | **18 July 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**

International application No.

**PCT/JP2023/018019**

| Box No. I | Nucleotide and/or amino acid sequence(s) (Continuation of item 1.c of the first sheet) |

1.  With regard to any nucleotide and/or amino acid sequence disclosed in the international application, the international search was carried out on the basis of a sequence listing:

   a.  ☑ forming part of the international application as filed:

      ☑ in the form of an Annex C/ST.25 text file.

      ☐ on paper or in the form of an image file.

   b.  ☐ furnished together with the international application under PCT Rule 13*ter*.1(a) for the purposes of international search only in the form of an Annex C/ST.25 text file.

   c.  ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

   "The form of Annex C/ST.25 text file" above shall read as "the form of ST.26.".

Form PCT/ISA/210 (continuation of first sheet) (January 2015)

INTERNATIONAL SEARCH REPORT
**Information on patent family members**

International application No.

**PCT/JP2023/018019**

| Patent document cited in search report | | | Publication date (day/month/year) | Patent family member(s) | | | Publication date (day/month/year) |
|---|---|---|---|---|---|---|---|
| JP | 2020-121996 | A | 13 August 2020 | WO | 2016/183577 | A1 | |
| | | | | paragraphs [0110], [0111] | | | |
| | | | | US | 2016/0331791 | A1 | |
| | | | | EP | 3294307 | A1 | |
| | | | | KR | 10-2018-0026376 | A | |
| | | | | CN | 107949391 | A | |
| JP | 2019-520340 | A | 18 July 2019 | WO | 2017/210428 | A1 | |
| | | | | claims | | | |
| | | | | US | 2017/0348360 | A1 | |
| | | | | EP | 3462882 | A1 | |
| | | | | CN | 109803534 | A | |
| WO | 2021/077107 | A1 | 22 April 2021 | JP | 2022-552005 | A | |
| | | | | EP | 4045630 | A1 | |
| | | | | KR | 10-2022-0101637 | A | |
| | | | | CN | 115279382 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SANDRA C. KIM et al.** *Gastroenterology*, 2005, vol. 128, 891-906
- **MONIKA SCHAUBECK et al.** *Gut*, 2016, vol. 65, 225-237
- **ARLETTE DARFEUILLE-MICHAUD et al.** *Gastroenterology*, 2004, vol. 127, 412-421
- **ADELINE SIVIGNON et al.** *Inflamm Bowel Dis*, 2015, vol. 21, 276-286
- **TOSHIFUMI OHKUSA et al.** *Journal of Medical Microbiology*, 2009, vol. 58, 535-545
- **KABAT et al.** *Ann.NY Acad, Sci*, 1971, vol. 190, 382-391
- **KABAT, E.A. et al.** Sequences of Proteins of Immunological Interest. U.S. Department of Health and Human Services, 1991, 91-3242
- **CHOTHIA et al.** *Nature*, 1989, vol. 342, 877-883
- **MACCALLUM et al.** *J.Mol.Biol.*, 1996, vol. 262, 732-745
- **LEFRANC, M.-P**. *Nucl.Acids Res.*, 2005, vol. 33, D593-D597
- **KARLIN S ; ALTSCHUL SF**. *Proc.Natl Acad Sci USA.*, 1990, vol. 87, 2264-2268
- **KARLIN S ; ALTSCHUL SF.** *Natl Acad Sci USA*, 1993, vol. 90, 5873-7
- **ALTSCHUL SF ; GISHW ; MILLER W ; MYERS EW ; LIPMAN DJ**. *J Mol Biol.*, 1990, vol. 215, 403-10
- **ANDREW L. KAU et al.** *Sci Transl Med*, 2015, vol. 7
- **HIROSUKE SUGAHARA et al.** *Frontiers in Microbiology*, 2017, vol. 8, 1757